# EUROPÄISCHE PATENTSCHRIFT

(11) **EP 2 794 890 B1**
(45) Veröffentlichungstag und Bekanntmachung des Hinweises auf die Patenterteilung: **06.09.2017**
(21) Anmeldenummer: 12837622.5
(22) Anmeldetag: 21.12.2012
(51) Int. Cl.: C12N 15/82, A01H 5/00, A01H 5/10

(54) **NEUE AUS PFLANZEN STAMMENDE CIS-REGULATORISCHE ELEMENTE FÜR DIE ENTWICKLUNG PATHOGEN-RESPONSIVER CHIMÄRER PROMOTOREN**
NOVEL PLANT-DERIVED CIS-REGULATORY ELEMENTS FOR THE DEVELOPMENT OF PATHOGEN-RESPONSIVE CHIMERIC PROMOTORS
NOUVEAUX ÉLÉMENTS CIS-RÉGULATEURS D'ORIGINE VÉGÉTALE POUR LE DÉVELOPPEMENT DE PROMOTEURS CHIMÉRIQUES RÉAGISSANT AUX PATHOGÈNES

(30) Priorität: 23.12.2011 DE 102011122267
(43) Veröffentlichungstag der Anmeldung: 29.10.2014
(73) Patentinhaber: KWS SAAT SE, 37574 Einbeck (DE)
(72) Erfinder: STAHL, Dietmar, 37574 Einbeck (DE); WELTMEIER, Fridtjof, 37574 Einbeck (DE); HEHL, Reinhard, 38114 Braunschweig (DE); KOSCHMANN, Jeanette, 38154 Königslutter (DE); NIEMEYER, Julia, 31855 Aerzen (DE)
(86) Internationale Anmeldenummer: PCT/DE2012/001223
(87) Internationale Veröffentlichungsnummer: WO 2013/091612

(56) Entgegenhaltungen:
- WO-A2-2007/147395
- DATABASE EMBL [Online] 7. Februar 2005 (2005-02-07), "Sequence 2372 from Patent EP1502950.", XP002698295, gefunden im EBI accession no. EMBL:CS004344 Database accession no. CS004344
- DATABASE EMBL [Online] 6. Oktober 1999 (1999-10-06), "Sequence 2058 from patent US 5837542.", XP002698296, gefunden im EBI accession no. EMBL:AR057854 Database accession no. AR057854
- P. J. RUSHTON: "Synthetic Plant Promoters Containing Defined Regulatory Elements Provide Novel Insights into Pathogen- and Wound-Induced Signaling", THE PLANT CELL ONLINE, Bd. 14, Nr. 4, 1. April 2002 (2002-04-01), Seiten 749-762, XP055012026, ISSN: 1040-4651, DOI: 10.1105/tpc.010412 in der Anmeldung erwähnt
- R. WINGENDER: "cis-Regulatory Elements Involved in Ultraviolet Light Regulation and Plant Defense", THE PLANT CELL ONLINE, Bd. 2, Nr. 10, 1. Oktober 1990 (1990-10-01), Seiten 1019-1026, XP055065391, ISSN: 1040-4651, DOI: 10.1105/tpc.2.10.1019
- SHOKOUHIFAR F ET AL: "Construction and functional analysis of pathogen-inducible synthetic promoters in", BIOLOGIA PLANTARUM, KLUWER ACADEMIC PUBLISHERS, DO, Bd. 55, Nr. 4, 15. Oktober 2011 (2011-10-15), Seiten 689-695, XP019965025, ISSN: 1573-8264, DOI: 10.1007/S10535-011-0169-5

## Beschreibung

Die vorliegende Erfindung betrifft cis-regulatorische Elemente und aus diesen cis-regulatorischen Elementen erstellte chimäre Promotoren mit einer Pathogen- oder Elizitorinduzierten Aktivität in Pflanzen. Darüber hinaus betrifft die vorliegende Erfindung eine Wirtszelle, eine transgene Pflanzenzelle, ein transgenes Pflanzengewebe sowie eine transgene Pflanze und deren Samen. Die vorliegende Erfindung betrifft weiterhin ein Verfahren zur Herstellung einer transgenen Pflanze, welche insbesondere resistent ist gegenüber einem Pathogen.

Durch Pilze, Viren, Nematoden und Bakterien hervorgerufene Pflanzenkrankheiten bewirken weltweit große Ernteverluste, beeinträchtigen die Qualität der Ernteprodukte und machen einen aufwendigen Einsatz chemischer Pflanzenschutzmittel notwendig, weil die natürlichen Abwehrmaßnahmen der Pflanzen, mit deren Hilfe sie die Mehrzahl potentieller Krankheitserreger abwehren oder deren Ausbreitung verzögern und einschränken können, häufig nicht ausreichen. Gentechnische Ansätze können zur Erzeugung von Pflanzen, die resistent gegenüber genannten Pathogenen sind, genutzt werden. Diese Pflanzen weisen eine erhöhte Resistenz auf, indem sie eine spezifisch am Infektionsort (Kontaktstelle zwischen Pathogen und Pflanze) erfolgende Expression von Proteinen (Effektoren) erzeugen, die eine starke Resistenzreaktion der Pflanze auslösen, oder von Molekülen, die selbst toxisch gegen Pathogene sind oder deren Wachstum oder Virulenz hemmen. Effektoren, die eine starke Resistenzreaktion der Pflanze auslösen, sind z.B. Proteine von autoaktivierten Resistenzgenen (R-Genen) oder Avirulenzgene, die zur Aktivierung endogener Resistenzgene der Pflanze führen. Zu der starken Resistenzreaktion gehören die hypersensitive Reaktion (HR), der kontrollierte Zelltod des Wirtsgewebes an der Infektionsstelle, die Verstärkung der pflanzlichen Zellwand durch Lignifizierung und Kallosebildung, die Bildung von Phytoalexinen und die Produktion von PR-(pathogenesis-related)-Proteinen.
Da diese Resistenzreaktion einen hohen Energiebedarf darstellen und zum Absterben von Pflanzenzellen (Nekrose) führen können, muss ihre Auslösung einer strengen Kontrolle unterliegen. Dasselbe gilt auch für die Expression von für Pathogene toxischen Proteinen oder Peptiden, sofern eine konstitutive Expression dieser Proteine oder Peptide eine nachteilige Wirkung auf die Pflanze bzw. deren agronomische Eigenschaften wie z.B. Ertrag hat. In einem transgenen Ansatz ist diese Kontrolle durch Verwendung von Promotoren mit der gewünschten Spezifität möglich.

So lässt sich eine Pathogen-induzierte Expression von Transgenen wie Effektoren durch Verwendung verschiedener bekannter, natürlicher Pathogen-induzierbarer Promotoren, wie z.B. des PR1-Promotors, erreichen (Rushton et al., 1996). Insbesondere die Entwicklung der Mikroarraytechnologie hat zur Identifikation einer Vielzahl von Pathogen-induzierbaren Promotoren geführt (WO 03/00898, WO 02/50293 oder JP 2003284566).
Solche natürlichen Pathogen-induzierbaren Promotoren können jedoch sehr unspezifische Aktivitäten zeigen, da sie durch zahlreiche unterschiedliche Stimuli aktiviert werden können. Zurückzuführen ist dies auf einen modularen Aufbau des Promotors aus mehreren unterschiedlichen cis-regulatorischen Elementen, welche verschiedenste unterschiedliche Signale in ein komplexes Expressionsprofil integrieren. Folglich sind natürliche Pathogen-induzierbare Promotoren auch durch unerwünschte Aktivitäten wie etwa in bestimmten Geweben oder durch eine hohe Hintergrundaktivität gekennzeichnet.
So sind beispielsweise Pathogen-induzierbare Promotoren von Defensin-Genen aus Weizen auch während der Samenentwicklung und Samenkeimung aktiv (Kovalchuk et al., 2010). Der bereits erwähnte PR1-Promotor wird nicht nur durch Pathogene, sondern auch durch Seneszenz induziert (Morris et al. 2000)
In anderen Untersuchungen zeigte sich, dass der natürliche, durch Rost induzierbare Fis1 Promotor aus Lein nach Transformation nicht dazu geeignet war, die Expression von autoaktiven Formen des L6 Rostresistenzgens in *Linum usitatissimum* so spezifisch zu regulieren, dass neben der Rostresistenz keine negative agronomischen Eigenschaften wie Kleinwüchsigkeit auftraten (Howles et al., 2005).
Eine Möglichkeit, die gewünschte Spezifität eines Promotors zu erhöhen, ist die Identifikation der für die gewünschte Induktion verantwortlichen cis-regulatorischen Elemente, und der Aufbau chimärer Promotoren aus diesen cis-regulatorischen Elementen (Venter, 2007). Sequenzmotive für andere Stimuli werden dagegen entfernt.
Die Promotoren vieler Pathogen-induzierter Gene sind genauer untersucht worden, wobei mehrere cis-regulatorische Elemente, die eine Pathogen-spezifische Induktion vermitteln können, identifiziert wurden (Strittmatter et al., 1996; Eulgem et al., 2000; Kirsch et al., 2000, 2001; Himmelbach et al., 2010). Weitere Bespiele sind auch die durch schrittweises Mutieren von natürlichen Pathogen-induzierbaren Promotoren identifizierten cis-regulatorischen Elemente D-Box, S-Box oder W-Box (WO 00/29592) oder die linker scanning Regionen LS10 oder LS7 (Lebel et al., 1998). Insbesondere die W-Box ist gut untersucht, und deren Kernsequenz TTGAC(C/T) kann genutzt werden, um zusätzliche Varianten der W-Box in natürlichen Pathogen-induzierbaren Promotoren aufzufinden.
Des Weiteren können neue cis-regulatorische Elemente bioinformatorisch mit Hilfe von Programmen wie MEME (Bailey und Elkan, 1994; Humphry et al., 2010) oder BEST (Che et al., 2005) identifiziert werden. Ein Vorteil hierbei liegt darin, dass ein cis-regulatorisches Element nicht als kurze Einzelsequenz, sondern als ein genau definiertes Sequenzmotiv identifiziert wird, über das gleich mehrere Varianten eines cis-regulatorischen Elementes, also mehrere Varianten einer Bindestelle eines Transkriptionsfaktors, erfasst werden. Allerdings sind solche bioinformatorischen Ansätze auch hochanfällig für die Bestimmung falsch-positiver Sequenzen, so dass sie lediglich zu einer Vorauswahl von potentiellen Sequenzen bzw. Sequenzmotiven führen. Zwingend und unerlässlich bleiben dann aber Nachweis und Überprüfung der Funktionalität als cis-regulatorisches Element im Allgemeinen und als Pathogeninduzierbarkeit-vermittelndes cis-regulatorisches Element im Speziellen. Solche experimentellen Analysen sind zudem mit nicht unerheblichem Aufwand verbunden.
Eine weitere Erhöhung der Spezifität eines Promotors ist durch die Verwendung von Kombinationen unterschiedlicher cis-regulatorischer Elemente möglich (Rushton et al., 2002). Dabei führt nicht die Kombination an sich zu einer Erhöhung der Aktivität (Synergismus), sondern ein solcher Synergismus tritt nur bei spezifischen einzelnen, nichtvorhersagbaren Kombinationen auf und muss in jedem Fall empirisch bestimmt werden. Bekannt sind z.B. chimäre Promotoren mit Kombinationen aus den cis-regulatorischen Elementen D-Box und S-Box (WO 00/29592). Die Zahl der Elementwiederholungen moduliert die Promotorstärke und die Hintergrundaktivität.
Ein weiteres Problem bei der Entwicklung von Pathogen-induzierbaren chimären Promotoren ist deren Funktionalität in unterschiedlichen Pflanzenspezies. Generell kann zwar in nahezu allen bisher untersuchten Pflanzenarten eine Pathogen-Induzierbarkeit durch die bekannten, Pathogen-induzierbaren chimären Promotoren festgestellt werden, jedoch zeigen diese weiterhin Hintergrundaktivität auch unter Nichtbefallsbedingungen durch einen Pathogen. Diese Hintergrundaktivität schwankt in Abhängigkeit von der Pflanzenart, in welcher die chimären Promotoren verwendet werden. Ebenso verhält es sich mit der Induktionsrate (Quotient aus der Promotoraktivität im infizierten Gewebe und die Promotoraktivität im nicht infizierten Gewebe) und der absoluten Aktivität der Promotoren (Promotorstärke). So kann beispielsweise durch eine zu starke Hintergrundaktivität in nicht infiziertem Gewebe dann nur noch eine geringe Pathogen-Induzierbarkeit im infizierten Gewebe festgestellt werden. Nach heutigem Wissensstand werden für die beschriebenen Schwankungen bezüglich Hintergrundaktivität, Induktionsrate, Promotorstärke, Induktionskinetik und der räumliche Ausdehnung der Promotoraktivierung die verwendeten cis-regulatorischen Elemente eines Promotors verantwortlich gemacht (Rushton et al., 2002, Venter, 2007). Auch wenn die bekannten chimären Promotoren den natürlichen Promotoren überlegen sind, besteht weiterhin ein Optimierungsbedarf dieser chimären Promotoren insbesondere im Hinblick auf die cis-regulatorischen Elemente und/oder auf die Kombinationen von cis-regulatorischen Elementen. Es fehlt weiterhin an gut charakterisierten cis-regulatorischen Elementen und an geeigneten Kombinationen solcher cis-regulatorischer Elemente, mit deren Hilfe chimäre Promotoren konstruiert werden können, welche eine hochspezifische und kontrollierte, bedarfsgerechte Pathogen-induzierte Expression von Transgenen an sich und auch in diversen Pflanzenspezies gewährleisten, wobei die Expression lediglich infolge eines Pathogenbefalls und nahezu ausschließlich an der Infektionsstelle stattfindet soll (Gurr & Rushton, 2005). Daher ist es Aufgabe der vorliegenden Erfindung solche neuen Pathogeninduzierbarkeit-vermittelnden cis-regulatorischen Elemente und Kombinationen davon bereitzustellen.

Einige der in dieser Anmeldung verwendeten Begriffe werden nachfolgend zunächst näher erläutert:
Ein "Elizitor" im Sinne der vorliegenden Erfindung stellt einen Induktor oder Botenstoff dar, welcher Abwehrmaßnahmen gegen pflanzliche Pathogene wie beispielsweise die Synthese von Phytoalexinen induziert. Elizitoren können entweder endogenen oder exogenen Ursprungs sein. Vorzugsweise stammt ein Elizitor (exogen) aus einem Pathogen und wird von der Pflanze erkannt. Zu diesen Elizitoren gehören auch die PAMPs (pathogen associated molucular pattern) wie beispielsweise Flagelin, PEP25 und Chitin. Elizitoren können verwendet werden, um eine Pathogeninfektion oder einen Kontakt mit einem Pathogen zu imitieren, indem der Elizitor künstlich, in der Abwesenheit des Pathogens, appliziert wird. Im Zusammenhang mit der vorliegenden Erfindung werden Elizitoren insbesondere genutzt um die Induzierbarkeit von Promotoren zu überprüfen.

Eine "Einzelsequenz" ist eine Abfolge von Nukleotiden oder Basen(paaren), wobei jede Position in der Einzelsequenz nur durch eine einzelne fest definierte Base (a, c, g oder t) festgelegt ist. Eine Einzelsequenz wird isoliert aus einem natürlichen Promotor und stellt das Ergebnis einer bioinformatorischen Analyse dar. Eine Einzelsequenz ist aus einer Kernsequenz und flankierenden Sequenzbereichen zusammengesetzt. Der Begriff "Einzelsequenz" meint auch ein Nukleinsäuremolekül, dessen Nukleotid- oder Basen(paaren)abfolge der Einzelsequenz entspricht.

Eine "Kernsequenz" ist die Abfolge von Nukleotiden oder Basen(paaren) in einem bestimmten Abschnitt eines cis-regulatorischen Elementes, wobei dieser Abschnitt für die Funktionalität des cis-regulatorischen Elementes essentiell ist. Die Kernsequenz stellt einen Teil der Einzelsequenz dar. Der Begriff "Kernsequenz" meint auch ein Nukleinsäuremolekül, dessen Nukleotid- oder Basen(paaren)abfolge der Kernsequenz entspricht.

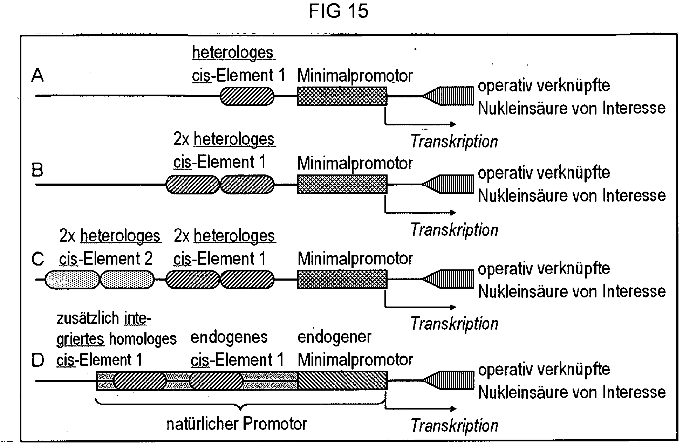

Ein "Promotor" meint eine nicht-translatierte DNA-Sequenz, typischerweise stromaufwärts einer kodierenden Region, welche die Bindestelle für die RNA-Polymerase beinhaltet und die Transkription der DNA initiiert. Ein Promotor enthält häufig zudem andere Elemente, die als Regulatoren der Genexpression fungieren (z.B. cis-regulatorische Elemente).
Ein "Minimalpromotor" ist ein Promotor, der lediglich die Grundelemente, welche für die Transkriptionsinitiation gebraucht werden, aufweist (z.B. TATA-Box und/oder Initiator).
Als "chimärer Promotor" wird ein Promotor bezeichnet, der so in der Natur nicht vorkommt, aus mehreren Elementen zusammengesetzt wird. Er beinhaltet einen Minimalpromotor und weist stromaufwärts des Minimalpromotors mindestens ein cis-regulatorisches Element auf, welches als Bindungsstelle für spezielle trans-wirkende Faktoren (*trans-acting factors,* z.B. Transkriptionsfaktoren) dient. Ein chimärer Promotor wird den gewünschten Anforderungen nach konzipiert und durch unterschiedliche Faktoren induziert oder reprimiert. Die Wahl des cis-regulatorischen Elements oder einer Kombination von cis-regulatorischen Elementen ist entscheidend beispielsweise für die Spezifität oder das Aktivitätslevel eines Promotors. Ein cis-regulatorisches Element in einem chimären Promotor ist entweder heterolog zu dem verwendeten Minimalpromotor, d.h. das cis-regulatorische Element stammt aus einem anderen Organismus oder einer anderen Spezies als der verwendete Minimalpromotor (beispielhaft in Fig. 15A-C dargestellt), oder ein cis-regulatorisches Element in einem chimären Promotor ist homolog zu dem verwendeten Minimalpromotor, d.h. das cis-regulatorische Element und der Minimalpromotor kommen auch in einem natürlichen Promotor zusammengesetzt vor, jedoch ist das cis-regulatorische Element für sich oder als zusätzliches Element innerhalb des chimären Promotor in einer unterschiedlichen genetischen Umgebung im Vergleich zu dem natürlichen Promotor lokalisiert. Ein chimärer Promotor meint demnach auch einen (natürlichen) Promotor, welcher durch Multimerisieren von mindestens einem cis-regulatorischen Element verändert wurde (beispielhaft in Fig. 15D dargestellt).

Eine "komplementäre" Nukleotidsequenz bedeutet bezogen auf eine doppelsträngige DNA, dass der zum ersten DNA Strang komplementäre zweite DNA Strang entsprechend den Basenpaarungsregeln und unter Berücksichtigung der Orientierung die Nukleotidbasen aufweist, die zu den Basen des ersten Stranges korrespondieren (Bsp: 5'-gcat-3' ist komplementär zu 5'-atgc-3').

Ein "Pathogen" meint einen Organismus, der in Interaktionen mit einer Pflanze zu Krankheitssymptomen an einem oder mehreren Organen bei der Pflanze führt. Zu diesen Pathogenen zählen beipielsweise tierische, pilzliche, bakterielle oder virale Organismen oder Oomyceten.

Unter einer "Pathogeninfektion" ist der früheste Zeitpunkt zu verstehen, bei dem der Stoffwechsel eines Pathogens auf eine Penetration des pflanzlichen Wirtsgewebes vorbereitet wird. Dazu gehören z.B. bei Pilzen oder bei Oomyceten das Auswachsen von Hyphen oder die Bildung von spezifischen Infektionsstrukturen wie Penetrationshyphen und Appressorien.

Unter "Pathogen-/Elizitor-Induzierbarkeit" oder unter "Pathogen-/Elizitor-induzierbar" meint im Sinne der Erfindung die spezifische Eigenschaft eines Promotors, welcher nach Pathogeninfektion oder Elizitor-Applikation eine mindestens zweifach verstärkte Transkription eines operativ verknüpften Gens verursacht. Des Weiteren ist unter "Pathogen-/Elizitor-Induzierbarkeit" oder unter "Pathogen-/Elizitor-induzierbar" im Sinne der Erfindung die Eigenschaft von Genen zu verstehen, die nach Pathogeninfektion oder Elizitor-Applikation mindestens zweifach verstärkt transkribiert werden.

Erfindungsgemäß erfolgt die Lösung der gestellten Aufgabe durch neue Pathogen- und/oder Elizitor-Induzierbarkeit vermittelnde cis-regulatorische Elemente. Diese unterscheiden sich insbesondere innerhalb der Kernsequenz signifikant von bereits bekannten Elementen und stellen somit auch keine Variationen der bekannten Elemente dar. Demnach sollten die erfindungsgemäßen cis-regulatorischen Elemente als Erkennungs- und/oder Bindestellen für neue Transkriptionsfaktoren dienen, was zur Folge hat, dass die vermittelte Pathogen-und/oder Elizitor-Induzierbarkeit eine neuartige Spezifität aufweist. Die erfindungsgemäßen cis-regulatorischen Elemente wurden über bioinformatorische Ansätze in Promotoren von Pathogen- oder PAMP (Elizitor)-induzierten Genen aus *Arabidopsis thaliana* identifiziert. Die isolierten Einzelsequenzen der cis-regulatorischen Elemente konnten infolge diverser Analyseschritte acht Motivgruppen (Motivgruppe 1, 5, 11, 12, 18, 21, 27 und 32) zugeordnet werden. Weiterhin konnten auch mehrere isolierte Einzelsequenzen einer Motivgruppe 21n zugeordnet werden. In einer Motivgruppe sind, diejenigen Einzelsequenzen zusammengefasst, welche beim Vergleich der identifizierten Motive ein hohes Maß an Konserviertheit aufzeigen. Einzelsequenzen einer Motivgruppe stimmen alle demnach in einem charakteristischen Kernsequenzmotiv überein. Somit wird die gestellte Aufgabe durch ein isoliertes cis-regulatorisches Element, welches ein Nukleinsäuremolekül umfasst, dessen Nukleotidsequenz ein Kernsequenzmotiv
vaaagtm, aufweist.
Weniger stark konservierte Basenpositionen innerhalb der charakteristischen Kernsequenzmotive a) bis i) sind wie folgt angegeben: 'r' steht für Guanin (g) oder Adenin (a), also eine Purinbase, 'k' steht für Guanin (g) oder Thymin (t)/Uracil (u), 's' steht für Guanin (g) oder Cytosin (c), 'm' steht für Adenin (a) oder Cytosin (c) und 'w' steht für Adenin (a) oder Thymin (t)/Uracil (u). Ein bestimmtes Kernsequenzmotiv gibt zumindest eine Teilsequenz der Kernsequenz einer jeden Einzelsequenz der zum Kernsequenzmotiv gehörigen Motivgruppe wieder, wobei die Teilsequenz mindestens 30% der Gesamtkernsequenz einer Einzelsequenz ausmachen kann. Für die Motivgruppen mit den Kernsequenzmotiven g) und h) entspricht das Kernsequenzmotiv der gesamten Kernsequenz der Einzelsequenzen. Von der Erfindung ist weiterhin auch ein isoliertes cis-regulatorisches Element mit eingeschlossen, welches ein Nukleinsäuremolekül umfasst, dessen Nukleotidsequenz einem zu a) bis i) komplementären Kernsequenzmotiv entspricht. Ein charakteristisches Kernsequenzmotiv einer bestimmten Motivgruppe kann zudem auch mehrfach in der Kernsequenz einer Einzelsequenz vorkommen, wobei die Kernsequenzmotive auch überlappend in der Kernsequenz auftauchen und/oder jeweils eine unterschiedliche Orientierung aufzeigen können.

Für Motivgruppen 1, 5, 11, 12, 21, 21n und 27 konnte auf Basis der experimentellen Daten zur Funktionalität ein Familienmotiv definiert werden, in welchem das charakteristische Kernsequenzmotiv eingebettet ist. Das Familienmotiv stellt ein abgeleitetes Erkennungsmerkmal für einen Transkriptionsfaktor oder eine Transkriptionsfaktorfamilie dar. Der Vorteil eines Familienmotivs liegt darin, dass es mögliche Varianten eines Erkennungs-/Bindungsbereichs zusammenfasst. Vorzugsweise umfasst ein Familienmotiv einer Motivgruppe alle Kernsequenzen der in der Motivgruppe zusammengefassten Einzelsequenzen. Hierfür wurde für einen Teil der Einzelsequenzen der komplementäre Strang des cis-regulatorischen Elements berücksichtigt; solche komplementären Sequenzen von identifizierten erfindungsgemäßen cis-regulatorischen Sequenzen sind, sofern es zum Verständnis notwendig ist, im Weiteren mit '(inv)' gekennzeichnet. Ein auf diese Weise definiertes Familienmotiv weist eine Länge von mindestens 15 Nukleotiden, bevorzugt von mindestens 13 Nukleotiden, besonders bevorzugt von mindestens 11 Nukleotiden auf. Neben dem entsprechenden Kernsequenzmotiv weist das Familienmotiv flankierende Bereiche auf, welche bei der Verwendung eines erfindungsgemäßen cis-regulatorischen Elementes in einem chimären Promotor einen wesentlichen quantitativen Einfluss auf dessen Eigenschaften wie Hintergrundaktivität und Expressionsstärke haben. Mit zunehmendem Abstand einer bestimmten Base der flankierenden Bereiche zur Kernsequenz in einer Einzelsequenz nimmt deren quantitativer Einfluss ab. Zur Definition des Familienmotivs können zusätzlich auch weitere über die Kernsequenzen der Einzelsequenzen hinausgehende hochkonservierte Einzelbasen aus den flankierenden Bereichen berücksichtigt werden, die dann das Familienmotiv erweitern. Die Familienmotive für die Motivgruppen 1, 5, 11, 12, 21, 21n und 27 sind in Spalte 2 der Tabelle 1 dargestellt.

Von der Erfindung ist demnach auch ein isoliertes cis-regulatorisches Element mit eingeschlossen, welches ein Nukleinsäuremolekül umfasst, dessen Nukleotidsequenz
a) einem Familienmotiv gemäß SEQ ID NO: 1, umfassend das Kernsequenzmotiv vaaagtm, entspricht,
oder dessen Nukleotidsequenz einem zu a) komplementären Familienmotiv entspricht. Zudem kann das Familienmotiv auch kürzer sein. In seiner kürzesten Form ist es definiert als ein minimales Familienmotiv, das nach Ausrichtung der Einzelsequenzen entsprechend dem gemeinsamen Kernsequenzmotiv nur diejenigen Basenpositionen in sich zusammenfasst, welche sich in den Kernsequenzen sämtlicher Einzelsequenzen einer Motivgruppe wiederfinden. In einigen Fällen entspricht das minimale Familienmotiv dem Kernsequenzmotiv. Tabelle 1 gibt in Spalte 2, überschrieben mit Familienmotiv, unterstrichen die minimalen Familienmotive der Motivgruppen 1,5, 11, 12, 21, 21n und 27 wieder.

**Tabelle 1: Darstellung der Motivgruppen 1, 5, 11, 12, 18, 21, 21n, 27 und 32, der den Gruppen zugrundeliegenden Kernsequenzmotive (1), Familienmotive (2) und der den Motivgruppen zugeordneten Einzelsequenzen (3); minimales Familienmotiv ist unterstrichen, Kernsequenzmotiv innerhalb des Familienmotivs ist fett gedruckt.**

| ('n' steht für eine beliebige Base; 'h' steht für a, c oder t/u; 'd' steht für a, g oder t/u; 'v' steht für a, g oder c; 'r' steht für g oder a, 'k' steht für g oder t/u, 's' steht für g oder c und 'm' steht für a oder c; 'y' steht für t/u oder c; 'w' steht für a oder t/u) | | | | |
|---|---|---|---|---|
| 1 | 2 | | 3 | |
| Kernsequenzmotiv | Familienmotiv | | Einzelsequenzbezeichnung | |
| Motivgruppe 27 | | | | |
| vaaagtm | nnhkdnn**vaaagtm**ndhy | (SEQ ID NO: 1) | 30I-8_M1_S1 | (SEQ ID NO: 7) |
| | | | 30I-8_M1_S2 | (SEQ ID NO: 8) |
| | | | GG13_M1_S2 | (SEQ ID NO: 9) |
| | | | 14S_M1_S1 | (SEQ ID NO: 10) |
| | | | 21S_M3_S1 | (SEQ ID NO: 11) |
| | | | 30I-8_M1_S3 | (SEQ ID NO: 12) |
| | | | Cis02 | (SEQ ID NO: 13) |
| | | | Cis05 | (SEQ ID NO: 14) |
| | | | sCis05 | (SEQ ID NO: 44) |
| | | | Cis13 | (SEQ ID NO: 15) |

| Motivgruppe 11 | | | | |
|---|---|---|---|---|
| aaacca | ynamcn**aaacca**wwny | (SEQ ID NO: 2) | GG11_M1_S1 | (SEQ ID NO: 16) |
| | | | 22DDD_M1_S1 | (SEQ ID NO: 17) |
| | | | 21G-2_M1_S2 | (SEQ ID NO: 18) |
| | | | GG6_M1_S1 | (SEQ ID NO: 19) |

| Motivgruppe 12 | | | | |
|---|---|---|---|---|
| scaaam | wnrm**scaaam**smw | (SEQ ID NO: 3) | 18H_M2_S1 | (SEQ ID NO: 20) |
| | | | 18H_M2_S3 | (SEQ ID NO: 21) |
| | | | 38M_M1_S1 | (SEQ ID NO: 22) |
| | | | 26LLL_M1_S2 | (SEQ ID NO: 23) |

| Motivgruppe 1 | | | | |
|---|---|---|---|---|
| acrcg | nnms**acrcg**ynwm | (SEQ ID NO: 4) | Cis09 | (SEQ ID NO: 24) |
| | | | Cis12 | (SEQ ID NO: 25) |
| | | | 12i_M1_S1 | (SEQ ID NO: 26) |

| Motivgruppe 21 | | | | |
|---|---|---|---|---|
| sktpkact | a**sktgkact**wkgwm | (SEQ ID NO: 5) | GG8_M1_S1 | (SEQ ID NO: 27) |
| | | | 27G-8_M1_S1 | (SEQ ID NO: 28) |

| Motivgruppe 21n | | | | |
|---|---|---|---|---|
| wwkgwc | snsnnn**wwkgw**cnnnsnm | (SEQ ID NO: 41) | GG8_M1_S1 | (SEQ ID NO: 27) |
| | | | 27G-8_M1_S1 | (SEQ ID NO: 28) |
| | | | 26WW_M2_S1 | (SEQ ID NO: 42) |
| | | | 27B-10_M1_S3 | (SEQ ID NO: 43) |

| Motivgruppe 5 | | | | |
|---|---|---|---|---|
| mrtsack | knwym**mrtsack**wmn | (SEQ ID NO: 6) | 20u_M1_S1 | (SEQ ID NO: 30) |
| | | | 20u_M1_S2 | (SEQ ID NO: 31) |
| | | | 28M-1_M1_S1 | (SEQ ID NO: 32) |

| Motivgruppe 18 | | | | |
|---|---|---|---|---|
| ccaccaa | | | 12G_M2_S1 | (SEQ ID NO: 33) |

| Motivgruppe 32 | | | | |
|---|---|---|---|---|
| tcgtctcttc (SEQ ID NO: 35) | | | 12r_M1_S1 | (SEQ ID NO: 34) |

Die Erfindung betrifft zudem auch identifizierte und isolierte Einzelsequenzen von erfindungsgemäßen cis-regulatorischen Elementen und deren Kernsequenzen (Tabelle 1 und 2). Die gestellte Aufgabe wird somit auch gelöst durch ein isoliertes cis-regulatorisches Element, umfassend ein Nukleinsäuremolekül
a) mit einer Nukleotidsequenz gemäß SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15 oder SEQ ID NO: 44, b) ein Nukleinsäuremolekül mit einer Nukleotidsequenz gemäß der Kernsequenz aus SEQ ID NO: 7, SEQ ID NO: 8, SEQ ID NO: 9, SEQ ID NO: 10, SEQ ID NO: 11, SEQ 10 NO: 12, SEQ ID NO: 13, SEQ ID NO: 14, SEQ ID NO: 15, oder SEQ ID NO: 44, oder
c) ein Nukleinsäuremolekül mit einer Nukleotidsequenz komplementär zu einer der Nukleotidsequenzen aus a) oder b).

Ein cis-regulatorisches Element, umfassend ein Nukleinsäuremoleküle mit einer Nukleotidsequenz gemäß SEQ ID NO: 24, SEQ ID NO: 25 oder SEQ ID NO: 26 ist der Motivgruppe 1, ein solches mit einer Nukleotidsequenz gemäß SEQ ID NO: 30, SEQ ID NO: 31 oder SEQ ID NO: 32 der Motivgruppe 5, ein solches mit einer Nukleotidsequenz gemäß SEQ ID NO: 16, SEQ ID NO: 17, SEQ ID NO: 18 oder SEQ ID NO: 19 der Motivgruppe 11, ein solches mit einer Nukleotidsequenz gemäß SEQ ID NO: 20, SEQ ID NO: 21, SEQ ID NO: 22 oder SEQ ID NO: 23 der Motivgruppe 12, ein solches mit einer Nukleotidsequenz gemäß SEQ ID NO: 33 der Motivgruppe 18, ein solches mit einer Nukleotidsequenz gemäß SEQ ID NO: 27 oder SEQ ID NO: 28 der Motivgruppe 21, ein solches mit einer Nukleotidsequenz gemäß SEQ ID NO: 27, SEQ ID NO: 28, SEQ ID NO: 42 oder SEQ ID NO: 43 der Motivgruppe 21n, ein solches mit einer Nukleotidsequenz gemäß SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 44 oder SEQ ID NO: 15 der Motivgruppe 27 und ein solches mit der Nukleotidsequenz gemäß SEQ ID NO: 34 der Motivgruppe 32 zugeordnet.

Ein erfindungsgemäßes cis-regulatorisches Element kann eine Länge von weniger als 50 Nukleotiden, bevorzugt von weniger als 40 Nukleotiden und besonders bevorzugt von weniger als 30 Nukleotiden aufweisen. Die Kernsequenz eines erfindungsgemäßen cis-regulatorischen Elementes kann eine Länge von weniger als 20 Nukleotiden, bevorzugt von weniger als 15 Nukleotiden und besonders bevorzugt von weniger als 10 Nukleotiden aufweisen, jedoch sollte die Kernsequenz nicht kürzer sein als 6 Nukleotide.

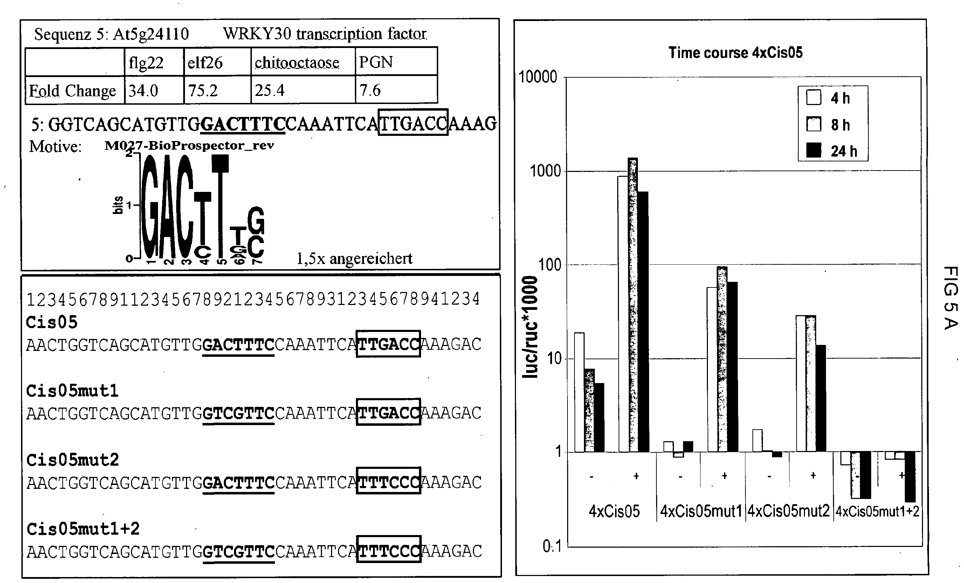

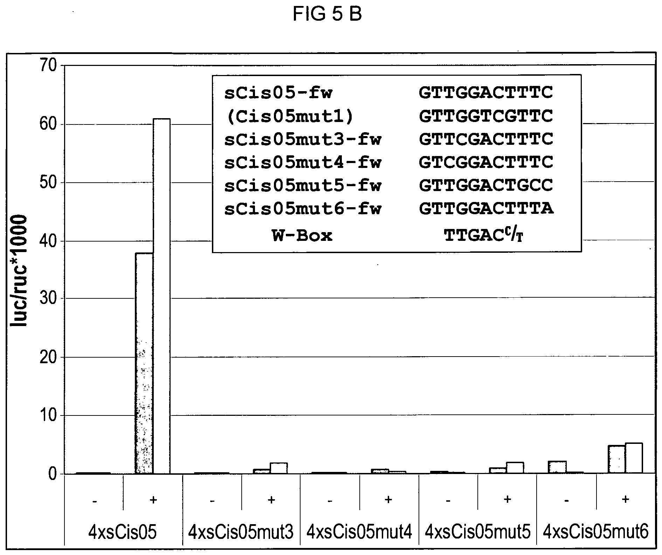

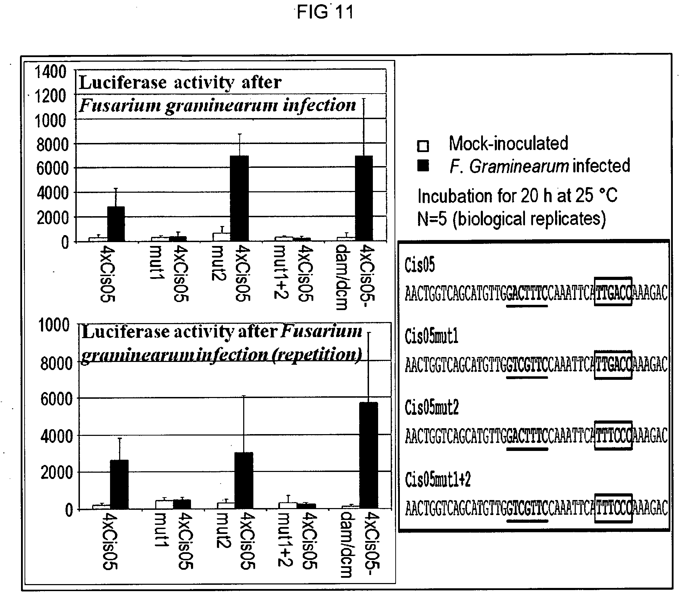

Einige Einzelsequenzen der erfindungsgemäßen cis-regulatorischen Elemente können neben der identifizierten neuen Kernsequenz auch zusätzlich die Kernsequenz eines bekannten Pathogen-/Elizitor-Induzierbarkeit vermittelnden cis-regulatorischen Elements aufweisen, das unter Umständen auch die Spezifität der neuen identifizierten Kernsequenz und/oder der identifizierten Einzelsequenz beeinflusst. Ein Beispiel ist das cis-regulatorische Element Cis05 (SEQ ID NO: 14), dass neben der identifizierten Kernsequenz zwischen den Nukleotidpositionen 14 und 20 auch eine W-Box Kernsequenz zwischen den Nukleotidpositionen 29 und 35 aufweist. Hierzu wurden umfangreiche Mutationsanalysen durchgeführt (siehe Fig. 5A, 5B und 11).

Ein erfindungsgemäßes cis-regulatorisches Element kann in einem chimären Promotor verwendet werden, wobei das cis-regulatorische Element dem chimären Promotor eine spezifische Pathogen- und/oder Elizitorinduzierbarkeit vermittelt. Die vorliegende Erfindung schließt somit auch einen chimären Promotor mit ein, welcher geeignet ist, induziert durch eine Pathogeninfektion oder eine Behandlung mit einem pathogenen Elizitor eine Expression eines operativ verknüpften Nukleinsäuremoleküls von Interesse, z.B. einer heterologen DNA-Sequenz, in einer pflanzlichen Zelle zu bewirken und welcher einen Minimalpromotor und mindestens ein erfindungsgemäßes cis-regulatorisches Element umfasst. Ein einzelnes erfindungsgemäßes cis-regulatorisches Element in einem solchen chimären Promotor allein ist bereits in der Lage eine signifikante Pathogen- und/oder Elizitorinduzierbarkeit zu vermitteln. So reicht dieses eine cis-regulatorische Element schon aus, um damit in Kombination mit einem Minimalpromotor einen Pathogen-/Elizitor-responsiven chimären Promotor zu konstruieren.

Vorzugsweise ist ein solcher chimärer Promotor, enthaltend als cis-regulatorische Elemente lediglich ein oder mehrere erfindungsgemäße cis-regulatorische Elemente, nur Pathogen und/oder Elizitor-responsiv, d.h. dieser Promotor ist nicht oder nur in einem geringen Ausmaß durch andere Stimuli wie abiotischen Stress induzierbar. Die Induktion eines solchen chimären Promotors umfassend ein oder mehrere erfindungsgemäße cis-regulatorische Elemente ist nach Pathogen/Elizitor-Kontakt mindestens 2-fach, bevorzugt mindestens 10-fach oder besonders bevorzugt mindestens 25-fach höher als die Induktion ohne Pathogen/Elizitor-Kontakt (Hintergrundaktivität).

In einer bevorzugten Ausgestaltung erfolgt die induzierte Expression nur lokal begrenzt auf den Infektionsort, d.h. in einem vergleichbarem oder in einem geringeren Ausmaß wie dies bei der kontrollierten Expression von natürlichen PR-Genen stattfindet. Besonders bevorzugt findet die Transkriptionsaktivierung kontrolliert durch einen chimären Promotor der vorliegenden Erfindung lediglich in den Zellen statt, die mit dem Pathogen oder dem pathogenen Elizitor in Kontakt kommen. Es kann jedoch auch aufgrund von Zell-Zell-Interaktionen eine Transkriptionsaktivierung in Zellen stattfinden, welche die Infektionsstelle(n) umgeben.

Chimäre Promotoren der vorliegenden Erfindung sind jedoch nicht beschränkt auf solche, die ausschließlich Pathogen-responsiv sind. Durch Kombination mit weiteren regulatorischen Elementen kann die induzierte Expression weiter spezifiziert werden, z.B. durch Kombintion mit einem cis-regulatorisches Element, das beispielsweise Gewebespezifität, Lagerungsinduzierbarkeit, Kälte- oder Hitzeinduzierbarkeit oder einer spezifischen Aktivität in bestimmten Entwicklungsstadien. Chimäre Promotoren der Erfindung können auch mindestens eine Kombination aus mindestens zwei cis-regulatorischen Elementen umfassen, wobei diese mindestens eine Kombination mindestens ein erfindungsgemäßes cis-regulatorischen Element umfasst. Als weitere cis-regulatorische Elemente in der Kombination können auch bekannten Pathogen-/Elizitor-Induzierbarkeit vermittelnde cis-regulatorische Elemente wie W-Box, S-Box oder D-Box (siehe WO 00/29592) zur Konstruktion eines chimären Promotors verwendet werden.

Zudem schließt die Erfindung auch einen chimären Promotor mit ein, der ein oder mehrere Monomere und/oder ein oder mehrere Multimere der erfindungsgemäßen cis-regulatorischen Elemente umfassen. Bevorzugte multimere Formen sind Dimere und Tetramere. Monomere für sich oder einzelne Monomere innerhalb eines Mutimers können unterschiedliche Orientierungen aufweisen, d.h. sie können beispielsweise komplementär angeordnet sein. Erfindungsgemäße cis-regulatorische Elemente einer multimeren Form können funktionell miteinander verknüpft sein, d.h. in multimerer Form zeigen sie eine synergistische oder antagonistische Wirkung beispielsweise auf die Bindekapazität des Transkriptionsfaktors, welcher unter anderem das charakteristische Kernsequenzmotiv einer bestimmten Motivgruppe erkennt. So schließt die Erfindung ebenfalls einen chimären Promotor mit ein, welcher geeignet ist, induziert durch eine Pathogeninfektion oder eine Behandlung mit einem pathogenen Elizitor eine Expression eines operativ verknüpften Nukleinsäuremoleküls von Interesse in einer pflanzlichen Zelle zu bewirken und welcher einen Minimalpromotor und mindestens zwei erfindungsgemäße cis-regulatorische Elemente umfasst, wobei die mindestens zwei cis-regulatorische Elemente funktionell verknüpft in homo- und/oder heteromerer Form vorliegen können.

Die Induktion eines chimären Promotors umfassend mindestens ein Multimer der erfindungsgemäßen cis-regulatorischen Elemente ist nach Pathogen/Elizitor-Kontakt mindestens 2-fach, bevorzugt mindestens 10-fach oder besonders bevorzugt mindestens 25-fach höher als die Induktion ohne Pathogen/Elizitor-Kontakt (Hintergrundaktivität).

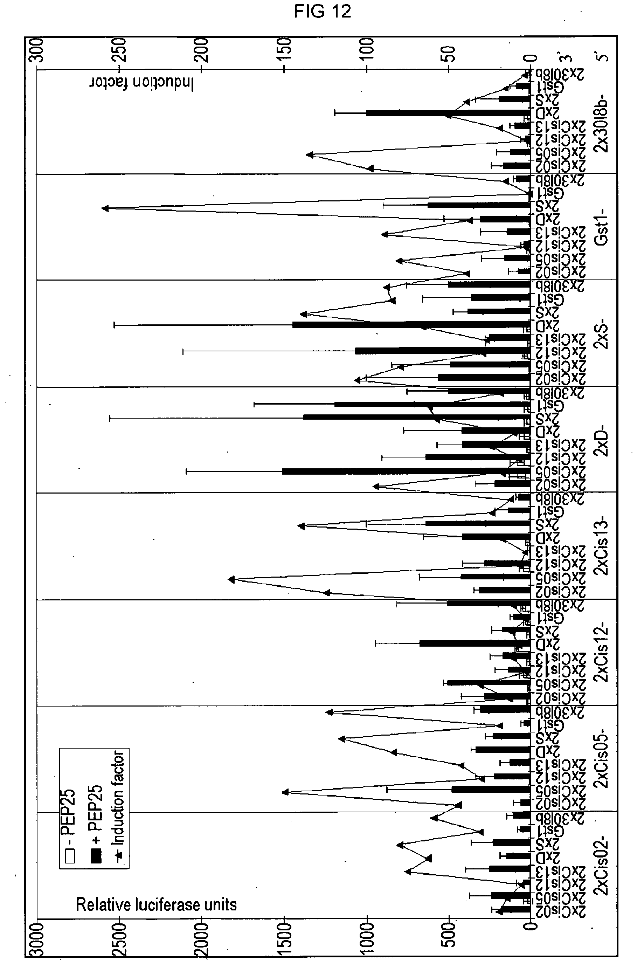

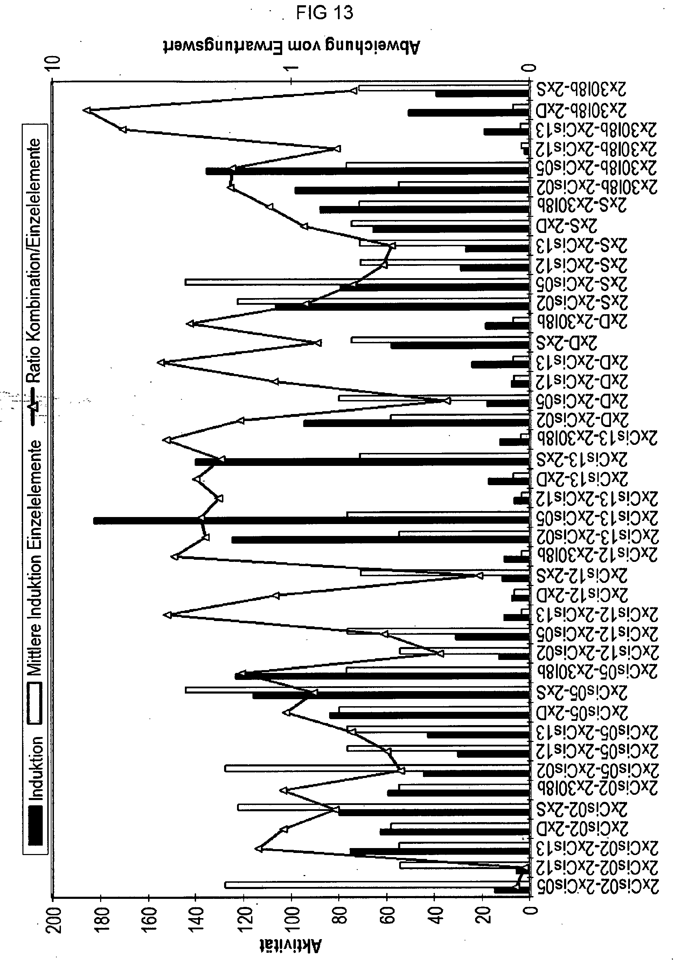

In einem bevorzugten Ausführungsbeispiel der chimären Promotoren der vorliegenden Erfindung beträgt der Abstand vom Minimalpromotor und zum ersten stromaufwärts befindlichen erfindungsgemäßen cis-regulatorischen Element zwischen 0 und 300 Basenpaaren, bevorzugt zwischen 0 und 70 Basenpaaren und besonders bevorzugt weniger als 10 Basenpaaren. Zusätzlich oder alternativ beträgt der Abstand zwischen zwei gleichen Monomeren der erfindungsgemäßen cis-regulatorischen Elemente in einer multimeren Form bevorzugt 0 bis 10 Basenpaare. Vorzugsweise sind zwei separate Multimere in einem chimären Promotor der Erfindung durch etwa 0 bis 50 Basenpaare getrennt.
Spezifische Kombinationen von erfindungsgemäßen cis-regulatorischen Elementen mit anderen erfindungsgemäßen cis-regulatorischen Elementen oder mit anderen bekannten regulatorischen Elementen oder Fragmenten wie S-Box, D-Box oder Gst1 zeigten in den experimentellen Analysen eine vorteilhaft und überraschende Wirkung im Hinblick auf Promotoreigenschaften wie eine geringe Hintergrundaktivität oder eine besonders spezifische oder eine besonders starke Induzierbarkeit (bis zum 183-fach 2xCis13-2xCis05). Dabei zeigten einige Kombinationen eine synergistische Wirkung hinsichtlich einer bestimmten Promotoreigenschaft wie beispielsweise hinsichtlich des Induktionsfaktors (vergleiche z.B. 2xCis13-2xCis05 in Petersilie, Figuren 12 und 13), während einige Kombinationen zwar eine antagonistische Wirkung beispielsweise hinsichtlich des Induktionsfaktor hatten, dabei aber eine besonders niedrige Hintergrundaktivität entwickelten (z.B. 4xCis05-2xD in Zuckerrübe). Die Erfindung schließt darüber hinaus sämtliche Kombination und Kombinationsmöglichkeiten der erfindungsgemäßen cis-regulatorischen Elemente mit sich selbst und mit bekannten cis-regulatorischen Elementen ein, die eine vorteilhafte synergistische oder antagonistische Wirkung auf die Induzierbarkeit des Promotors haben. Vorteilhafte Kombinationen sind solche, welche ausgewählt werden können aus folgender Gruppe: 4x sCis05, 4x 20u_M1_S1, 4x 27G-8_M1_S1, 4x 38M_M1_S1, 4x 18H_M2_S3, 4x 18H_M2_S1, 4x GG13_M1_S2, 4x 21S_M3_S1, 4x 30I-8_M1_S2, 2x Cis02 - 2x Cis02, 2x Cis02 - 2x Cis05, 2x Cis02 - 2x Cis12, 2x Cis02 - 2x Cis13, 2x Cis02 - 2x D, 2x Cis02 - 2x S, 2x Cis02 - Gst1, 2x Cis02 - 2x 30I-8_M1_S2, 2x Cis05 - 2x Cis02, 2x Cis05 - 2x Cis05, 2x Cis05 - 2x Cis12, 2x Cis05 - 2x Cis13, 2x Cis05 - 2x D, 2x Cis05 - 2x S, 2x Cis05 - Gst1, 2x Cis05 - 2x 30I-8_M1_S2, 2x Cis12 - 2x Cis02, 2x Cis12 - 2x Cis05, 2x Cis12 - 2x Cis12, 2x Cis12 - 2x Cis13, 2x Cis12 - 2x D, 2x Cis12 - 2x S, 2x Cis12 - Gst1, 2x Cis12 - 2x 30I-8_M1_S2, 2x Cis13 - 2x Cis02, 2x Cis13 - 2x Cis05, 2x Cis13 - 2x Cis12, 2x Cis13 - 2x Cis13, 2x Cis13 - 2x D, 2x Cis13 - 2x S, 2x Cis13 - Gst1, 2x Cis13 - 2x 30I-8_M1_S2, 2x D - 2x Cis02, 2x D - 2x Cis05, 2x D - 2x Cis12, 2x D - 2x Cis13, 2x D - 2x 30I-8_M1_S2, 2x S - 2x Cis02, 2x S - 2x Cis05, 2x S - 2x Cis12, 2x S - 2x Cis13, 2x S - 2x 30I-8_M1_S2, Gst1 - 2x Cis02, Gst1 - 2x Cis05, Gst1 - 2x Cis12, Gst1 - 2x Cis13, Gst1 - 2x 30I-8_M1_S2, 2x 30I-8_M1_S2 - 2x Cis02, 2x 30I-8_M1_S2 - 2x Cis05, 2x 30I-8_M1_S2 - 2x Cis12, 2x 30I-8_M1_S2 - 2x Cis13, 2x 30I-8_M1_S2 - 2x D, 2x 30I-8_M1_S2 - 2x S, 2x 30I-8_M1_S2 - Gst1 und 2x 30I-8_M1_S2 - 2x 30I-8_M1_S2. Besonders vorteilhafte Kombinationen mit überraschender Wirkung im Hinblick auf Induktionsfaktor und Aktivität sind in der Tabelle 3 aufgeführt.
Weiterhin bezieht sich die vorliegende Erfindung auch auf chimäre Promotoren, die mindestens eine der oben genannten Kombinationen von cis-regulatorischen Elementen umfassen.

In einer bevorzugten Ausgestaltung der chimären Promotoren der vorliegenden Erfindung stammt der Minimalpromotor beispielsweise aus einem CaMV35S-Promotor, für monocotyledone Pflanzen beispielsweise aus dem Weizen-TaPal-Promotor (SEQ ID NO: 39), dem Mais-ZmUbiquitin-Promotor (SEQ ID NO: 40) oder dem Reis-OsGns1-Promotor (SEQ ID NO: 38), oder für dicotyledone Pflanzen aus bekannten Minimalpromotoren (WO 07/147395). Darüber hinaus können aber auch Minimalpromotoren aus anderen Quellen zur Konstruktion eines chimären Promotors im Sinne der vorliegenden Erfindung verwendet werden.

Ein chimärer Promotor der vorliegenden Erfindung erfüllt in jedem Fall die wesentlichen Erfordernisse, die an die stringente Expressionsregulation eines Transgens in einem gentechnischen Ansatz, z.B. zur Herstellung einer Pathogen-/Krankheits-resistenten Pflanze, gestellt werden. Das Transgen ist ein mit dem chimären Promotor operativ verknüpftes Nukleinsäuremolekül von Interesse, z.B. eine heterologe DNA-Sequenz, welche beispielsweise für ein Resistenzgen (R-Gen), ein autoaktiviertes Resistenzgen, ein Avirulenzgen, einen anderen Effektor, ein Protein, das toxisch gegenüber mindestens einem Pathogen ist, Signaltransduktionskomponenten, ein Protein welches an der Synthese von Phytoalexinen, eine doppelsträngige RNA für die Bildung von gegen einen Pathogen gerichteten siRNAs oder ein antimikrobielles Peptid kodiert. Zudem zeigten zahlreiche Versuche an Petersilie (*Petroselinum erispum*), *Arabidopsis thaliana,* Weizen (*Triticum sp.*) und Zuckerrübe (*Beta vulgaris*)*,* dass ein chimärer Promotor der vorliegenden Erfindung speziesübergreifend funktioniert und verwendet werden kann (siehe zum Beispiele 4xCis05 in Petersilie, Zuckerrübe und Weizen).

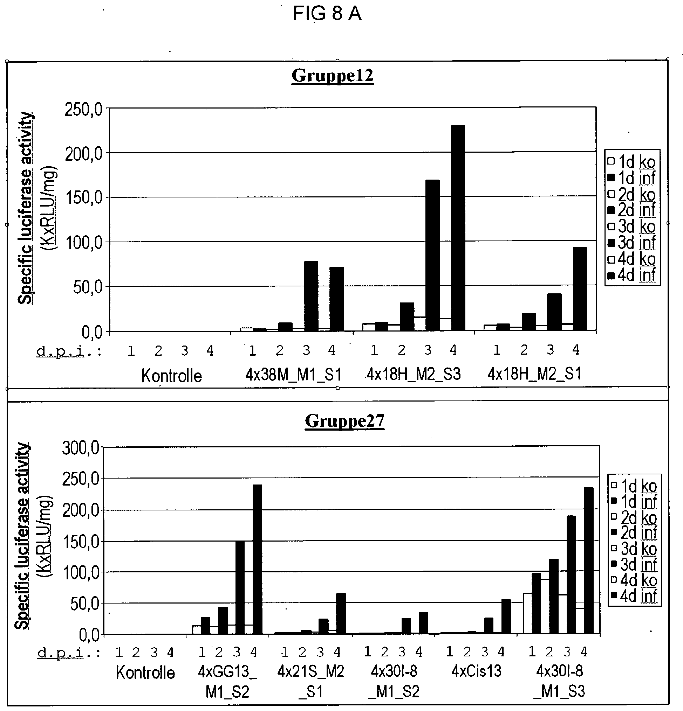

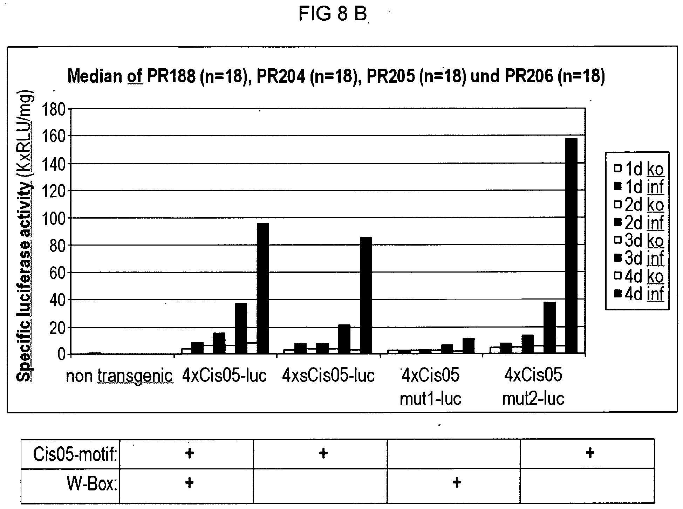

Die Definition eines Familienmotivs für die Motivgruppe 27 eröffnet einem Fachmann neue Möglichkeiten bei der Konstruktion von chimären Promotoren der vorliegenden Erfindung. Die beobachteten Aktivitäten der verschiedenen Mitglieder der Motivgruppen 27 und 12 (Fig. 8) zeigen, dass die flankierenden Sequenzbereiche zur bedarfgerechten Feinabstimmung des gewünschten Expressionslevels herangezogen werden können. Dies gilt auch für eine spezies-abhängige Abstimmung des Expressionslevels. Das Familienmotiv gibt die Variationsmöglichkeiten einzelner Basen in diesen flankierenden Bereichen wieder und lehrt damit dem Fachmann, inwieweit die flankierenden Bereiche modifiziert werden können. Zudem erhält der Fachmann aus dem Familienmotiv Informationen darüber wie stark einzelne Basenpositionen innerhalb des Familienmotivs konserviert vorliegen. Dabei ist davon auszugehen, dass die Modifikation einer stark-konservierten Base eine deutlichere Auswirkung auf die resultierenden Eigenschaften des chimären Promotors hat als eine schwach konservierte Base.

Weiterhin betrifft die Erfindung auch ein rekombinantes Gen, welches einen chimären Promotor der vorliegenden Erfindung umfasst. Vorzugsweise ist das rekombinante Gen derart gestaltet, dass der chimäre Promotor operativ verknüpft ist mit einem Nukleinsäuremolekül, z.B. einer heterologen DNA-Sequenz. Eine solche heterologe DNA-Sequenz kodiert insbesondere für ein (Poly)petid, ein cytotoxisches Protein (wie Bt-Toxin, Avirulenzprotein oder Enzyme wie Glucoseoxidasen, welche reaktive Sauerstoffspezies erzeugen), einen Antikörper, eine Antisinn-RNA, eine Sinn-RNA, einen Transkriptionsfaktor, eine Protease, eine Nuklease, eine Lipase, einen Enzyminhibitor oder einen messbaren Marker (wie Luziferase, GFP oder β-Galactosidase). Letztgenannte Marker sowie andere aus dem Stand der Technik bekannte Marker können in Testsystemen verwendet werden, um die Pathogenspezifität eines chimären Promotors der vorliegenden Erfindung zu ermitteln oder Effektoren zu identifizieren, die eine Induktion des chimären Promotors bewirken oder hemmen.
Chimäre Promotoren der Erfindung können auch in RNAi-basierten Verfahren zum 'Gene Silencing' genutzt werden, wobei das operativ verknüpfte Nukleinsäuremolekül von Interesse beispielsweise eine Antisinn-RNA, eine Sinn-RNA oder eine doppelsträngige RNA (dsRNA) kodiert. Das RNA-Molekül kann dann eine kurze Nukleotidsequenz (im Allgemeinen mindestens 10 Nukleotide, bevorzugt mindestens 14 Nukleotide und optionell bis zu 100 oder mehr Nukleotide lang) darstellen, welche im Wesentlichen komplementär ist zu einer spezifischen mRNA-Sequenz und/oder einer DNA-Sequenz eines Gens von Interesse. Standardmethoden der RNAi-Technologie sind im Stand der Technik beschrieben. Prinzipiell ist es möglich die operativ verknüpfte kodierende Sequenz derart zu modifizieren, dass das Produkt der Translation in einem gewünschten Zellkompartiment wie Nukleus, endoplasmatisches Retikulum, Mitochondrium, Cytoplasma oder Vakuole oder auch extrazellulär (apoplastisch) lokalisiert wird. Hierfür geeignete Verfahren zur Modifikation sind dem Fachmann aus dem Stand der Technik bekannt (Gorlich, Science 271 (1996), 1513-1518; Hicks, Plant Physiol. 107 (1995), 1055-1058; Rachubinski, Cell 83 (1995), 525-528; Schatz, Science 271 (1996), 1519-1526; Schnell, Cell 83 (1995), 521-524; Verner, Science 241 (1988), 1307-1313; Vitale, BioEssays 14 (1992),151-160).

Ein rekombinantes Gen der vorliegenden Erfindung kann sowohl allein als auch als ein Teil eines Vektors genutzt werden. Demnach betrifft die vorliegende Erfindung auch einen Vektor, welcher den chimären Promotor dieser Erfindung oder das rekombinante Gen dieser Erfindung umfasst. Bevorzugt ist der Vektor ein pflanzlicher Expressionsvektor, welcher vorzugsweise weiterhin auch einen Selektionsmarker für Pflanzen umfasst. Beispiele für geeignete Marker sind bereits weiter oben aufgeführt. Verfahren zur Konstruktion solcher Vektoren sind aus dem Stand der Technik dem Fachmann bekannt, z.B. beschrieben in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.V. (1989).

Die vorliegende Erfindung betrifft zudem eine prokaryotische oder eine eukaryotische Wirtszelle, welche einen chimären Promotor, ein rekombinantes Gen oder einen Vektor gemäß der Erfindung umfasst, wobei der chimäre Promotor an sich oder als Teil des rekombinanten Gens oder als Teil des Vektors oder jeweils ein Teil des chimären Promotors wie ein cis-regulatorisches Element heterolog ist zu der prokaryotischen oder eukaryotischen Wirtszelle, also beispielsweise von einer Zelle oder einem Organismus mit einem anderen genetischen Hintergrund stammt, oder homolog ist zu der prokaryotischen oder eukaryotischen Wirtszelle, dann aber in einer unterschiedlichen genetischen Umgebung lokalisiert ist und sich so von dem natürlicherweise vorhandenen chimären Promotor oder dessem Teil unterscheidet.
Der chimäre Promotor, das rekombinante Gen oder der Vektor gemäß der Erfindung können entweder in das Genom der prokaryotischen oder eukaryotischen Wirtszelle intergriert sein, bevorzugt stabil integriert, oder können in einer extrachromosomalen Form wie einem Plasmid in der Zelle verbleiben.

Weiterhin stellt die Erfindung ein Verfahren zur Herstellung einer transgenen Pflanze zu Verfügung, umfassend das Einbringen eines chimären Promotors, eines rekombinanten Gens oder eines Vektors gemäß der vorliegenden Erfindung in mindestens eine Zelle der Pflanze oder umfassend das Einbringen eines chimären Promotors, eines rekombinanten Gens oder eines Vektors gemäß der vorliegenden Erfindung in mindestens eine pflanzliche Zelle in einer Zellkultur, aus welcher anschließend die transformierte bzw. transgene Pflanze regeneriert wird. Vorzugsweise wird der chimäre Promotor, das rekombinante Gen oder der Vektors in das Genom der Pflanze integriert, besonders bevorzugt stabil integriert. Für die Expression des Nukleinsäuremoleküls von Interesse unter der Kontrolle eines chimären Promotors gemäß der vorliegenden Erfindung in Pflanzenzellen, kann das Nukleinsäuremolekül mit weiteren regulatorischen Sequenzen wie am 3'-Ende einem Poly-A-Schwanz verbunden sein. Verfahren zum Einbringen von Genen oder genetischem Material in eine Pflanze oder in eine pflanzliche Zelle sowie Verfahren zur Regeneration von transformierten pflanzliche Zellen sind aus dem Stand der Technik bekannt, beispielsweise *Agrobacterium tumefaciens-* oder *Agrobacterium rhizogenes*-vermittelte Transformation von pflanzlichen Zellen oder Geweben mit T-DNA, die Protoplastenfusion, Injektion, Elektroporation, Vakuuminfiltration oder biolistische Methoden. Ebenso sind Verfahren zur Präparation von geeigneten Vektoren zum Einbringen von Genen oder genetischem Material in eine Pflanze oder in eine pflanzliche Zelle dem Fachmann geläufig (Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.V. (1989)).

In einer alternativen Ausführungsform kann eine pflanzliche Zelle derart modifiziert werden, dass diese pflanzliche Zelle ein endogenes Gen unter der Kontrolle eines chimären Promotors gemäß der vorliegenden Erfindung oder unter der Kontrolle eines durch erfindungsgemäße cis-regulatorische Elemente modifizierten nativen Promotors des endogenen Gens exprimiert. Das Einbringen eines solchen chimären Promotors, welcher natürlicherweise nicht die Expression eines bestimmten Gens oder einer bestimmten genomischen Sequenz reguliert, an die gewünschte Stelle im Pflanzengenom oder das Einbringen von erfindungsgemäßen cis-regulatorischen Elementen in einen nativen Promotor, kann über bekannte Standardverfahren beispielsweise durch gezielte Integration ('gene targeting') mittels Zink-Finger-Nukleasen (Urnov et al., Nature Reviews 2010_Genome editing with engineered zinc finger nucleases; Townsend et al., Nature 2009_High-frequency modification of plant genes using engineered zinc-finger nucleases) oder TAL-Effektor-Nukleasen (WO 2010/079430; WO 2011/072246) erfolgen. Die Modifikation eines nativen Promotors eines endogenen Gens meint dabei auch das zusätzliche Einbringen eines erfindungsgemäßen cis-regulatorischen Elements in den nativen Promotor, welcher bereits ein erfindungsgemäße cis-regulatorischen Element natürlicherweise aufweist, also eine Multimerisierung vorhandener cis-regulatorischer Elemente. Ein solcher modifizierter Promotor kann im Vergleich mit der nativen Version geänderte Eigenschaften hinsichtlich beispielsweise Spezifität, Expressionslevel oder Hintergrundaktivität aufweisen.
Aus den modifizierten pflanzlichen Zellen können mit Hilfe bekannter Verfahren modifizierte Pflanzen regeneriert werden.

Somit betrifft die Erfindung weiterhin beschriebene transgene (transformierte) Pflanzen, welche mit einem chimären Promotor, einem rekombinanten Gen einen Vektor gemäß vorliegender Erfindung transformiert wurden, und beschriebene Pflanzen, modifiziert durch das Einbringen von mindestens einem erfindungsgemäßen cis-regulatorischen Element oder von einem chimären Promotor gemäß der Erfindung. Transgene bzw. modifizierte Pflanzen können aus jeder gewünschten Pflanzenspezies stammen. Sie können monokotyledone, dikotyledone oder angiosperme Pflanzen sein, vorzugsweise gehören sie zu Pflanzenspezies von agrarwirtschaftlichem oder hortikulturellem Interesse, beispielsweise Mais, Reis, Weizen, Roggen, Gerste, Hafer, Sorghum, Kartoffeln, Ölraps, Sonnenblume, Sojabohne, Baumwolle oder Zuckerrübe.
In einer bevorzugten Ausgestaltung der Erfindung ist eine transgene oder modifizierte Pflanze resistent oder zeigt eine gesteigerte Resistenz gegenüber einem oder einer Vielzahl von Pathogenen im Vergleich zu einer nicht-transgenen oder nicht-modifizierten Pflanze derselben Spezies (Wildtyp).

Von der vorliegenden Erfindung sind weiterhin ein Pflanzenteil, ein Pflanzengewebe, eine Pflanzenzelle oder ein Samen der transgenen und der modifizierten Pflanze der vorliegenden Erfindung mit eingeschlossen, wobei dieser Pflanzenteil, dieses Pflanzengewebe, diese Pflanzenzelle oder dieser Samen ebenfalls das in die Pflanze eingebrachte Transgen oder die eingebrachte Modifikation aufweisen.

Ausführungsformen der vorliegenden Erfindung werden in exemplarischer Weise mit Bezug auf die angehängten Figuren und Sequenzen beschrieben:

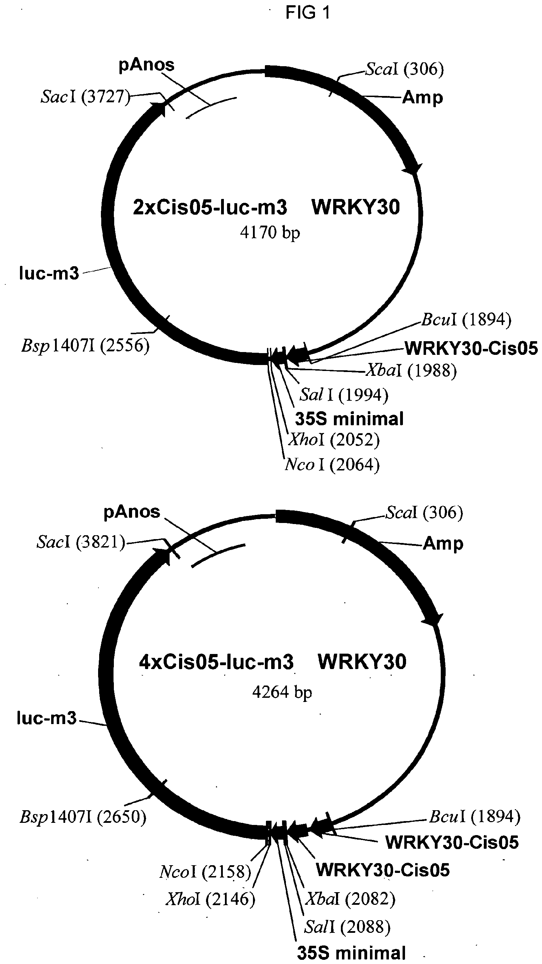

Fig 1: Als Beispiel für Klonierung der Einzelsequenzen als chimäre Promotoren sind die Plasmide mit 2xCis05 Element und dem multimerisierten 4xCis05 Element gezeigt. Die Plasmide leiten sich von pBT10-GUS (Sprenger and Weisshaar (2000): The Plant Journal 22, 1-8) ab. Der Aufbau der anderen Plasmide ist entsprechend. Amp: Ampicilin-Resistenz; WRKY30-Cis05: Doppelte Einzelsequenz Cis05. 35S-minimal: 35S-Minmalpromotor; Luc-m3: Luziferase-Reportergen; pAnos: Nos-terminator.

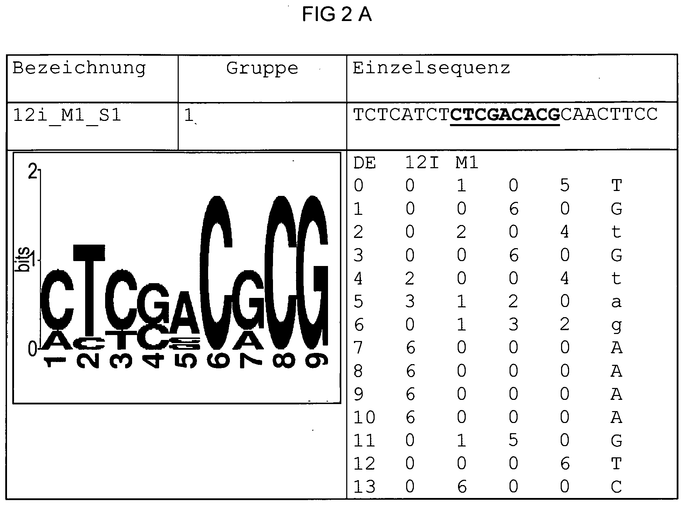

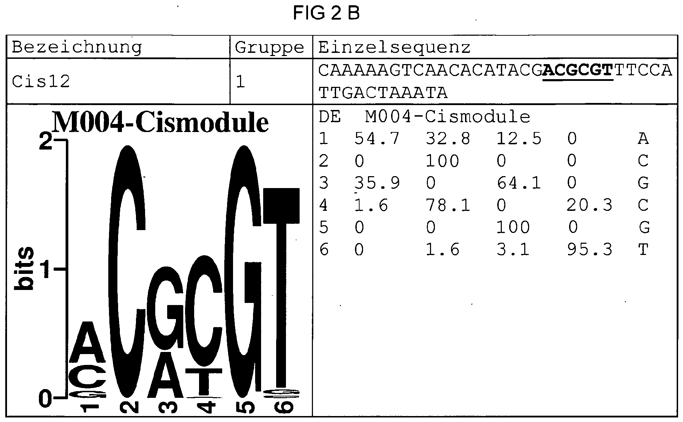

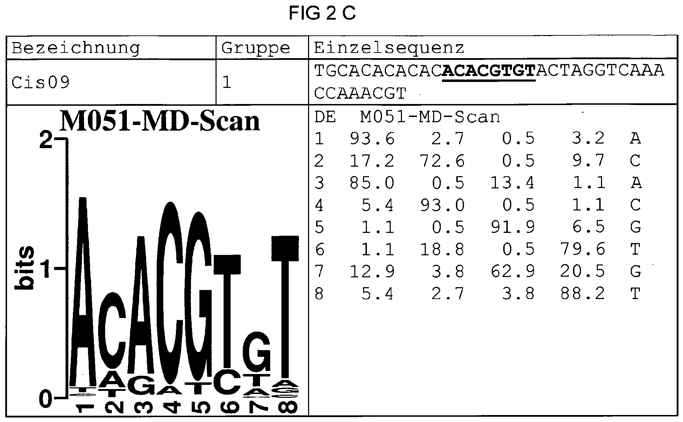

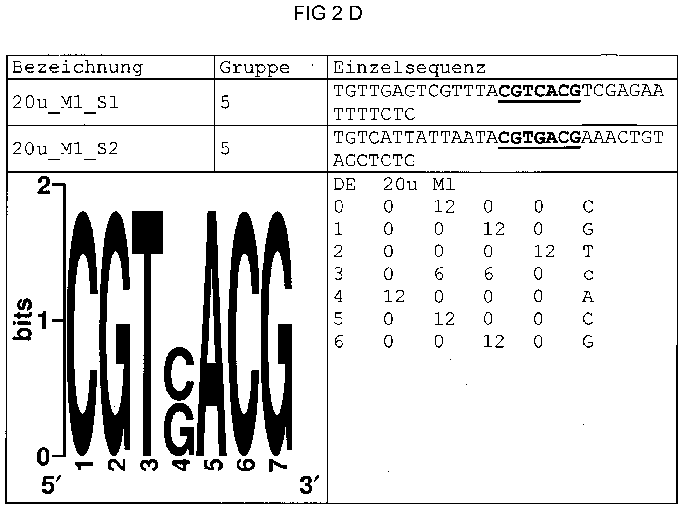

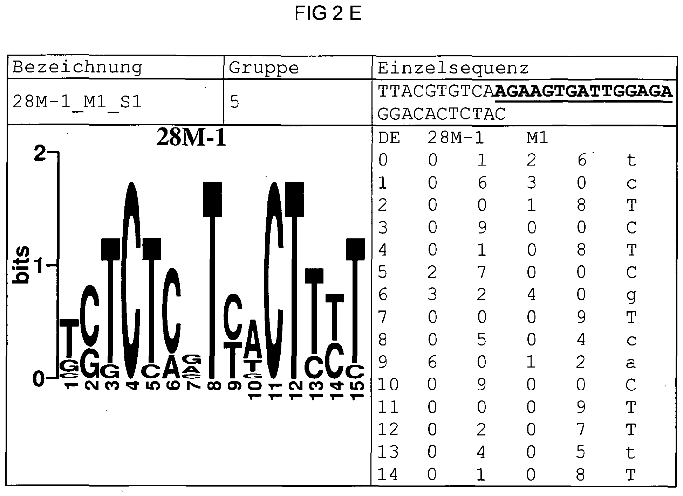

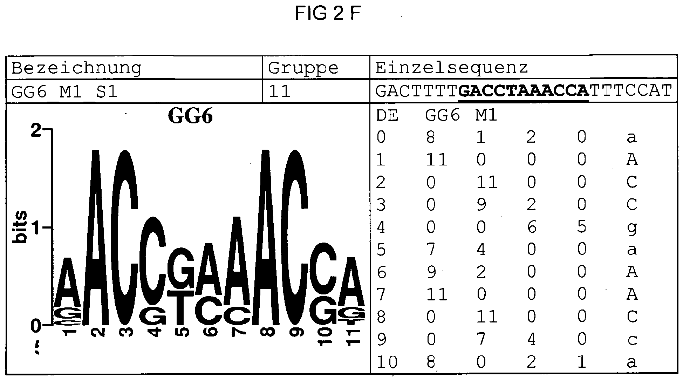

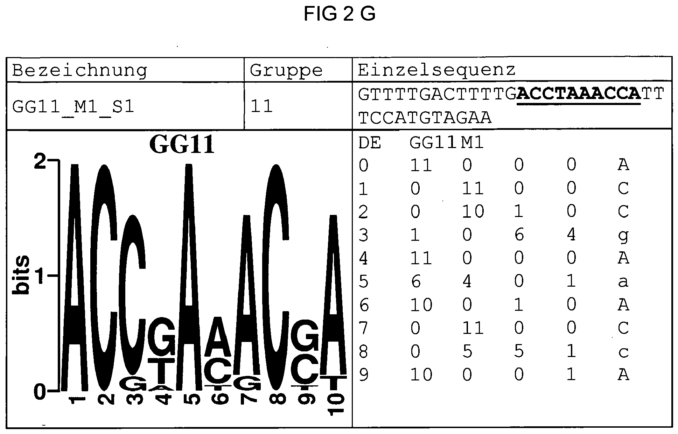

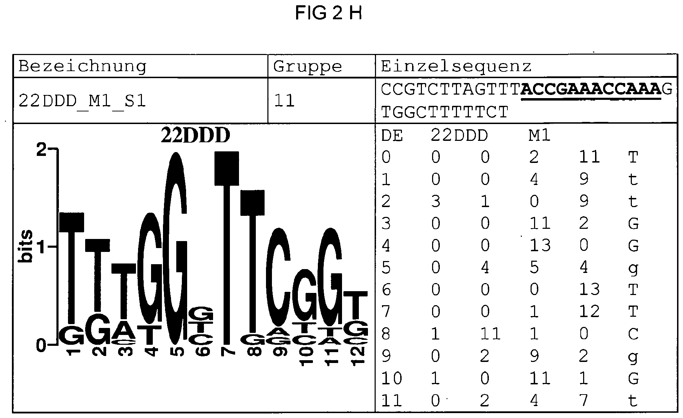

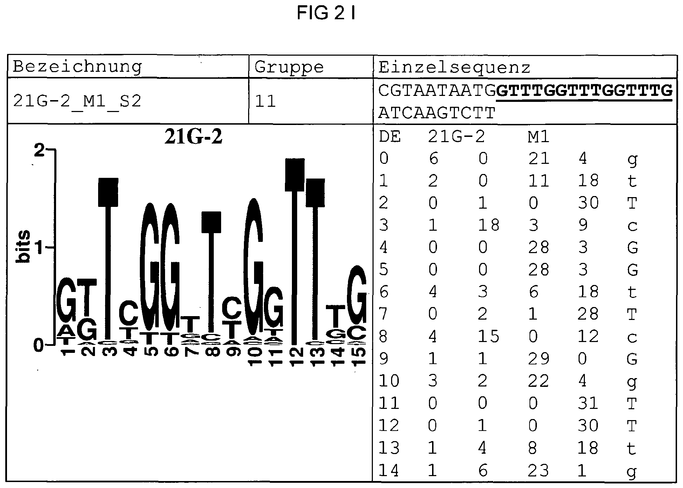

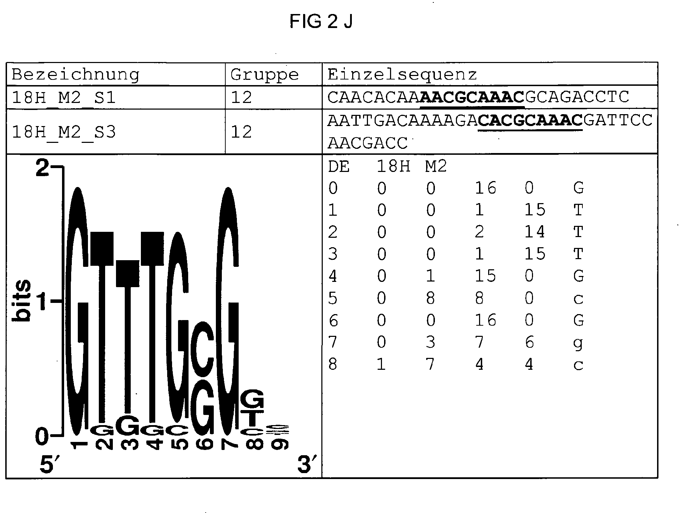

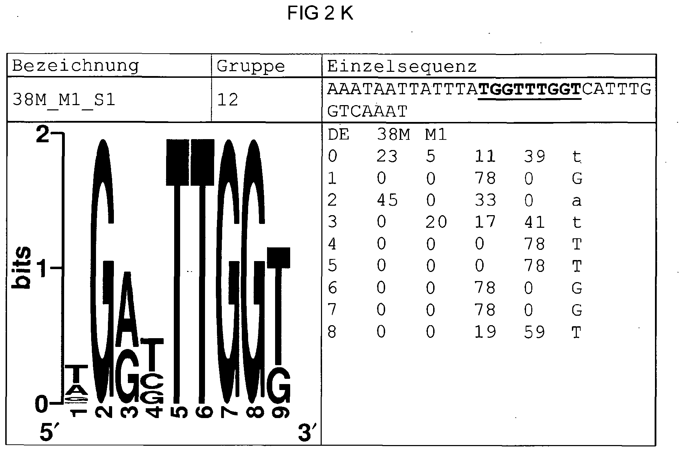

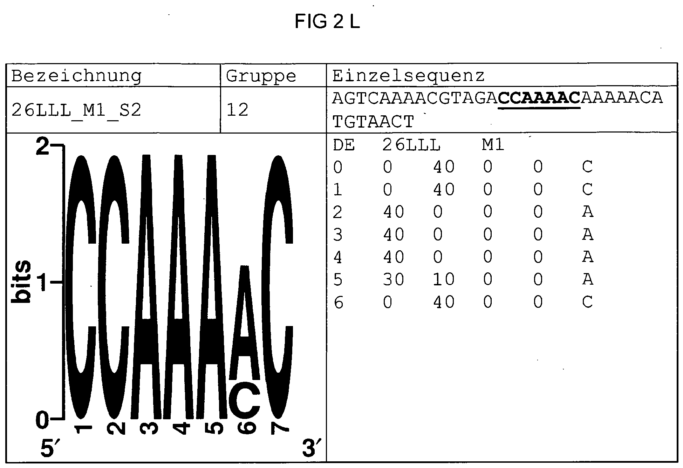

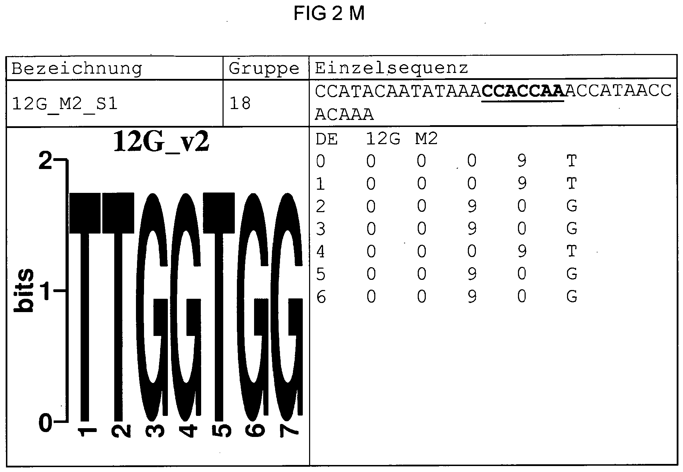

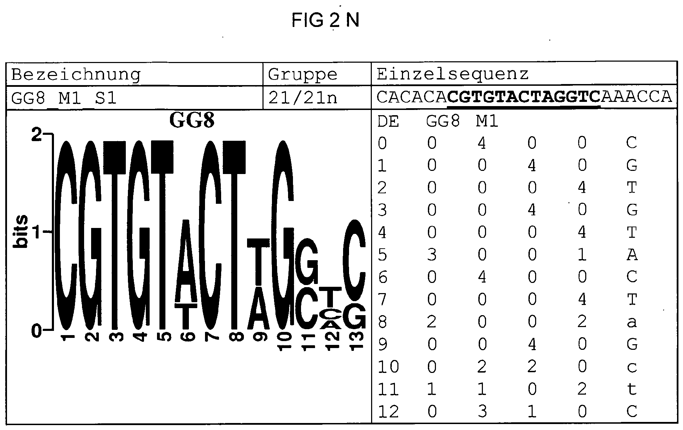

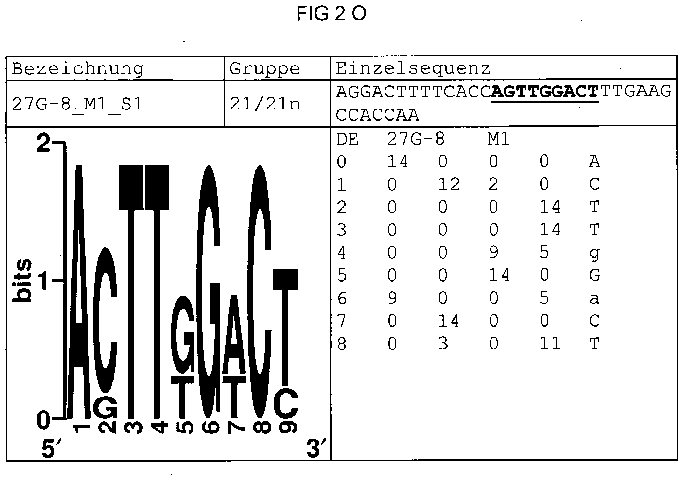

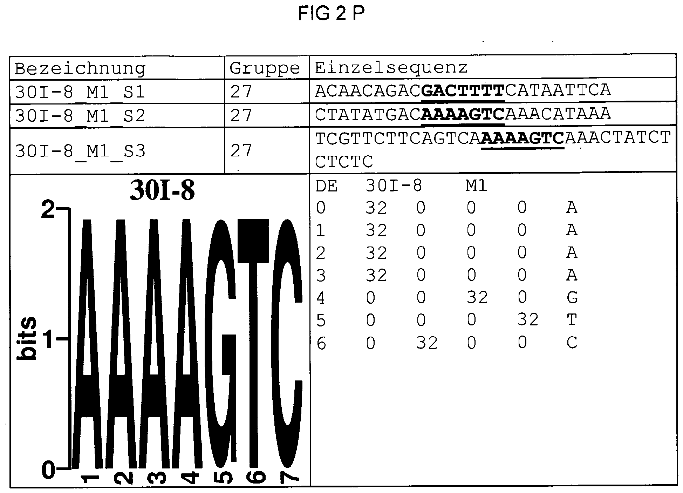

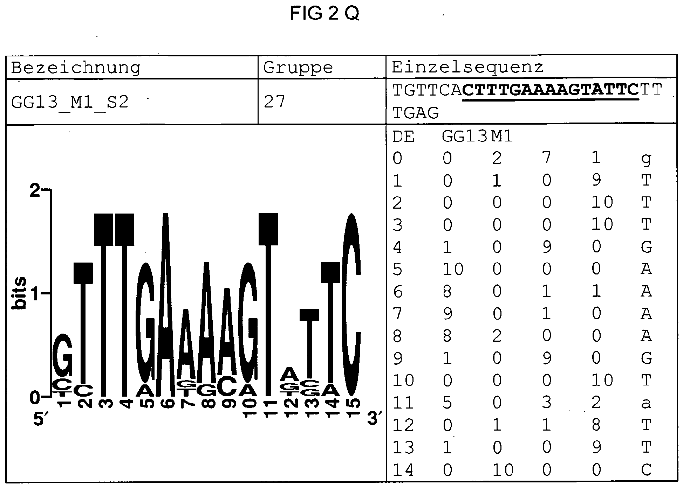

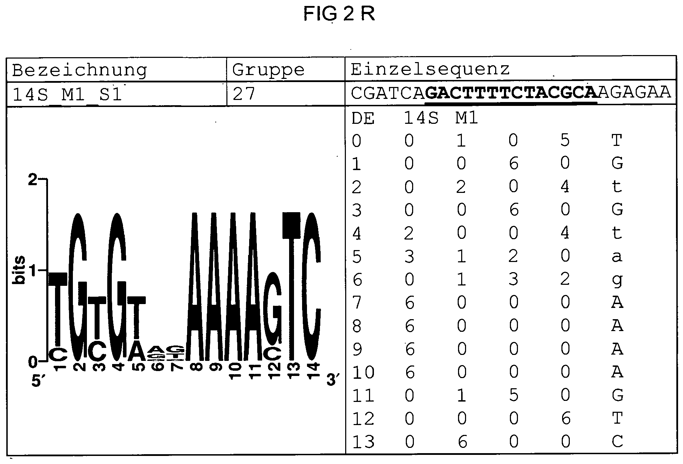

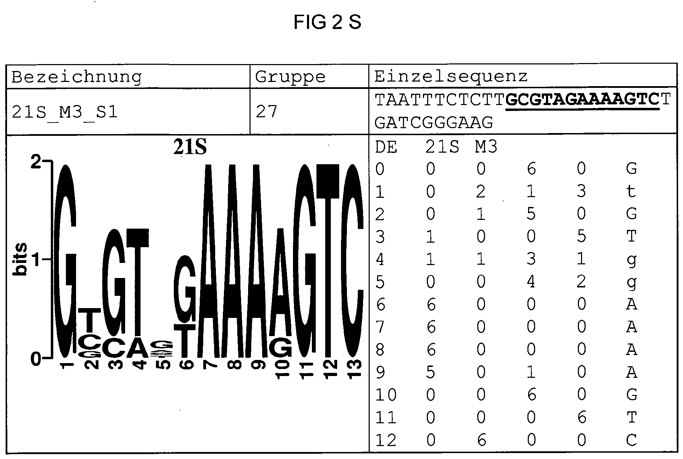

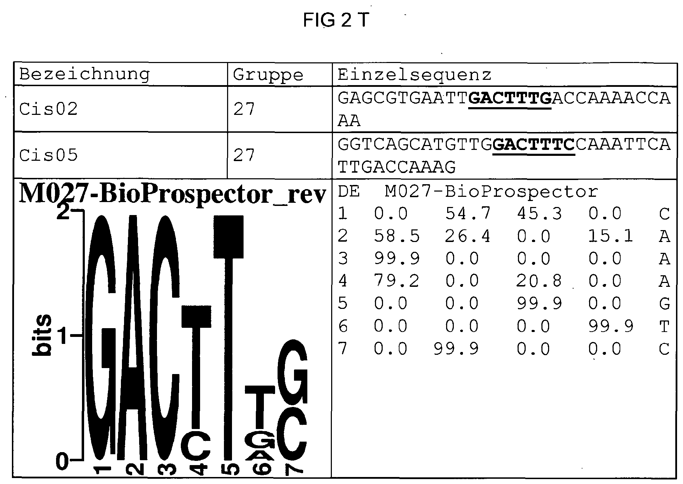

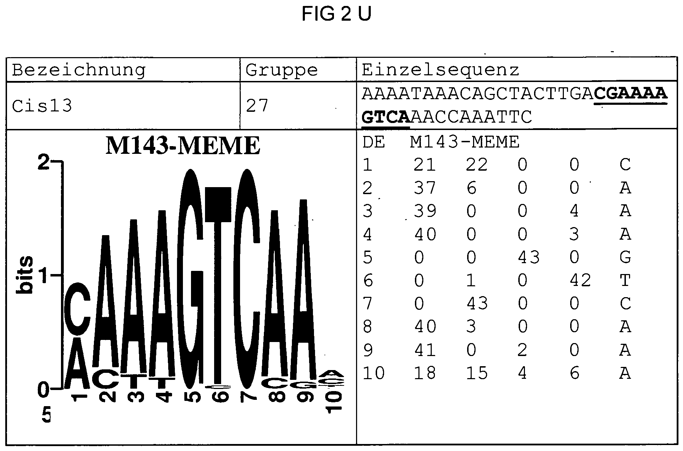

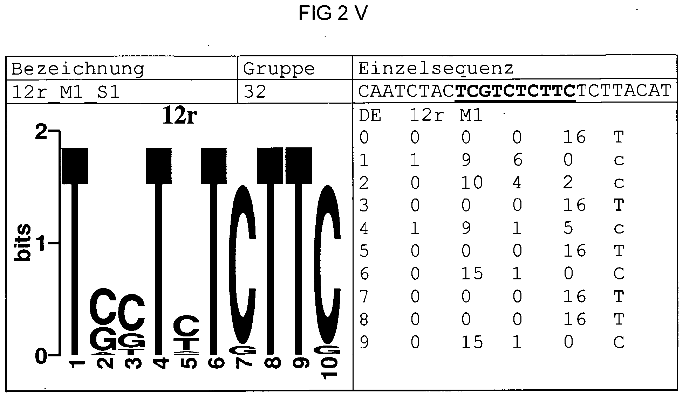

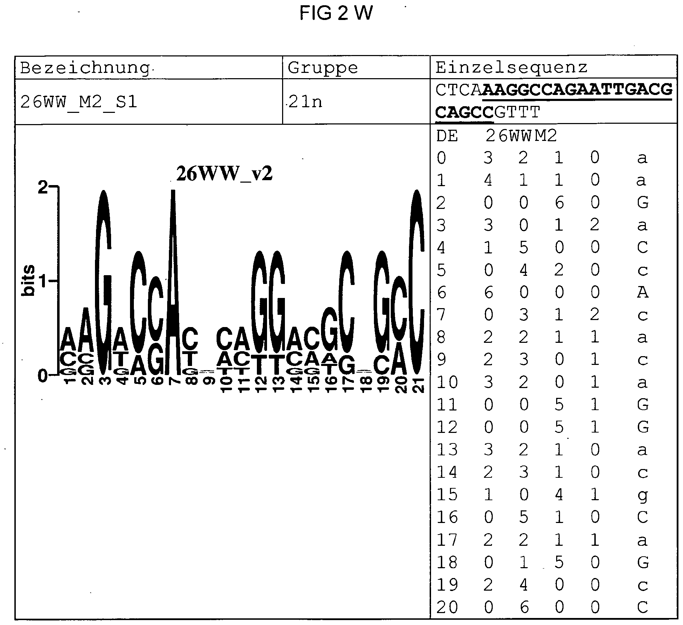

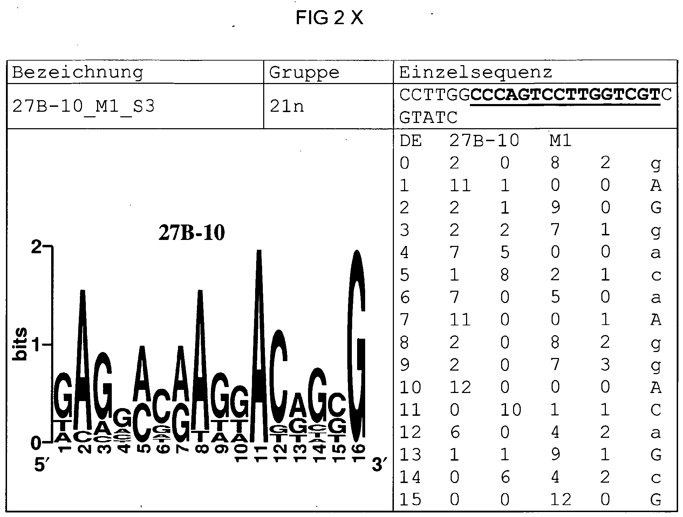

Fig 2: Spezies-übergreifend Pathogen-induzierbare Einzelsequenzen (A - X). In den oberen Zeilen sind jeweils die Bezeichnung der Einzelsequenz, die Motivgruppe, und die Einzelsequenzen selbst wiedergegeben. Die Kernsequenzen des Motivs, das zur Auswahl der Einzelsequenz geführt hat, ist fett geschrieben und unterstrichen. Darunter ist jeweils das Motivlogo des zugrundeliegenden bioinformatorisch identifizierten Motivs sowie dessen Matrize wiedergegeben. Sind mehrere Einzelsequenzen von einem Motiv getestet worden, so sind diese zusammengefasst. Figuren 2A-O und V-X dienen dem besseren Verständnis und sind nicht Teil der vorliegenden Erfindung.

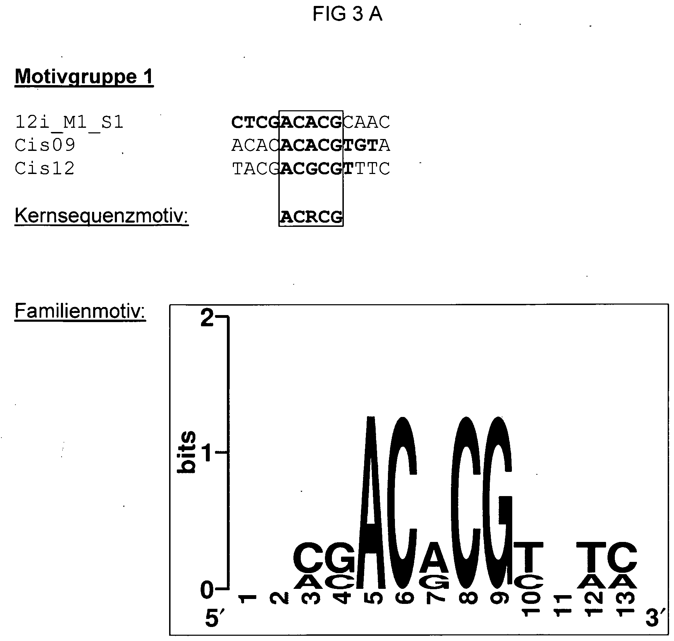

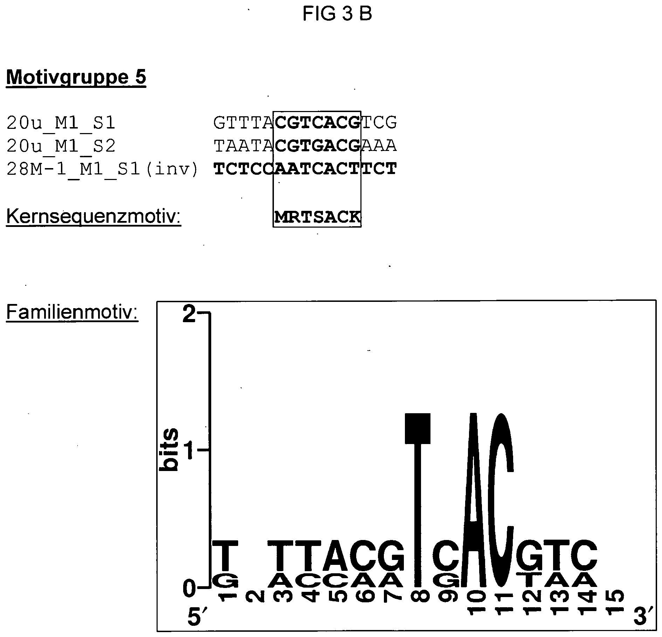

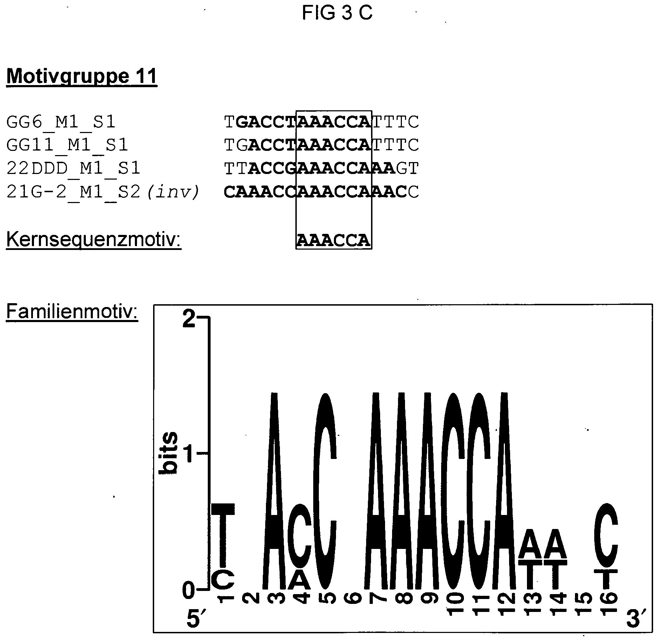

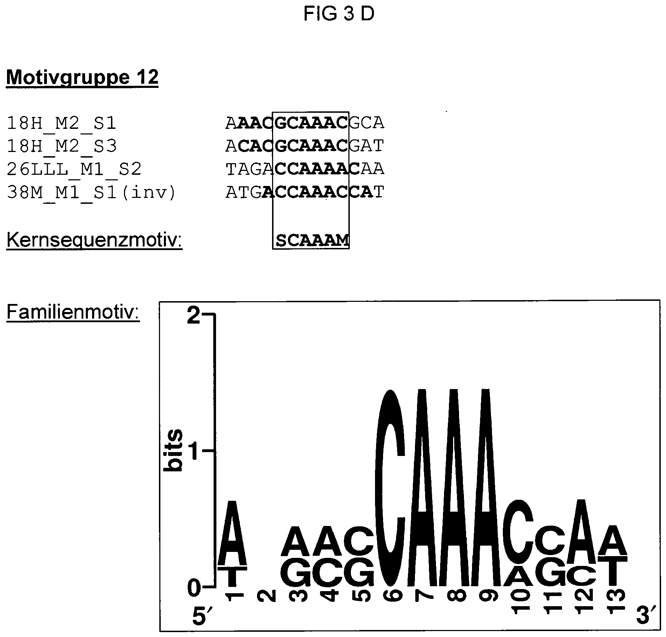

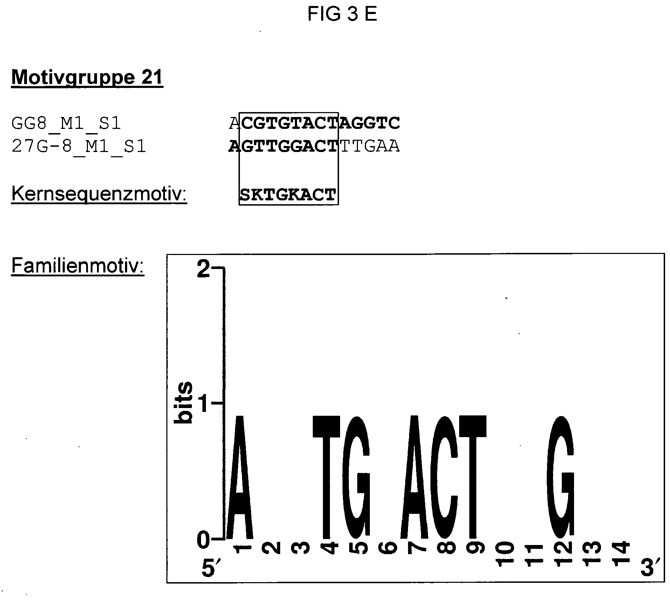

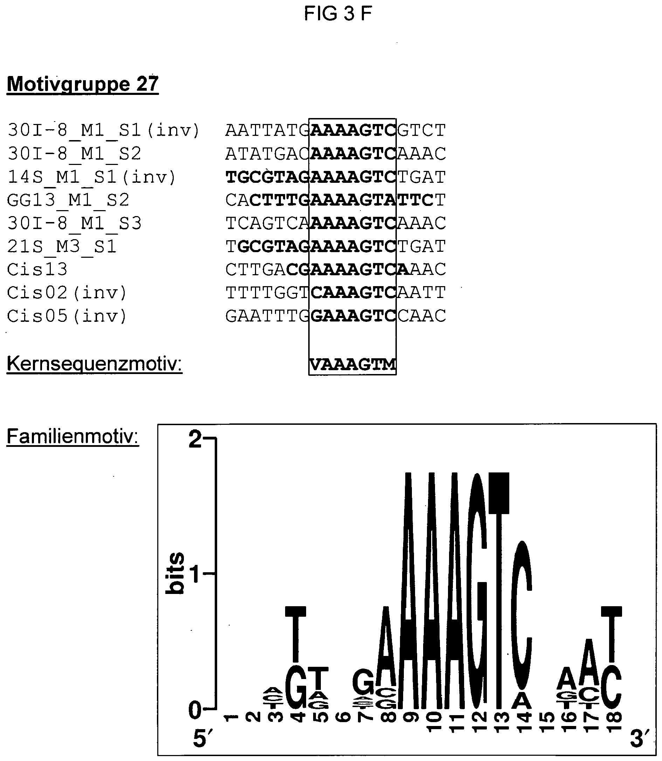

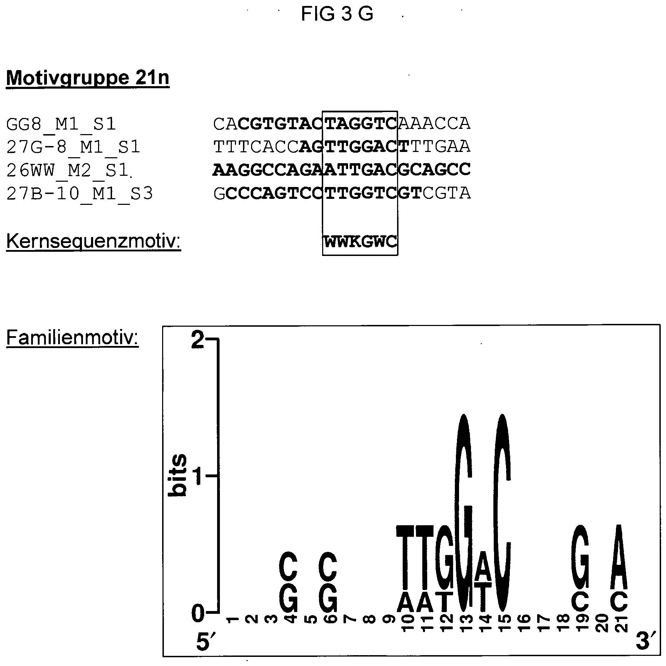

Fig 3: Zusammenfassung aller positiv getesteten Einzelsequenzen einer Motivgruppe und Darstellung der daraus abgeleiteten Sequenz- und Familienmotive (Fig. 3A - G). In der obersten Zeile ist die jeweilige Motivgruppe genannt. Darunter ist eine Alignment aller positiv getesteter Einzelsequenzen gezeigt, das mindestens die Kernsequenzen und, falls vorhanden, weitere konservierte Basen umfasst. Die Basen, aus denen sich das Kernsequenzmotiv ableitet, sind mit einem Rahmen versehen. Figuren 3A-E und G dienen dem besseren Verständnis und sind nicht Teil der vorliegenden Erfindung.

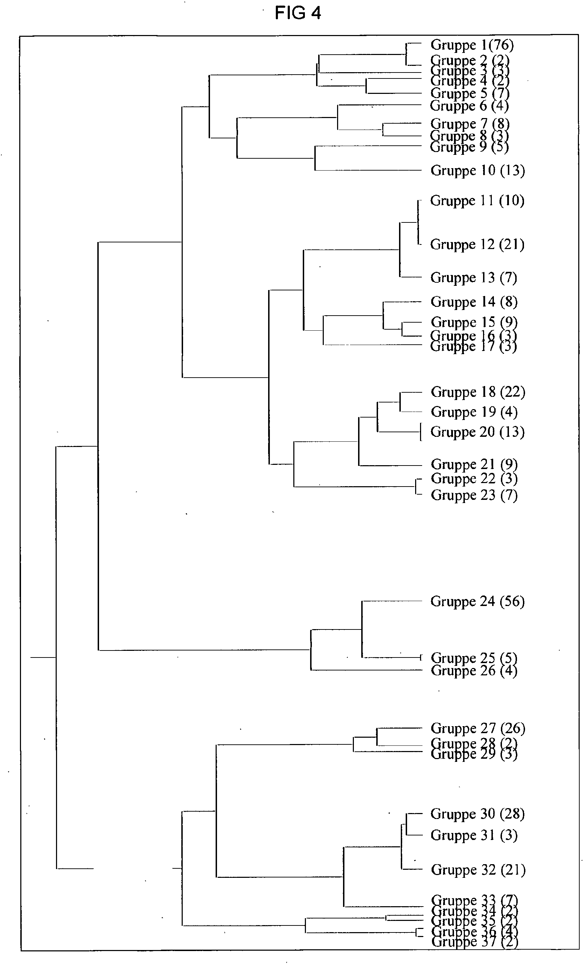

Fig 4: Phylogenetischer Stammbaum der identifizierten Motivgruppen. Der Stammbaum wurde durch eine Clusteranalyse mithilfe des Webservers STAMP erstellt. Hinter der Nummer der Motivgruppe ist in Klammern die Zahl der in der Motivgruppe enthaltenen Motive wiedergegeben.
Fig 5: A) Mutagenese der Cis05-Einzelsequenz. Die verwendete Sequenz enthält neben dem Cis05-Motive (Fett, unterstrichen) eine W-Box (fett und umrahmt). In beide Motive wurden Mutationen eingeführt. Mit den tetramerisierten mutierten Derivaten wurden wie beschrieben Petersilien-Assays auf Induktion durch das PAMP PEP25 durchgeführt. Die PAMP-induzierte Aktivität der chimären Promotoren wurde nach 4, 8 und 24 Stunden gemessen (rechte Seite). Mutationen in dem Cis05-Motiv (Cis05mut1) sowie in der W-Box (Cis05mut2) führen zu einem deutlichen Rückgang der induzierten Aktivität. Nur wenn beide Motive mutiert sind (Cis05mut1+2) kommt es zu einem vollständigen Verlust der Induzierbarkeit. B) sCis05 ist eine verkürzte Variante von Cis05, die nur das Cis05-Motiv, aber nicht mehr die W-Box enthält. Die PEP25-Induzierbarkeit von sCis05 und mutierten Derivaten wurde, wie in 5A beschrieben, getestet. Die beiden Balken stehen für zwei biologische Replikate (unabhängige Transformationen). Zur Orientierung ist unter sCis05-Derivaten der W-Box Konsensus dargestellt.

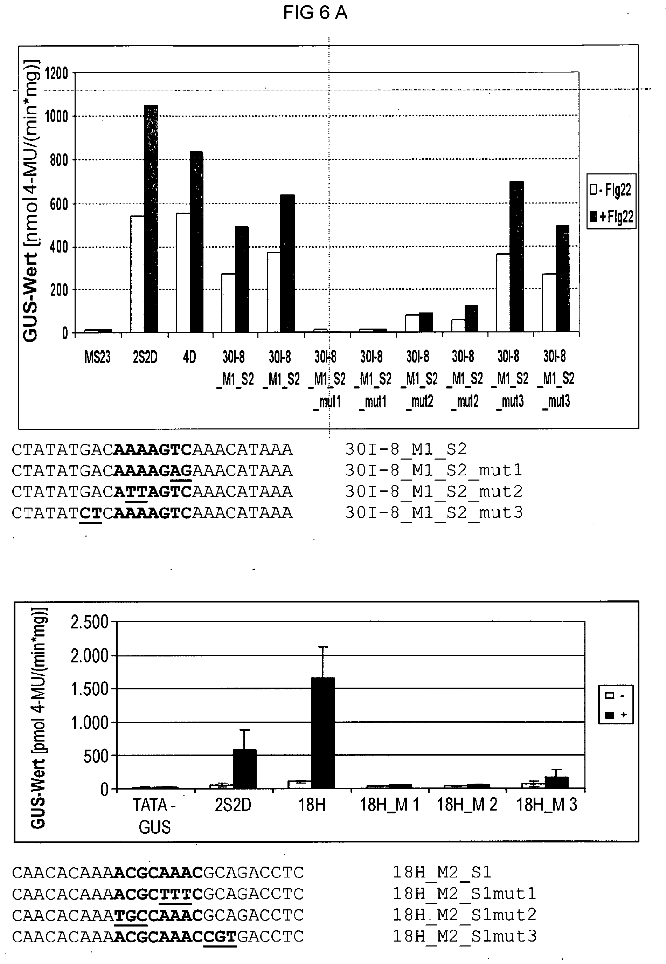

Fig 6 A: Elizitor-responsive Reportergenexpression der chimären Promotoren 4x30I-8_M1_S2 und 4x18H_M2_S1 mit Mutationen in den Einzelsequenzen. Die mutierten Basen sind unterstrichen. Die Elizitierung erfolgte durch PEP25 in Petersilienprotoplasten. Die Nukleotid-Sequenzen der mutierten Derivate der Einzelsequenzen sind unter den Diagrammen wiedergegeben. +, mit Elizitor PEP25; -, ohne Elizitor. 2S2D: Positivkontrolle; MS23GUS: Negativkontrolle (Leervektor).

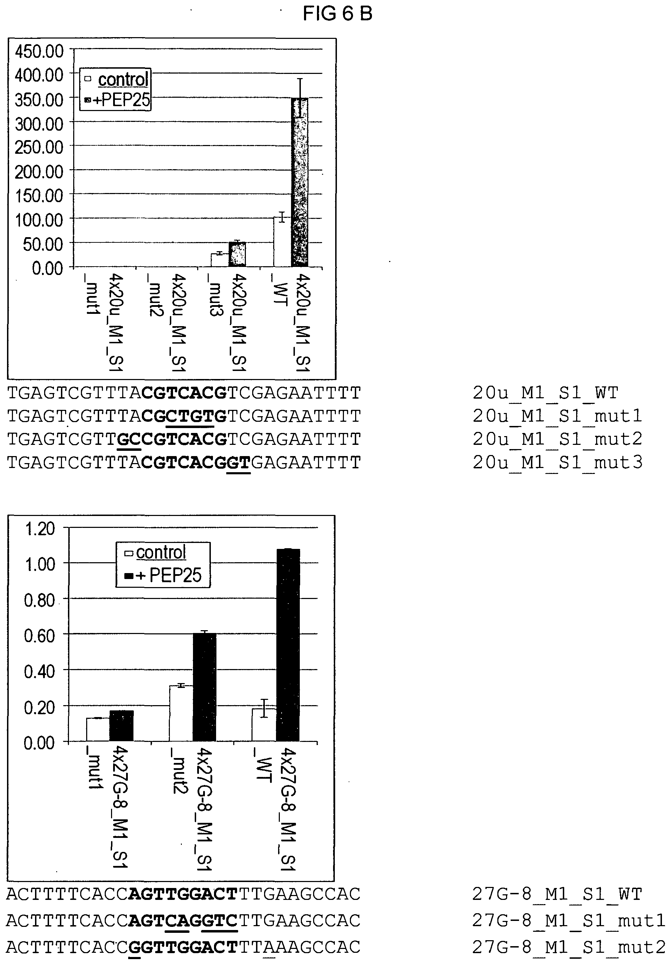

Fig 6 B: Elizitor-responsive Reportergenexpression der chimären Promotoren 4x20u_M1_S1 und 4x27G-8_M1_S1 mit Mutationen in den Einzelsequenzen. Die mutierten Basen sind unterstrichen. Die Elizitierung erfolgte durch PEP25 in Petersilienprotoplasten. Die Nukleotid-Sequenzen der mutierten Derivate der Einzelsequenzen sind unter den Diagrammen wiedergegeben. Diese Figur dient lediglich dem besseren Verständnis und ist nicht Teil der vorliegenden Erfindung.

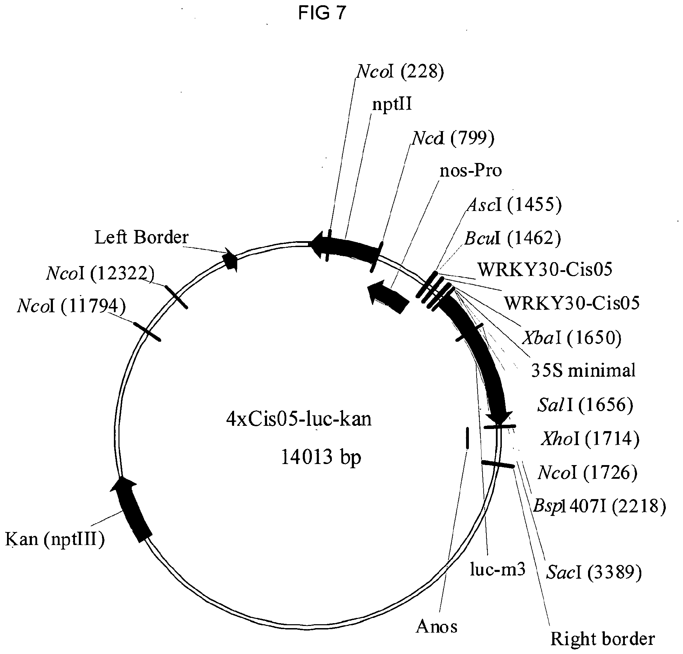

Fig 7: Binärer Vektor für die Transformation des Luziferase-Reportergens unter Kontrolle der chimären Promotoren in Zuckerrübe. Als Beispiel ist der Vektor mit dem chimären Promotor 4xCis05 gezeigt. nptII: Kanamycin-Resistenz; WRKY30-Cis05: Doppelte Einzelsequenz Cis05. 35S-minimal: 35S-Minmalpromotor; luc-m3: Luziferase-Reportergen; Anos: Nos-terminator.
Fig 8 A: *Cercospora beticola* induzierte Promotoraktivität in stabil transformierten Zuckerrüben. Von den angegebenen Konstrukten wurde für mehrere unabhängige Transformanten die Luziferase-Aktivität nach *C. beticola* Infektion von in vitro-Pflanzen bestimmt (4 Replikate pro Transformante und Zeitpunkt). Aus den erhaltenen Messwerten wurde der Median berechnet. In dem oberen Diagramm sind die Ergebnisse für Einzelsequenzen aus der Motivgruppe 12 zusammengefasst, in dem unteren Diagramm die Ergebnisse für Elemente aus der Motivgruppe 27. Ko: Kontrolle (Mock-Infektion); inf; Infektion mit *C. beticola;* 1d -4d: Tage nach Inokulation (d.p.i.). Kontrolle: Nicht transgene Pflanzen.
Fig 8 B: *Cercospora beticola* induzierte Promotoraktivität von 4xCis05 und seinen Derivaten in stabil transformierten Zuckerrüben. Für 4xCis05 und seine Derivate wurde die Luziferase-Aktivität nach *C. beticola* Infektion jeweils in mehreren unabhängige Transformanten bestimmt (4 Replikate pro Transformante und Zeitpunkt). Aus den erhaltenen Messwerten wurde der Median berechnet. Die Sequenz der verschiedenen Derivate ist in Fig. 5A und 5B wiedergegeben. Unter dem Diagramm ist zusätzlich für jedes der verschiedenen Derivate angegeben, ob es das Cis05-Motiv oder die W-Box enthält. Ko: Kontrolle (Mock-Infektion); inf; Infektion mit C. beticola; 1d -4d: Tage nach Inokulation (d.p.i.). non transgenic: Nicht transgene Kontrolle.

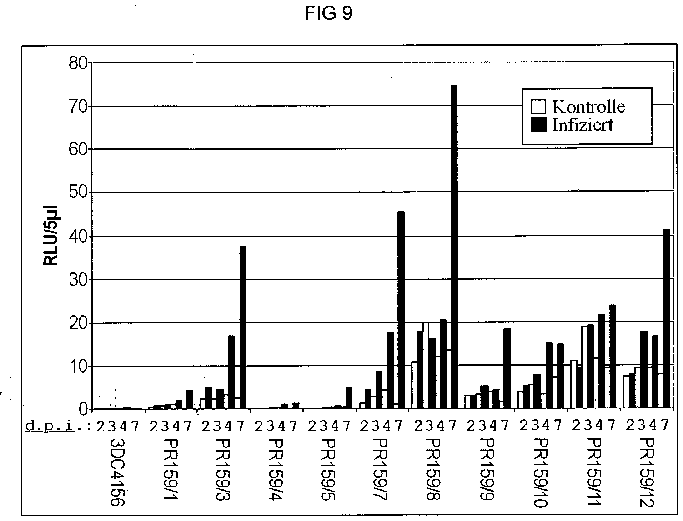

Fig 9: *Cercospora beticola* induzierte Promotoraktivität des chimären Promotors 4xGG6_M1_S1 in stabil transformierten Zuckerrüben. Für 10 unabhängige Transformanten mit dem Konstrukt 4xGG6_M1_S1-luc wurde die Luziferase-Aktivität nach *C. beticola* Infektion von in vitro-Pflanzen bestimmt (4 Replikate pro Transformante und Zeitpunkt). Ko: Kontrolle (Mock-Infektion); inf; Infektion mit *C. beticola*; 2d, 3d, 4d und 7d: Tage nach Inokulation (d.p.i.). 3DC4156: Nicht transgene Kontroll-Pflanzen. Diese Figur dient lediglich dem besseren Verständnis und ist nicht Teil der vorliegenden Erfindung.

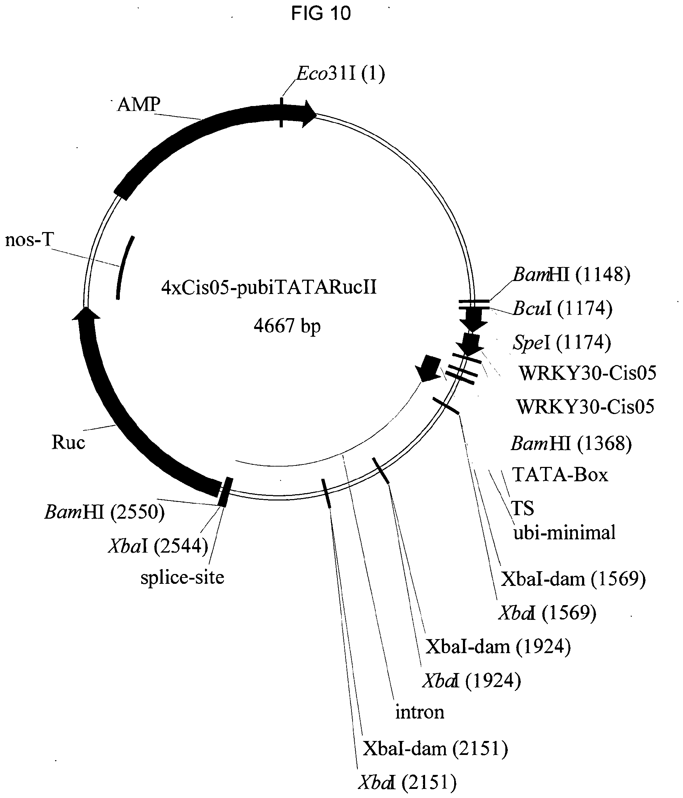

Fig 10: Plasmidkarte des für die transienten Tests in Weizen verwendeten Plasmids. Als Beispiel wird das Plasmid mit dem chimären 4xCis05-Promotor gezeigt. Ruc: *Renilla-*Luziferase Reportergen. AMP: Ampicilin-Resistenz. WRKY30-Cis05: Doppelte Einzelsequenz Cis05.
Fig 11: Test der Induktion des chimären Promotors 4xCis05 und seiner mutierten Derivate mit Mutationen in dem Cis05-Motiv (Cis05mut1), in der W-Box (Cis05mut2) oder in beiden Motiven (Cis05mut1+2) durch Fusarium. Die entsprechenden Konstrukte wurde transient in Weizen transformiert, und die Luziferase-Aktivität 20 Stunden nach Inkubation mit Fusarium gemessen. 4xCis05-dam/dcm bezeichnet einen Versuch, bei dem Plasmid-DNA einem nicht methylierenden E.Coli-Stamm verwendet wurde um eine Induktion durch dam/dcmmethylierte DNA (ebenfalls ein potentielles PAMP) auszuschließen. Wird die Kernsequenz von Cis05 mutiert, so geht die Induzierbarkeit vollständig verloren. Die Mutation in der W-Box hingegen hat keinen Effekt. Die Sequenzen von Cis05 und seinen mutierten Derivaten sind rechts wiedergegeben. Mutierte Basen sind rot unterlegt.
Fig 12: Induzierte und nicht-induzierte Aktivität der chimären Kombinatorik-Promotoren nach PEP25-Induktion in Petersilie. Die Tests wurden in drei biologischen Replikaten durchgeführt, die blaue Linie gibt den Induktionsfaktor wieder. Unter dem Diagramm sind in unteren Reihe die Elemente in 5' Position und in der oberen Reihe die Elemente in 3' Position angegeben. 30I8b ist eine andere Bezeichnung für die Einzelsequenz 30I-8_M1_S2.
Fig 13: Synergistische und antagonistische Interaktionen von Einzelsequenzen in den chimären Kombinatorik-Promotoren nach PEP25-Induktion in Petersilie. In Violet ist der tatsächlich gemessene Induktionsfaktor wiedergegeben, in Blau der auf Grund der Induktionsfaktoren der Einzelelemente erwartete Induktionsfaktor. Das Verhältnis beider Werte ist durch die gelbe Linie dargestellt. Liegen die Punkte der gelben Linie über dem Wert 1, so zeigen die Einzelelemente eine synergistische Interaktion.

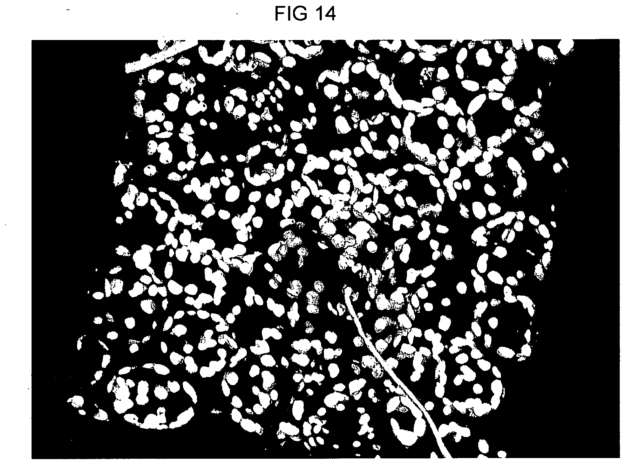

Fig 14: Transgene Zuckerrüben mit 4xCis05-RFP Konstrukt wurden mit *Cercospora beticola* infiziert. Die Infektion führt zur Aktivierung des chimären 4xCis05-Promotors, was zur Bildung des rot fluoreszierenden RFP-Proteins führt. Das Protein ist unter dem Mikroskop als rote Fluoreszenz zu sehen. Wie zu erkennen ist, ist die Induktion und damit die Fluoreszenz auf den Bereich um die Penetrationsstelle bzw. den Infektionsort beschränkt.
Fig 15: Beispielhafte schematische Darstellungen eines chimären Promotors im Sinne der Erfindung. Der chimäre Promotor ist operativ mit einem Nukleinsäuremolekül von Interesse verknüpft und umfasst (A) neben einem Minimalpromotor ein heterologes cis-regulatorisches Element, (B) neben einem Minimalpromotor ein Dimer/Multimer eines heterologen cis-regulatorisches Elements oder (C) neben einem Minimalpromotor zwei Dimere/Multimere mit jeweils unterschiedlichen heterologen cis-regulatorischen Elementen. Zudem zeigt (D) beispielhaft als chimären Promotor einen natürlichen Promotor, umfassend einen endogenen Minimalpromotor und ein endogenes cis-regulatorisches Element, wobei dieser Promotor durch Integration eines zusätzlichen homologen cis-regulatorischen Elements modifiziert wurde.

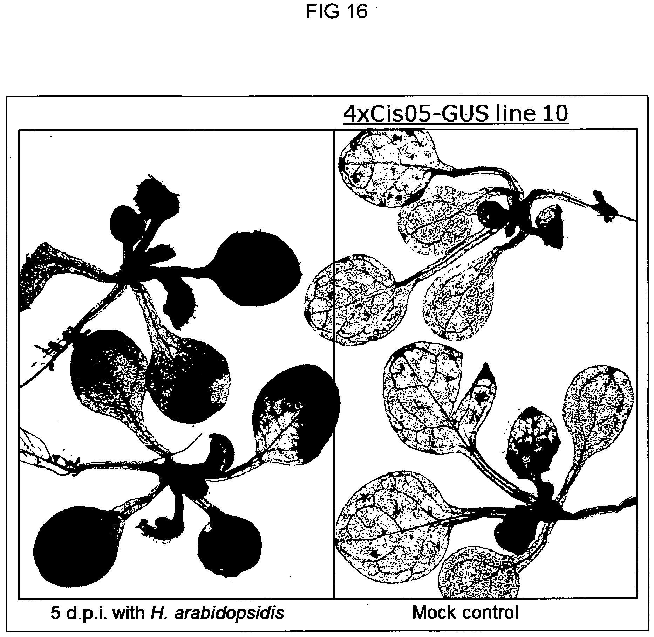

Fig 16: Transgene Arabidopsis-Pflanzen mit einem Tetramer des cis-regulatorischen Elements Cis05 in einem chimäre Promotoren, der die Expression des GUS-Reportergens kontrolliert, um die Pathogen-induzierte, Wund-induzierte und Gewebe-spezifische Aktivität des Elements zu untersuchen. Für den Promotor wurden 10 unabhängige Transformanten untersucht. Gezeigt ist jeweils eine repräsentative Linie. Das linke Bild (5 d.p.i. with *H*. *arabidopsidis*) zeigt jeweils die Aktivität des Promotors nach Infektion mit dem kompatiblen Pathogen *Hyaloperonospora arabidopsidis,* das rechte Bild (Mock control) ist die entsprechende Kontrolle. Der Promotor zeigt eine klare Induktion durch *Hyaloperonospora arabidopsidis* (Dunkelfärbung des pflanzlichen Gewebes).

Zudem ist auf dem rechten Bild ein Blatt eingeschnitten. Eine Blaufärbung an dieser Schnittstelle würde eine Wundinduzierbarkeit des Promotors mit dem Tetramer des cis-regulatorischen Elements Cis05 anzeigen.

### Bioinformatorische Identifizierung der erfindungsgemäßen cis-regulatorischen Elemente:

Grundlage für die bioinformatorische Identifizierung der neuen cis-regulatorischen Elemente sind öffentlich verfügbare Mikroarray-Expressionsdaten. Diese Expressionsdaten sind in Datenbanken wie TAIR, NASCArrays, Geo oder ArrayExpress hinterlegt oder können direkt aus entsprechenden Publikationen von Mikroarray-Experimenten bezogen werden (z.B. Rhee et al., 2003; Craigon et al., 2004; Barret und Edgar, 2006; Brazma et al., 2006, Zipfel et al., 2004, 2006; Bülow et al., 2007; Wan et al., 2008). Für die bioinformatorische Identifizierung neuer cis-regulatorischer Elemente wurden zunächst anhand der öffentlich verfügbaren Mikroarray-Expressionsdaten Gruppen von Genen der Pflanze *Arabidopsis thaliana* definiert, deren Expression durch Pathogene wie *P. syringae* oder *B*. *cinerea* oder PAMPs wie *flg22* oder Chitin induziert wird. Anschließend wurden die Sequenzen der Promotoren dieser Gen-Gruppen aus der Genom-Sequenz von *Arabidopsis thaliana* extrahiert (TAIR; http://www.arabidopsis.org). Unter Verwendung unterschiedlicher bekannter Algorithmen (MEME, Bioprospector, Alignace, BEST u. Ä.) wurden die Promotorsequenzen auf angereicherte Motive hin untersucht.

### Datenbankabfragen

Für die Datenbankabfrage zur Identifizierung koregulierter Gene wurde ein Softwaretool geschrieben, welches es erlaubt Gene zu identifizieren, die bei bis zu sechs verschiedenen Stimuli gemeinsam hochreguliert bzw. induziert werden. Es wurden mehr als 700 Datenbankabfragen zur Identifizierung koregulierter Gene durchgeführt. Dieser Abfrageprozess lieferte mehr als 400 Gruppen von gemeinsam induzierten Genen, die für eine Identifizierung gemeinsamer *cis*-regulatorischer Motive mit dem BEST-Softwarepaket geeignet sind (Che et al., 2005). Bei 77 Gruppen wurde durch ein Anheben des notwendigen Induktionsfaktors die Anzahl von gemeinsam regulierten Genen auf 120 Gene reduziert. Die Gesamtanzahl von Gengruppen koregulierter Gene (2-120) lag danach bei 510.

Von diesen 510 Gengruppen wurden 500 bp oder 1000 bp lange Promotorsequenzen stromaufwärts vom Transkriptionsstart (TSS) der koregulierten Gene extrahiert, deren TSS bekannt war. Mit den Programmen BEST, Cismodule, MD-Scan, BioProspector oder MEME wurden die Promotorsequenzen der koregulierten Gengruppen nach konservierten Sequenzmotiven untersucht. Dabei wurden Motivlängen von 5-10nt, 10-15nt und 15-20nt gewählt. Es wurden also etwa 500 x 3 (Motivlängen) = 1500 Abfragen durchgeführt. In den meisten Fällen wurden durch Verlängerung der Motivlängen keine weiteren Motive identifiziert, die nicht schon bei den kürzeren Motivlängen auftraten. In den BEST-Analysen wurde immer dann ein Motiv klassifiziert, wenn es von mindestens zwei der insgesamt 4 BEST-Programme gefunden wurde.

Bei bestimmten unterschiedlichen Stimuli wurden die gleichen koregulierten Gene, und daher auch die gleichen Motive erhalten. Es folgte eine Zusammenfassung der identischen Motive redundanter Gengruppen (GG) zu neuen Motiven (Tabelle Gengruppen), damit bei der vergleichenden, systematisierenden Analyse aller Motive untereinander nur einzigartige Motive verglichen wurden.

Es wurde ein Katalog erstellt, welcher alle identifizierten Motive plus Auswertungsdatei und die Sequenzlogos der einzelnen Motive enthält. Die Sequenzlogos (Crooks et al., 2004), erstellt unter http://weblogo.berkeley.edu/, spiegeln die Konserviertheit der Nukleotide an den einzelnen Positionen des Motivs wieder. Die Matrize (Matrix) wurde aus den Motiv bildenden Sequenzen erstellt, deren Sequenzen sowie deren zugehörige Gene ebenfalls wiedergegeben sind.

Mittels des Programms STAMP (Mahony and Benos, 2007), das unter der Internet-Adresse http://www.benoslab.pitt.edu/stamp aufgerufen werden kann, wurden die identifizierten Motive mit bereits bekannten *cis*-regulatorischen Elementen (PLACE, Agris, Athamap) verglichen. Darüber hinaus können mithilfe des Webservers STAMP ähnliche Motive zu einer Motivgruppe gruppiert werden. Das Programm gibt einen phylogenetischen Stammbaum heraus, an dem die Ähnlichkeiten der Motivgruppen zusammen gefasst dargestellt werden (Fig. 4).

### Nachweis der Pathogen-Induzierbarkeit der identifizierten Cis-regulatorischen Elemente

Da bioinformatorische Ansätze anfällig für die Bestimmung falsch-positiver Sequenzen sind muss die Pathogen-Induzierbarkeit experimentell bestätigt werden. Für die experimentelle Bestätigung wurden die bioinformatorisch identifizierten Sequenzen unter Nutzung von Standard DNA Klonierungstechniken als Tetramer vor ein Luciferase-Reportergen kloniert und in einem transienten Expressionssystemen in Petersilie auf ihre Induzierbarkeit durch das PAMP PEP25 getestet.

Für die experimentelle Untersuchung wurden Elemente ausgewählt, die neu sind und keine Ähnlichkeit zu bekannten cis-regulatorischen Elementen der Pathogen-vermittelten Induktion zeigen. Die in Tabelle 2 aufgeführten Einzelsequenzen wurden mit Spei und XbaI oder mit Spei und Sall Linkern in vitro synthetisiert und in das Plasmid MS23 kloniert. MS23 trägt dabei entweder ein ß-Glucuronidase (GUS)-Reportergen oder ein Luziferase Reportergen mit einem 35S Minimalpromotor. Alle Elemente wurden tetramerisiert und zur Überprüfung sequenziert. Als Beispiel für Klonierung der Einzelsequenzen als chimäre Promotoren sind in Fig. 1 die Plasmide mit 2xCis05 Element und dem multimerisierten 4xCis05 Element gezeigt.

**Tabelle 2: Untersuchte Einzelsequenzen. In der Tabelle sind die neuen Bezeichnungen und Sequenzen der untersuchten potentiell cis-regulatorischen Elemente aufgeführt. Die bioinformatisch identifizierten Kernsequenzen sind hervorgehoben (fett und unterstrichen). In der letzten Spalte ist das Ergebnis des PEP25/Petersilien Tests wiedergegeben (-: keine Induktion; +:Induzierbar).**

| **Einzelsequenz-Bezeichnung** | **Motivgruppe** | **AGI** | **Einzelsequenz** | **Induzierbarkeit** |
|---|---|---|---|---|
| 12i_M1_S1 | 1 | At5g04340 | TCTCATCT**CTCGACACG**CAACTTCC | + |
| Cis09 | 1 | At1g27730 | TGCACACACAC**ACACGTGT**ACTAGGTCAAACCAAACGT | + |
| Cis12 | 1 | At2g33580 | CAAAAAGTCAACACATACG**ACGCGT**TTCCATTGACTAAATA | + |
| 12c_M1_S1 | 4 | At1g21100 | TCTACTAG**AGGCCCATTAGG**ACCGGCAT | - |
| 20u_M1_S1 | 5 | At1g13990 | TGTTGAGTCGTTTA**CGTCACG**TCGAGAATTTTCTC | + |
| 20u_M1_S2 | 5 | At4g05020 | TGTCATTATTAATA**CGTGACG**AAACTGTAGCTCTG | + |
| 28M-1_M1_S1 | 5 | At1g09080 | TTACGTGTCA**AGAAGTGATTGGAGA**GGACACTCTAC | + |
| 28M-1_M1_S2 | 5 | At4g17500 | AAGACAAGTT**GAGAGAGACGAGACC**AATCACAACA | - |
| 28M-8_M1_S1 | 6 | At3g21520 | ATCCAACATCTC**GGACCGGATCA**ATGATTTATCAT | - |
| 24F_M1_S1 | 7 | At2g40750 | TCATCAAT**GTGACATAA**GCAAAGCT | - |
| 3C_M1_S1 | 11 | At3g51440 | TTTGATAC**GGTTACGGTTA**ATTAACG | - |
| GG6_M1_S1 | 11 | At2g40140 | GACTTTT**GACCTAAACCA**TTTCCAT | + |
| GG11_M1_S1 | 11 | At2g40140 | GTTTTGACTTTTG**ACCTAAACCA**TTTCCATGTAGAA | + |
| GG6_M1_S2 | 11 | At5g59820 | AAGATTCTCATCC**AACCGAAACGA**CTCTTTCGTTTT | - |
| GG11_M1_S2 | 11 | At5g59820 | AGATTCTCATCCA**ACCGAAACGA**CTCTTTCGTTTTT | - |
| GG11_M1_S3 | 11 | At1g27730 | TCTTCTTCATTTT**ACCAACACCA**CTTGCACACACAC | - |
| 22DDD_M1_S1 | 11 | At3g14990 | CCGTCTTAGTTT**ACCGAAACCAAA**GTGGCTTTTTCT | + |
| 21G-2_M1_S1 | 11 | At1g01560 | AGTTGAATTA**GTTCGGTTCGGTTCG**GTTGATATTG | - |
| 21G-2_M1_S2 | 11 | At3g55470 | CGTAATAATG**GTTTGGTTTGGTTTG**ATCAAGTCTT | + |
| 37E_M1_S1 | 12 | At2g39200 | CGATA**AACTTGCGAAACCC**TAAAA | - |
| 18H_M2_S1 | 12 | At1g70170 | CAACACAA**AACGCAAAC**GCAGACCTC | + |
| 18H_M2_S2 | 12 | At2g35980 | TATTGGAA**GTTTGGGGC**AACATCAC | - |
| 16MM_M1_S1 | 12 | At5g05340 | TTCAACCCTATA**AACCAAAACA**AATAACAGAATGC | - |
| 38M_M1_S1 | 12 | At4g23810 | AAATAATTATTTA**TGGTTTGGT**CATTTGGTCAAAT | + |
| 3I_M3_S1 | 12 | At1g26390 | CGCCTCAAT**CATGAAAACGAATCCTCT**GTAGTAGTG | - |
| 18H_M2_S3 | 12 | At1g70170 | AATTGACAAAAGA**CACGCAAAC**GATTCCAACGACC | + |
| 26LLL_M1_S1 | 12 | At1g67920 | TTACCGACACGTAA**CCAAAAC**TCACCGAACACCGT | - |
| 38M_M1_S2 | 12 | At2g29460 | GTTTCGAACGGGA**ACCAAACCA**TAATATGCGATGC | - |
| 38M_M1_S3 | 12 | At3g54960 | ACACTATTGGTCT**TGGTTTGGT**TTATATGCACGAC | - |
| 23LLL_M1_S1 | 12 | At2g30770 | GAAAACGATGGGTT**CCAAAACT**GTCGCTAATAAACT | - |
| 37D_M1_S1 | 12 | At1g32940 | TCTCCACTCGTTGT**GATTTGGT**CTGCAAGAAAACTA | - |
| 23LLL_M1_S2 | 12 | At1g01480 | ACGTTTTGAAATAT**TGTTTTGG**ATGGAGATTTTTTC | - |
| 34G-4_M1_S1 | 12 | At3g28740 | ATTTTTCATTTCGC**CCAAAACA**ATTATCCTAACGTT | - |
| 26LLL_M1_S2 | 12 | At4g01700 | AGTCAAAACGTAGA**CCAAAAC**AAAAACATGTAACT | + |
| 27H-8_M1_S1 | 12 | At4g18430 | TTTTATAACACTA**CCAAAACCAA**TAAGCCCTTTCGT | - |
| 26KKK_M1_S1 | 12 | At2g43510 | TCATCAAACCAATC**GGTTTGG**TCCTAAAGATAATT | - |
| 19Q_M1_S1 | 13 | At4g35180 | GTCAATATACAC**AGCCACCGAAC**AAATTACTCTAT | - |
| 21S_M1_S1 | 14 | At2g14610 | AAGCGATGT**TTACGAACC**CCAAAATC | - |
| 28H-9_M1_S1 | 14 | At1g19020 | AGATTT**GTTCGAGAACCT**TGAGAAA | - |
| 28H-9_M1_S2 | 14 | At4g37370 | TTGCTA**CTTCGAGAACATT**GGTCAA | - |
| 20a_M1_S1 | 15 | At5g04340 | TTAGAAGT**GGCTCGAGTG**TTCTACTT | - |
| 20a_M1_S2 | 15 | At5g20230 | AAGAAAGA**CAATCGAGCC**TAGAAATT | - |
| 12G_M2_S1 | 18 | At1g61800 | CCATACAATATAAA**CCACCAA**ACCATAACCACAAA | + |
| 3D_M1_S1 | 18 | At4g39950 | AATAATGTTCAAC**GTTGGTGGTG**GTACTCAAGATGG | - |
| 41J_M1_S1 | 19 | At3g09940 | TCAAATACAGGCA**ACCAAGACTC**GAGATCCTCATCG | - |
| 37C_M1_S1 | 20 | At3g51440 | AGAAAAATA**TTGGGCC**TACTGGGAA | - |
| 3M_M3_S1 | 20 | At3g02800 | AGATTCCTGAAGTGA**GGTCCA**CCCTAAAATCCATTT | - |
| GG8_M1_S1 | 21//21n | At1g27730 | CACACA**CGTGTACTAGGTC**AAACCA | + |
| 27G-8_M1_S1 | 21//21n | At2g38860 | AGGACTTTTCACC**AGTTGGACT**TTGAAGCCACCAA | + |
| 27G-8_M1_S2 | 21 | At1g72060 | GGTTT**AGTCAAAG**TAAACA**AGACTTTGACT**GTTCA | - |
| 27B-10_M1_S1 | 21 | At1g22890 | TGAACTTAAT**CACTGTCATTGTTTTC**GTAACAATTT | - |
| 26WW_M2_S1 | 21n | At4g02380 | CTC**AAAGGCCAGAATTGACGCAGC**CGTTT | + |
| 27B-10_M1_S3 | 21n | At1g21120 | CCTTGG**CCCAGTCCTTGGTCGT**CGTATC | + |
| GG4_M2_S1 | 22 | At3g14990 | GAAAAA**TGTGTGTGTTTGTG**TTAATT | - |
| GG4_M2_S2 | 22 | At5g59820 | ATAGTT**CCCAAACGGACACG**AACACA | - |
| 30A-8_M1_S1 | 24 | At3g49620 | GCAAACTA**ACGCCGGCGG**CCGTCTTG | - |
| 30I-8_M1_S1 | 27 | At5g12930 | ACAACAGAC**GACTTTT**CATAATTCA | + |
| 30I-8_M1_S2 | 27 | At1g26390 | CTATATGAC**AAAAGTC**AAACATAAA | + |
| GG13_M1_S2 | 27 | At3g26830 | TGTTCA**CTTTGAAAAGTATTC**TTTGAG | + |
| 30H-8_M1_S1 | 27 | At1g35230 | TAGCTGTT**GAAATTTCC**AAGAAAAT | - |
| 14S_M1_S1 | 27 | At1g76960 | CGATCA**GACTTTTCTACGCA**AGAGAA | + |
| 21S_M3_S1 | 27 | At1g76960 | TAATTTCTCTT**GCGTAGAAAAGTC**TGATCGGGAAG | + |
| 30I-8_M1_S3 | 27 | At5g24110 | TCGTTCTTCAGTCA**AAAAGTC**AAACTATCTCTCTC | + |
| Cis02 | 27 | At5g64905 | GAGCGTGAATT**GACTTTG**ACCAAAACCAAA | + |
| Cis05 | 27 | At5g24110 | GGTCAGCATGTTG**GACTTTC**CAAATTCATTGACCAAAG | + |
| Cis13 | 27 | At1g26380 | AAAATAAACAGCTACTTGA**CGAAAAGTCA**AACCAAATTC | + |
| 22AAA_M1_S1 | 31 | At2g40000 | TTTTT**CTCGTCCCCATCCTC**TATCC | - |
| 12r_M1_S1 | 32 | At1g73480 | CAATCTAC**TCGTCTCTTC**TCTTACAT | + |
| GG3_M1_S1 | 32 | At5g44420 | TAGGTT**CCTGCCCTCTCCGT**TCCTCC | - |
| GG3_M1_S2 | 32 | At4g39980 | TCGAAA**CCAACCCTCTCCCT**TATAAA | - |
| 20EE_M1_S1 | 32 | At4g39980 | GAAACCAA**CCCTCTCCCT**TATAAATA | - |
| 24P_M1_S1 | 32 | At1g30135 | TGTTTTGTTTCCA**CCGTCTCTCC**CGTGTCCTCTCTC | - |

Im Folgenden wurden die tetramerisierten Einzelsequenzen in transient transformierten Petersiliezellen aus einer Zellkultur auf ihre Induzierbarkeit durch das PAMP PEP25 getestet. So sollte neben einer generellen Validierung sichergestellt werden, dass sich die Elemente in verschiedenen Pflanzenspezies und durch verschiedene Elizitoren induzieren lassen.

Für den Test in Petersilie wurden aus einer 5 Tage alten Petersilienzellkultur Protoplasten isoliert. Dazu wurden 35 ml Zellkultur abzentrifugiert und in 90 ml einer sterilfiltrierten Lösung mit 0,5% Cellulase, 0,2% Macerozyme R-10 und 0,24 M CaCl₂ resuspendiert und über Nacht unter Schwenken bei 26°C inkubiert. Danach wurden die freigesetzten Protoplasten durch Zentrifugation pelletiert, mit 40 ml 0,24 M CaCl₂ gewaschen und anschließend in 50 ml P5-Medium (1 Tüte Fertigmedium Gamborgs B-5, 1 mg 2,4 D, 96,9 g Sacharose, pH 5,7 mit 1M KOH, steril filtrieren) resuspendiert. Nach Zentrifugation schwimmen die Protoplasten an der Oberfläche des P5-Mediums und können abgenommen werden. Die Aufreinigung mit P5-Medium wurde 2x wiederholt.

Für die Transformation wurden in 10 ml Schraubdeckelröhrchen 5 µg des zu testenden Promotorkonstrukts, 2,5 µg eines konstitutiv Renilla-Luciferase exprimierenden Normalisierungsvektors und 200 µl PEG vorgelegt. 200 µl Protoplasten wurden zugegeben, dann wurde vorsichtig gemischt und 20 min bei Raumtemperatur im Dunkeln inkubiert. Anschließend wurde die Reaktion durch Zugabe von 5 ml 0,275M CaNO₃-Lsg gestoppt. Die transformierten Protoplasten wurden abzentrifugiert, in 6ml P5-Medium aufgenommen und in 2 Aliquots aufgeteilt. Ein Aliquot wurde mit Pep25 (Endkonz: 300ng/ml; Sequenz: VTAGAEVWNQPVRGFKVYEQTEMT) elizitiert, das andere dient als Kontrolle. Nach Inkubation über Nacht wurden die Protoplasten durch Zentrifugation geerntet. Die Luziferaseaktivität wurde mit dem Dual Luziferase Kit (Promega, Mannheim, Deutschland) in einem Sirius Luminometer (Berthold Detection System GmbH, Pforzheim, Deutschland) bestimmt. Dazu wurde die Petersilienzellen durch Zentrifugation pelletiert und für 20 Minuten bei 4°C in 150 µl PLB-Puffer (Passive Lysis Buffer; Promega, Mannheim, Deutschland) lysiert. Die Zellreste werden für 20 Minuten bei 13.000 rpm und 4°C in einer Tischzentrifuge abzentrifugiert.

Mit dem Lysat wurde abhängig davon, ob das Luziferase-Reportergen oder das GUS-Reportergen verwendet wurde, unterschiedlich verfahren. Wurde das Luziferase-Reportergen verwendet, wurden von dem Überstand mit der freigesetzten Luziferase 5µl Probe in 5ml Röhrchen (Sarstedt, Art.Nr. 55.476) mit 50 µl LARII Puffer (Promega, Mannheim, Deutschland) gemischt. Der Puffer enthält das Substrat der Luziferase, so dass die Aktivität des Enzyms und damit des Promotors mit dem Luminometer gemessen werden kann. Die Messung erfolgt mit 2 Sekunden Vormesszeit und 10 Sekunden Luciferasemesszeit. Anschließend werden 50µl Stop & Glo Puffer (Promega, Mannheim, Deutschland) zugegeben und vorsichtig durch aufziehen gemischt. Dieser Puffer stoppt die Luziferaseaktivität und macht die konstitutive Renilla-Luciferase Aktivität des Normalisierungsvektors messbar. Dieser Messwert wird zur Normalisierung der unterschiedlichen Transformationseffizienzen verwendet. Die Messung erfolgt ebenfalls mit 2 Sekunden Vormesszeit und 10 Sekunden Luciferasemesszeit.

Wurde das ß-Glucuronidase (GUS) Reportergen aus *E. coli* verwendet (Jefferson *et al.,* 1987), erfolgte der Nachweis in einer Enzymreaktion, in der das Substrat MUG zu 4-MU hydrolysiert wird. Dann wird 4-MU durch seine Fluoreszenz nachgewiesen und quantifiziert. Nach diesen Methoden wurde für alle untersuchten Einzelsequenzen die Aktivität mit und ohne das PAMP PEP25 gemessen.

In Tabelle 2 sind alle getesteten Einzelsequenzen aufgeführt. Zudem ist angegeben, ob sie durch das PAMP PEP25 in Petersilie induzierbar waren. Die als Pathogen-Induzierbar identifizierten cis-regulatorische Elemente (Einzelsequenzen) und ihre Motive sind in Fig. 2 zusammengefasst.

### Mutationsanalysen der identifizierten Kernsequenzen

Um nachzuweisen, dass jeweils die identifizierte Kernsequenz für die PAMP- und Pathogen-Induzierbarkeit der Einzelsequenzen verantwortlich ist, wurden für ausgewählte Einzelsequenzen Mutationsanalysen durchgeführt. Dafür wurden mutierte Derivate der Einzelsequenzen erstellt. Die mutierten Einzelsequenzen wurden als Oligonukleotide synthetisiert. Die Klonierung der Plasmide wurde entsprechend zu den Konstrukten mit chimären Promotoren ohne Mutationen durchgeführt. Anschließend wurden die Konstrukte wie zuvor oben beschrieben auf ihre PEP25-Induzierbarkeit in Petersilie getestet. Die Ergebnisse der Mutationsanalysen sind in den Fig. 5A, Fig. 5B, Fig. 6A und Fig. 6B wiedergegeben. Für alle fünf untersuchten Elemente konnte gezeigt werden, dass die identifizierte Kernsequenz für die Induzierbarkeit verantwortlich ist.
Besondere Bedeutung hat dies für die Einzelsequenzen Cis05 und 30I-8 M1 S2 der Gruppe 27. Die Kernsequenz von 30I-8_M1_S2 überlappt teilweise mit einer zur W-Box (TTGAC; Rushton et al., 1996) komplementären Sequenz (GTCAA). Eine überlappende W-Box Sequenz wurde auch in den Einzelsequenzen 30I-8_M1_S3, Cis02 und Cis13 gefunden. Es konnte jedoch durch die Mutationsanalysen, insbesondere durch die Variante 30I-8_M1_S2_mut2, gezeigt werden, dass Basen außerhalb der W-Box entscheidend für die PAMP-Induzierbarkeit sind (Fig. 6A). Zudem zeigen andere Mitglieder der Gruppe 27 keine überlappende W-Box-Sequenz. Entsprechend ist die für die W-Box zwingend notwendige Kernsequenz TGAC nicht Teil des Sequenz- oder Familienmotivs der Gruppe 27 (Fig. 3F). Somit handelt es sich bei den Motiven bzw. den Einzelsequenzen der Gruppe 27 nicht um Varianten der W-Box.
In der Einzelsequenz Cis05 findet sich eine W-Box Sequenz außerhalb der Kernsequenz. Hier konnte durch die Mutationsanalysen gezeigt werden, das diese W-Box zwar PAMP-Induzierbarkeit vermittelt. Jedoch führt die Mutation nur der Cis05 Kernsequenz oder nur der W-Box zu einer Abnahme der Induzierbarkeit, und nur durch Mutation beider Elemente kommt es zu einem vollständigen Verlust der Induzierbarkeit. Somit handelt es sich um eine Einzelsequenz mit zwei funktionellen, PAMP- und Pathogen-induzierbaren cis-regulatorischen Elementen, der bekannten W-Box und dem neuen Cis05-Motiv (Fig. 2T). Dabei zeigt die Kombination beider Elemente eine deutlich höhere Aktivität als die Einzelelemente allein.
Um den Sequenzbereich des Elements Cis05-Motivs (Fig. 2T) einzugrenzen, der die Induzierbarkeit vermittelt, wurde dieses separat von der W-Box tetramerisiert (sCis05) und es wurden vier weitere mutierte Derivate des Cis05-Motivs erstellt. Die Derivate wurden wie zuvor beschrieben in Petersilie auf ihre Induzierbarkeit durch das PAMP PEP25 getestet (Fig. 5B). Dabei konnte neben der entscheidenden Bedeutung der Kernsequenz für die Induzierbarkeit gezeigt werden, dass auch die beiden in 5'-Richtung vor der Kernsequenz liegenden Basen essentiell für die Induzierbarkeit von Cis05 sind, was weiter bestätigt, das es sich nicht um ein W-Box-Variante handelt.

Durch weitere Mutationsanalysen konnten auch die Kernsequenzen von den Elementen 20u_M1_S1 und 27G-8_M1_S1 bestätigt werden. Die Versuche wurden wie oben bereits beschrieben durchgeführt. Die Ergebnisse dieser Mutationsanalysen sind in Fig. 6B (oben: 20u_M1_S1; unten: 27G-8_M1_S1) wiedergegeben.

### Ableitung von Familienmotiven

Dadurch, dass die in *Arabidopsis*-Promotoren identifizierten cis-regulatorischen Elemente in Petersilie getestet wurden, wurde eine Pflanzen-Spezies übergreifende biologische Funktionalität der identifizierten cis-regulatorischen Elemente sichergestellt. Wie erwartet zeigten die Experimente, dass nicht alle der bioinformatorisch identifizierten Sequenzen funktionell sind. Etwa ein Drittel der getesteten DNA Sequenzen waren in Petersilie durch PEP25 induzierbar. Die anderen Sequenzen waren falsch-positive Sequenzen aus der bioinformatorischen Analyse, oder zeigten nicht die gewünschte Spezies-übergreifende biologische Funktionalität. Diese wichtigen Ergebnisse konnten genutzt werden, um auf der Grundlage der funktionell wirksamen Einzelsequenzen die Familienmotive der Motivgruppen 1, 5, 11, 12, 21, 21n und 27 sowie die dazugehörigen stark konservierten, charakteristischen Kernsequenzen zu identifizieren (Fig. 3). Dafür wurden alle zu einer Motivgruppe gehörenden funktionell wirksamen Einzelsequenzen zusammengefasst und aus ihnen ein Konsensus und ein Motiv abgeleitet. Als charakteristisches Kernsequenzmotiv der Familienmotive wurde zunächst der Bereich definiert, der in allen zugrundeliegenden Einzelsequenzen Teil der Kernsequenz ist.

Die Kernsequenz von Motivgruppe 27 findet sich auch in der Sequenz LS10 (Lebel et al., 1998). Dort wurde in dem nativen PR-1 Promotor in diesem Sequenzbereich eine 10 Basen umfassende Mutation erzeugt, die zu einem starken Rückgang einer SA- oder INA-Induzierbarkeit des nativen Promotors führte. Die dort gezeigten Ergebnisse ermöglichen jedoch nicht die Ableitung eines Motivs oder einer Kernsequenz. Zudem ist die Verwendbarkeit von LS10 für chimäre Promotoren nicht gezeigt. Des Weiteren grenzt das Familienmotiv der Motivgruppe 27 die Motivgruppe von der LS10-Sequenz ab. Das Familienmotiv schließt an der Position 5 ein C aus, während in LS10 an dieser Position ein C vorhanden ist. Des Weiteren schließt es an der Position 17 ein G aus, während in LS10 an dieser Position ein G vorhanden ist. Schließlich erfordert es an Position 18 ein T oder C, während in LS10 an dieser Position ein A vorhanden ist.

### Nachweis der Induzierbarkeit durch den pathogenen Pilz Cercospora beticola in stabil transformierten Zuckerrüben

Die neuen cis-regulatorischen Elemente sollen sich dadurch auszeichnen, dass sie in verschiedenen Pflanzenspezies durch unterschiedliche PAMPs und Pathogene induziert werden können. Um die Induzierbarkeit in einer agronomisch wichtigen Pflanze durch ein agronomisch wichtiges Pathogen zu zeigen, wurde die in Petersilie positiv getesteten Einzelsequenzen stabil in Zuckerrüben transformiert. Dazu wurden die chimären Promotoren mit den tetramerisierten Einzelsequenzen inklusive des luc-Gens über die Ascl- und Sacl-Schnittstellen in den binären Vektor 1xW1-luc-kan, ein auf dem binären Vektor pGPTV basierendes Plasmid, umkloniert. Als Beispiel ist in Fig. 7 der Vektor 4xCis05-luc-kan gezeigt. Entsprechende Vektoren wurden für alle untersuchten Elemente erstellt. Die Plasmid-DNA der binären Vektoren wurden aus *E. Coli* isoliert und mit einem Gene Pulser® II. Electroporation System mit den Einstellung 25 mF und 2.5 kV in den *Agrobacterium tumefaciens* Stamm GV3101 transformiert. Die Selektion rekombinater *A. tumefaciens-*Klone erfolgte unter Verwendung des Antibiotikums Kanamycin (50 mg/l). Die Transformation der Zuckerrüben erfolgte nach Lindsey et al. (1991) unter Verwendung des Antibiotikums Kanamycin.
Die Transgenität der Pflanzen wurde durch PCR überprüft. Die Verwendung der Primer GTGGAGAGGCTATTCGGTA (SEQ ID NO: 36) und CCACCATGATATTCGGCAAG (SEQ ID NO: 37) führte zu der Amplifikation eines 553 bp großen DNA-Fragments aus dem *npt*II-Gen. Die PCR wurde unter Verwendung von 10 ng genomischer DNA, einer Primerkonzentration von 0,2 µM bei einer Annealingtemperatur von 55°C in einem Multicycler PTC-200 (MJ Research, Watertown, USA) durchgeführt.

10 bis 20 unabhängige transgene Linien wurden in *in vitro* Kultur klonal vermehrt und mit Cercospora beticola (Isolat Ahlburg) infiziert. Nach 1, 2, 3 und 4 Tagen wurden je 4 Pflanzen / Linie geerntet und ihre Luziferase-Aktivität mit dem Promega Luciferase Assay System, 100 assays, Kat.Nr. E1500 (LAR) gemessen. Dazu wurden die Proben in 4 Volumen CCLR-Buffer (Cell Culture Lysis Reagent, 5x) aufgenommen und mit Hilfe eines Heidolph (RZR 2020) aufgearbeitet. Die Pflanzenreste wurden 10min bei 4°C und 14000rpm abzentrifugiert und der Überstand für die Luziferase-Messung eingesetzt. Zur Messung wurden 10µl Probe in ein 5ml Röhrchen (Sarstedt, Art.Nr. 55.476) pipettiert und 100µl Luziferase Assay Reagent (LAR; Promega, Mannheim, Deutschland) zugeben. Dann wurde vorsichtig gemischt und die Luziferase-Aktivität in einem Sirius Luminometer (Berthold Detection System GmbH, Pforzheim, Deutschland) bestimmt.
Unter anderem wurden neue Elemente aus den Gruppen 12 und 27 (Zur Gruppen-Einteilung siehe Fig. 3D und F) getestet. Aus den gemessenen Luziferase-Aktivitäten der etwa 10 bis 20 unabhängige transgene Linien mit einem chimären Promotor wurde der Median berechnet. Die Tests der verschiedenen Promotoren sind nach den Gruppen, aus denen die Elemente stammen, zusammengefasst. Die Ergebnisse sind in Fig. 8A gezeigt. Der direkte Vergleich zeigt, dass die Einzelsequenzen einer Motivgruppe trotz der hohen Homologie der in ihnen enthaltenen Motive deutlich unterschiedliche Aktivitäten aufweisen. Die an die Kernsequenz angrenzenden Nukleotide des Familenmotivs können die Promotorstärke und die Hintergrundaktivität also stark beeinflussen. Die verschiedenen Sequenzen einer Motivgruppe sind somit in ihrer Funktion nicht identisch sondern erlauben die Entwicklung von chimären Promotoren mit quantitativen Unterschieden in der Regulation der Genexpression.
Um eine weitere Motivgruppe zu testen, wurde das Elemente GG6_M1 (Einzelsequenz GG6_M1_S1) entsprechend den Angaben oben stabil in Zuckerrübe transformiert und auf die Induzierbarkeit durch Cercospora getestet, wobei das Element GG6_M1 (Einzelsequenz GG6_M1_S1) lediglich dem besseren Verständnis dient und nicht Teil der Erfindung ist.

Dazu wurden 10 unabhängige transgene Zuckerrüben wurden in *in vitro* Kultur klonal vermehrt und mit Cercospora beticola (Isolat Ahlburg) infiziert. Nach 2, 3, 4 und 7 Tagen wurden je 4 Pflanzen / Linie geerntet und ihre Luziferase-Aktivität mit dem Promega Luciferase Assay System, 100 assays, Kat.Nr. E1500 (LAR) gemessen. Die Ergebnisse sind in Fig. 9 gezeigt und zeigen eine klare Induzierbarkeit der Einzelsequenz GG6_M1_S1 in Zuckerrübe durch Cercospora beticola. Des Weiteren zeigte eine histochemische Analyse, dass die Induktion weitestgehend im Leitgewebe erfolgt.

Um den Einfluss der W-Box Sequenz in der Einzelsequenz Cis05 in stabil transformierten Pflanzen zu untersuchen, wurden die mutierten Derivate Cis05mut1 und Cis05mut2 sowie die verkürzte Einzelsequenz sCis05 aus den Petersilientests stabil in Zuckerrübe transformiert. Konstrukterstellung und Transformation wurden wie oben beschreiben durchgeführt. Jeweils 18 unabhängige transgene Linien wurden in vitro klonal vermehrt und mit *Cercospora beticola* (Isolat Ahlburg) infiziert. Nach 1, 2, 3 und 4 Tagen wurden je 4 Pflanzen / Linie geerntet und ihre Luziferase-Aktivität wie oben beschrieben gemessen (Fig. 8 B).
Die Mutationsanalyse in stabil transformierten Pflanzen zeigt, dass diese W-Box alleine, d. h. mit mutiertem Cis05-Motiv, nur eine schwache Pathogen-Induzierbarkeit durch *Cercospora* vermittelt. Das Cis05-Motiv alleine (mutierte W-Box oder verkürztes Element ohne W-Box) ist hingegen deutlich induzierbar. Die komplette Cis05-Einzelsequenz mit W-Box ist ebenfalls induzierbar, zeigt aber relativ zu den Derivaten ohne W-Box eine erhöhte Hintergrundaktivität. Somit ist in stabil transformierten Zuckerrüben das Cis05 Motiv alleine der Kombination mit der W-Box gleichwertig oder sogar überlegen.
Ein weiteres wichtiges Merkmal der erfindungsgemäßen chimären Promotoren ist, dass die Pathogen-induzierte Aktivität auf den Bereich der Infektionsstelle beschränkt ist. Dies wird in Fig. 14 am Beispiel des Erfindungsgemäßen Promotor 4xCis05 gezeigt. Dieser Promotor wurde mit dem rot Fluoreszierenden Reportergen RFP fusioniert, und das so erhaltene Konstrukt stabil in Zuckerrübe transformiert. Unter dem Laser Scanning Mikroskop lässt sich die Aktivität als rote Fluoreszenz beobachten. In Fig. 14 ist die lokale Induktion des 4xCis05 Promotors rund um die Penetrationsstelle einer *Cercospora-Hyphe* gezeigt.

### Erweiterte Speziesübergreifende Pathogeninduzierbarkeit

Als weiteres Beispiel für breite Verwendbarkeit der cis-regulatorischen Elemente in unterschiedlichen Pflanzenspezies wurde für Cis05 in transienten Versuchen die Induzierbarkeit in der Monokotylen Pflanze Weizen durch den Pilz Fusarium culmorum gezeigt. Da der 35S minimal Promotor in Weizen eine unzureichende Aktivität vermittelt mussten die Cis05-Elemente umkloniert werden. Dazu wurden sie mit den Enzymen Eco31I und XbaI aus den für die Petersilien-Tests verwendeten Plasmiden ausgeschnitten und in den mit Eco31I und BcuI geöffneten Vektor pubiTATARucII kloniert (Fig. 10). Entsprechende Konstrukte wurden für Cis05 sowie die mutierten Cis05-Einzelsequenzen Cis05mut1 und Cis05mut2 erstellt, in denen entweder das Cis05-Motiv bzw. die W-Box mutiert ist. Diese Konstrukte wurden biolistisch in mit Fusarium graminearum infizierte Primärblätter der Weizensorte "Taifun" sowie nicht infizierte Kontroll-Blätter transformiert.

Für die Infektion wurde *Fusarium graminearum* Mycel mit einem Objektträger von bewachsene Pilzplatte abgekratzt und in 200 ml Wasser kurz mit einem Ultraturrax zerkleinern und suspendiert. Anschließend wurden 200µl 2% Triton zugegeben und die Weizen-Primärblätter 1 Minute in der Suspension geschwenkt. Anschließend wurden die infizierten Blattstückchen und nicht infizierte Kontroll-Blattstückchen auf H₂O-Agarplatten aufgelegt und mit einer Biorad particle gun nach Herstellerangaben unter Verwendung von 1100 Psi Berstscheiben transient transformiert. Als Normalisierungsvektor wurde ein konstitutiv Luziferase exprimierender Vektor verwendet.

Die Weizenblätter wurden anschließend über Nacht bei 25°C inkubiert. Zur Bestimmung der Luciferase-Aktivitäten werden die Blätter in je 1 ml PLB-Puffer mit Seesand aufgemörsert. Nach Zentrifugation für 20 Minuten bei 4°C werden von dem Überstand mit der freigesetzten Luziferase 5µl Probe in 5ml Röhrchen (Sarstedt, Art.Nr. 55.476) mit 50 µl LARII Puffer (Promega, Mannheim, Deutschland) gemischt. Der Puffer enthält das Substrat der Luziferase, so dass die Aktivität des Normalisierungsvektors gemessen werden kann. Dieser Messwert wird zur Normalisierung der unterschiedlichen Transformationseffizienzen verwendet. Die Messung erfolgt mit 2 Sekunden Vormesszeit und 10 Sekunden Luziferasemesszeit. Anschließend werden 50µl Stop & Glo Puffer (Promega, Mannheim, Deutschland) zugegeben und vorsichtig durch aufziehen gemischt. Dieser Puffer stoppt die Luziferaseaktivität und macht die Renilla-Luciferase Aktivität, die der Aktivität der Cis05-Promotoren entspricht, messbar. Die Messung erfolgt ebenfalls mit 2 Sekunden Vormesszeit und 10 Sekunden Luciferasemesszeit.

Die induzierten bzw. nicht induzierten Aktivitäten des 4xCis05-Promotors sowie seiner mutierten Derivate wurde in 5 biologischen Wiederholungen gemessen (Fig. 11). Anschließend wurde der komplette Versuch noch einmal wiederholt. Beide Wiederholungen zeigen, dass in Weizen die Pathogen-induzierte Aktivität von dem Cis05-Motiv stammt, während das W-Box Motiv keine von *Fusarium graminearum* induzierte Aktivität vermittelt.

### Analyse der Pathogen- oder Wund-induzierten und der gewebespezifischen Aktivität der Elemente Cis02, Cis05, Cis09, Cis12 oder Cis13 in Arabidopsis

Die neuen cis-regulatorischen Elemente sollen sich dadurch auszeichnen, dass sie in verschiedenen Pflanzenspezies durch unterschiedliche PAMPs und Pathogene induziert werden können. Als weitere Kontrolle für die Aktivität der chimären Promotoren wurden 6 Promotoren mit tetramerisierten Einzelsequenzen stabil in Arabidopsis transformiert. Dazu wurden die tetramerisierten Elemente Cis02, Cis05, Cis09, Cis12 oder Cis13 mit 35S-Minimalpromotor vor das GUS-Reportergen in den Transformationsvektor pBIN-GUS kloniert. Das fertige Konstrukt wurde in Agrobakterien transformiert. Im Folgenden wurde eine floral-dip Transformation (Clough and Bent, 1998) von Arabidopsis Pflanzen durchgeführt, um die Promotor-GUS Konstrukte stabil in das Pflanzengenom zu integrieren. Die Selektion transgener Pflanzen erfolgte unter Verwendung des Antibiotikums Kanamycin. Für jedes Element wurden 10 unabhängige Transformaten untersucht.

Die Aktivität des GUS-Reportergens und damit der Promotoren lässt sich durch eine GUS-Färbung sichtbar machen (Jefferson et al., 1987). Die Aktivierung der jeweiligen chimären Promotoren führt zur Expression eines Enzyms (GUS), welches einen blauen Farbstoff bildet. Die Färbung zeigt somit die Aktivität des jeweiligen chimären Promotors an.

Um die Pathogen-Induzierbarkeit der Promotoren zu testen, wurden die transgenen Arabidopsis-Pflanzen mit dem kompatiblen Pathogen *Hyaloperonospora arabidopsidis* infiziert. 5 Tage nach Infektion wurde die Aktivität der Promotoren durch einen GUS-Färbung nachgewiesen. Zudem wurden einzelne Blätter durch Einschneiden mit einer Schere verwundet, um die Wundinduzierbarkeit der Promotoren zu testen (Fig. 16).

Das Element Cis02 zeigte eine gute Pathogen-Induzierbarkeit und nur eine geringe WundInduzierbarkeit. Das Element Cis05 zeigte eine generell starke Aktivität und wird ebenfalls stark durch *H. Arabidopsidis* induziert. Das Elemente Cis09 zeigte eine gute Induktion des Promotors nach Infektion, und kaum unerwünschte Aktivität nach Verwundung. Das Elemente Cis12 zeigte wie Element Cis09 in allen untersuchten Linien kaum unerwünschte Aktivität nach Verwundung, während eine klare Induktion durch das Pathogen H. *arabidopsidis* zu beobachten war. Die Elemente Cis12 und Cis09 teilen ein gemeinsames Familienmotiv. Auch das Element Cis13 wird stark durch Infektion mit *H. arabidopsidis* induziert. Eine klare Wundinduzierbarkeit ist jedoch nicht zu beobachten. Exemplarisch ist in Fig. 16 die GUS-Färbung von transgenen A. thaliana-Pflanzen gezeigt, die das GUS-Reportergen unter der Kontrolle eines chimären Promotors mit 4x Cis05 exprimieren.

### Kombinatorik der cis-regulatorischen Elemente

Durch eine synergistische Interaktion oder durch Integration verschiedener Signalwege (denkbar wäre ein Promotor, der Signale "EF-Tu" UND "fig22" wahrnehmen und integrieren muss, um aktiviert zu werden) können chimären Kombinatorik-Promotoren, die aus verschiedenen cis-regulatorische Elementen zusammengesetzt sind, eine höhere Spezifität und/oder Aktivität zeigen als die in Ihnen vorhandenen Einzel-Elemente (Rushton et al., 2002). Um optimale Kombinationen der neuen cis-regulatorischen Elemente für chimären Kombinatorik-Promotoren zu ermitteln, wurden unter Nutzung von Standard DNA Klonierungstechniken chimäre Kombinatorik-Promotoren mit allen möglichen 2x2-Kombination der hier beschriebenen cis-regulatorischen Elemente Cis02, Cis05, Cis12, Cis13 und 30I-8_M1_S2 sowie der bereits veröffentlichten Elemente D-Box (Rushton et al., 2002), S-Box (Kirsch et al., 2000) und Gst1-Box (Strittmatter et al., 1996) erzeugt. Dabei wurde so vorgegangen, wie für die Tetramerisierung der Einzelsequenzen beschrieben, nur das nicht zwei identische Dimere (z.B. 2x30I-8_M1_S2 und 2x30I-8_M1_S2), sondern zwei unterschiedliche Dimere (z.B. 2xCis05 und 2x30I-8_M1_S2) aneinander kloniert werden. Die so entstandenen chimären Kombinatorik-Promotoren wurden in dem transienten Expressionssystemen in Petersilie auf ihre Induzierbarkeit durch das PAMP PEP25 getestet. Die Ergebnisse (absolute nicht-induzierte und induzierte Aktivität der chimären Kombinatorik-Promotoren und der Induktionsfaktor) sind in Fig. 12 wiedergegeben. Es konnten chimäre Kombinatorik-Promotoren mit besonders starker und spezifischer Induzierbarkeit identifiziert werden, deren Induktionsfaktor und Aktivität höher ist als bei chimären Promotoren, die nur aus Wiederholungen der einzelnen cis-regulatorischen Elemente aufgebaut sind (Fig. 13). Die chimären Kombinatorik-Promotoren mit den höchsten Induktionsfaktoren und den stärksten Aktivitäten sind in Tabelle 3 zusammengefasst.

**Tabelle 3: Kombinationen von Pathogen-induzierbare Einzelsequenzen mit den höchsten Induktionsfaktoren und induzierten Aktivitäten.**

| Kombinationen mit dem höchsten Induktionsfaktor | | | | | |
|---|---|---|---|---|---|
| | | MW | stabw | Varianzkoeffizient | Induktion |
| *2xCis05-2xCis05* | - | *3,23* | *2,06* | *0,64* | **149,58** |
| *2xCis05-2xCis05* | + | *482,53* | *398, 05* | *0,82* | |
| 2xCis05-2xS | - | 2,00 | 1,51 | 0,76 | **115,63** |
| 2xCis05-2xS | + | 231,82 | 48,39 | 0,21 | |
| 2xCis05-2x30I8b | - | 2,47 | 1,42 | 0,58 | **123,16** |
| 2xCis05-2x30I8b | + | 304,21 | 45,38 | 0,15 | |
| 2xCis13-2xCis02 | - | 2,50 | 0,50 | 0,20 | **124,53** |
| 2xCis13-2xCis02 | + | 311,48 | 37,76 | 0,12 | |
| 2xCis13-2xCis05 | - | 2,33 | 0,41 | 0,18 | **182,59** |
| 2xCis13-2xCis05 | + | 425,23 | 258,29 | 0,61 | |
| 2xCis13-2xS | - | 4,55 | 0,65 | 0,14 | **139,85** |
| 2xCis13-2xS | + | 636,85 | 364,80 | 0,57 | |
| 2xS-2xCis02 | - | 5,28 | 3,36 | 0,64 | **106,32** |
| 2xS-2xCis02 | + | 561,76 | 440,81 | 0,78 | |
| *2xS-2xS* | - | 2,76 | *0,49* | *0,18* | **138,80** |
| *2xS-2xS* | + | 382,69 | *92,20* | *0,24* | |
| Gst1-2xS | - | 2,41 | 0,87 | 0,36 | **258,83** |
| Gst1-2xS | + | 624,18 | 274,19 | 0,44 | |
| 2x30I8b-2xCis05 | - | 0,90 | 0,30 | 0,34 | **135,12** |
| 2x30I8b-2xCis05 | + | 121,20 | 88,90 | 0,73 | |

| Kombinationen mit der höchsten Aktivität (induziert) | | | | | |
|---|---|---|---|---|---|
| | | MW | stabw | Varianzkoeffizient | Induktion |
| 2xD-2xCis05 | - | 84,57 | 49,56 | 0,59 | 17,88 |
| 2xD-2xCis05 | + | **1511,90** | 580,33 | 0,38 | |
| 2xD-2xS | - | 23,96 | 17,66 | 0,74 | 57,72 |
| 2xD-2xS | + | **1382,94** | 1180,84 | 0,85 | |
| 2xD-Gst1 | - | 19,25 | 20,18 | 1,05 | 62,10 |
| 2xD-Gst1 | + | **1195,21** | 486,92 | 0,41 | |
| 2xS-2xCis12 | - | 36,84 | 19,34 | 0,53 | 29,05 |
| 2xS-2xCis12 | + | **1070,08** | 1043,46 | 0,98 | |
| 2xS-2xD | - | 22,13 | 23,38 | 1,06 | 65,56 |
| 2xS-2xD | + | **1450,50** | 1085,69 | 0,75 | |

### Referenzen

Bailey, T.L., C. Elkan (1994). "Fitting a mixture model by expectation maximization to discover motifs in biopolymers." Proc Int Conf Intell Syst Mol Biol. 2: 28-36.
Bulow, L., M. Schindler, et al. (2007). "PathoPlant: a platform for microarray expression data to analyze co-regulated genes involved in plant defense responses." Nucleic Acids Res 35(Database issue): D841-845.
Che, D., S. Jensen, et al. (2005). "BEST: binding-site estimation suite of tools." Bioinformatics 21(12): 2909-2911.
Clough, S.J. and Bent, A.F. (1998). "Floral dip: a simplified method for Agrobacterium-mediated transformation of Arabidopsis thaliana." Plant J. 16(6):735-43.
Eulgem, T., P. J. Rushton, et al. (2000). "The WRKY superfamily of plant transcription factors." Trends Plant Sci 5(5): 199-206.
Gorlich, D. and I. W. Mattaj (1996). "Nucleocytoplasmic transport." Science 271(5255): 1513-1518.
Gurr, S. J. and P. J. Rushton (2005). "Engineering plants with increased disease resistance: how are we going to express it?" Trends Biotechnol 23(6): 283-290.
Hahlbrock, K., D. Scheel, et al. (1995). "Oligopeptide elicitor-mediated defense gene activation in cultured parsley cells." Proc Natl Acad Sci U S A 92(10): 4150-4157.
Hicks, G. R., H. M. Smith, et al. (1995). "Three classes of nuclear import signals bind to plant nuclei." Plant Physiol 107(4): 1055-1058.
Himmelbach, A., L. Liu et al. (2010). "Promoters of the Barley Germin-Like GER4 Gene Cluster Enable Strong Transgene Expression in Response to Pathogen Attack" The Plant Cell 22(3): 937-952.
Howles, P., G. Lawrence, et al. (2005). "Autoactive Alleles of the Flax L6 Rust Resistance Gene Induce Non-Race-Specific Rust Resistance Associated with the Hypersensitive Response." Molecular Plant-Microbe Interactions 18(6): 570-582.
Humphry, M., P. Bednarek, et al. (2010). "A regulon conserved in monocot and dicot plants defines a functional module in antifungal plant immunity." Proc Natl Acad Sci U S A 107(50): 21896-21901.
Jefferson, R. A., Kavanagh, T. A & Bevan, M. W. (1987). "GUS fusions, 8-glucuronidase as a sensitive and versatile gene fusion marker in higher plants." EMBO J 6(13): 3901-3907.
Kirsch, C., M. Takamiya-Wik, et al. (2000) "A novel regulatory element involved in rapid activation of parsley ELI7 gene family members by fungal elicitor or pathogen infection." Mol Plant Pathol. 1(4): 243-51.
Kirsch, C., E. Logemann, et al. (2001) "A highly specific pathogen-responsive promoter element from the immediate-early activated CMPG1 gene in Petroselinum crispum." Plant J. 26(2): 217-27.
Kovalchuk, N., M. Li, et al. (2010). "Defensin promoters as potential tools for engineering disease resistance in cereal grains." Plant Biotechnol J 8(1): 47-64.
Lebel, E., P. Heifetz, et al. (1998). "Functional analysis of regulatory sequences controlling PR-1 gene expression in Arabidopsis." Plant J 16(2): 223-233.
Liu, X., D. L. Brutlag, et al. (2001). "BioProspector: discovering conserved DNA motifs in upstream regulatory regions of co-expressed genes." Pac Symp Biocomput: 127-138.
Morris, K., S. A. MacKerness, et al. (2000). "Salicylic acid has a role in regulating gene expression during leaf senescence." Plant J 23(5): 677-685.
Rushton, P. J., A. Reinstadler, et al. (2002). "Synthetic plant promoters containing defined regulatory elements provide novel insights into pathogen- and wound-induced signaling." Plant Cell 14(4): 749-762.
Rushton, P. J., J. T. Torres, et al. (1996). "Interaction of elicitor-induced DNA-binding proteins with elicitor response elements in the promoters of parsley PR1 genes." EMBO J 15(20): 5690-5700.
Schatz, G. and B. Dobberstein (1996). "Common principles of protein translocation across membranes." Science 271(5255): 1519-1526.
Schumacher, S. B., O. Van den Hauwe, et al. (2003). "Development of a dual luciferase reporter screening assay for the detection of synthetic glucocorticoids in animal tissues." Analyst 128(12): 1406-1412.
Strittmatter, G., G. Gheysen, et al. (1996). "Infections with various types of organisms stimulate transcription from a short promoter fragment of the potato gst1 gene." Mol Plant Microbe Interact 9(1): 68-73.
Venter, M. (2007). "Synthetic promoters: genetic control through cis engineering." Trends Plant Sci 12(3): 118-124.
Verner, K. and G. Schatz (1988). "Protein translocation across membranes." Science 241(4871): 1307-1313.
Wan, J., X. C. Zhang, et al. (2008). "A LysM receptor-like kinase plays a critical role in chitin signaling and fungal resistance in Arabidopsis." Plant Cell 20(2): 471-481.
Zipfel, C., G. Kunze, et al. (2006). "Perception of the bacterial PAMP EF-Tu by the receptor EFR restricts Agrobacterium-mediated transformation." Cell 125(4): 749-760.
Zipfel, C., S. Robatzek, et al. (2004). "Bacterial disease resistance in Arabidopsis through flagellin perception." Nature 428(6984): 764-767.

WO 00/29592 (Max-Planck-Gesellschaft zur Förderung der Wissenschaften e.V.). Chimeric promoters capable of mediating gene expression in plants upon pathogen infection and uses thereof.

WO 02/50293 (Max-Planck-Gesellschaft zur Förderung der Wissenschaften e.V.). Pflanzen mit verbesserter Widerstandskraft.

WO 03/00898 (Syngenta Participations AG). Plant genes involved in defense against pathogens.

WO 07/147395 (KWS SAAT AG). Pathogen induzierbarer synthetischer Promotor

WO 10/079430 (U. Bonas et al.). Modular DNA-binding domains and methods of use.

WO 11/072246 (Regents of the University of Minnesota; lowa State University Research Foundation Inc.). TAL effector-mediated DNA modification.

## Patentansprüche

1. Verwendung einer isolierten DNA-Sequenz-, welche ein Nukleinsäuremolekül umfasst, dessen Nukleotidsequenz das Kernsequenzmotiv vaaagtm oder ein dazu komplementäres Kernsequenzmotiv aufweist, als Pathogeninduzierbarkeit- oder Elizitorinduzierbarkeitvermittelndes cis-regulatorisches Element in einem chimären Promotor, wobei 'v' für Adenin (a), Guanin (g) oder Cytosin (c) und 'm' für Adenin (a) oder Cytosin (c) steht.

2. Verwendung gemäß Anspruch 1 **dadurch gekennzeichnet, dass** die Nukleotidsequenz des Nukleinsäuremoleküls die Kernsequenz des cis-regulatorischen Elementes darstellt, wobei die Kernsequenz für die Funktionalität des cis-regulatorischen Elementes essentiell ist.

3. Isoliertes cis-regulatorisches Element, welches ein Nukleinsäuremolekül umfasst, dessen Nukleotidsequenz ein Familienmotiv gemäß SEQ ID NO: 1, umfassend das Kernsequenzmotiv vaaagtm, oder ein dazu komplementären Familienmotiv aufweist, wobei 'v' für Adenin (a), Guanin (g) oder Cytosin (c) und 'm' für Adenin (a) oder Cytosin (c) steht und
wobei das Nukleinsäuremolekül
a) eine Nukleotidsequenz gemäß SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 oder SEQ ID NO: 15 oder
b) eine Nukleotidsequenz komplementär zu einer Nukleotidsequenz aus a) aufweist.

4. Isoliertes cis-regulatorisches Element gemäß Anspruch 3 weiterhin **dadurch gekennzeichnet, dass** das cis-regulatorische Element eine Länge von weniger als 50 Nukleotiden aufweist.

5. Chimärer Promotor, welcher geeignet ist, induziert durch eine Pathogeninfektion oder eine Behandlung mit einem pathogenen Elizitor eine Expression eines operativ verknüpften Nukleinsäuremoleküls in einer pflanzlichen Zelle zu bewirken und welcher einen Minimalpromotor und mindestens ein cis-regulatorisches Element umfasst, **dadurch gekennzeichnet, dass** das cis-regulatorische Element
a) ein Nukleinsäuremolekül umfasst, dessen Nukleotidsequenz das Kernsequenzmotiv vaaagtm oder ein dazu komplementäres Kernsequenzmotiv aufweist, oder
b) ein cis-regulatorisches Element gemäß Anspruch 3 oder 4 ist.

6. Chimärer Promotor nach Anspruch 5 **dadurch gekennzeichnet, dass** der chimäre Promotor mindestens ein Multimer von cis-regulatorischen Elementen umfasst, wobei mindestens eines der cis-regulatorischen Elemente ein cis-regulatorisches Element wie definiert in Anspruch 5 ist.

7. Chimärer Promotor nach Anspruch 6 **dadurch gekennzeichnet, dass** das Multimer ein Dimer oder Tetramer ist.

8. Ein rekombinantes Gen, welches den chimären Promotor nach einem der Ansprüche 5 bis 7 umfasst.

9. Ein Vektor, welcher den chimären Promotor nach einem der Ansprüche 5 bis 7 oder das rekombinante Gen nach Anspruch 8 umfasst.

10. Eine prokaryotische oder eukaryotische Wirtszelle, welche den chimären Promotor nach einem der Ansprüche 5 bis 7, das rekombinante Gen nach Anspruch 8 oder den Vektor nach Anspruch 9 umfasst.

11. Eine transgene Pflanze, transformiert mit dem chimären Promotor nach einem der Ansprüche 5 bis 7, mit dem rekombinanten Gen nach Anspruch 8 oder mit dem Vektor nach Anspruch 9.

12. Samen, Zelle, Gewebe oder Teil der Pflanze nach Anspruch 11 enthaltend den chimären Promotor nach einem der Ansprüche 5 bis 7, das rekombinante Gen nach Anspruch 8 oder den Vektor nach Anspruch 9.

13. Methode zur Herstellung einer Pflanze nach Anspruch 11, umfassend
a) das Einbringen des chimären Promotors nach einem der Ansprüche 5 bis 7, des rekombinanten Gens nach Anspruch 8 oder des Vektors gemäß Anspruch 9 in mindestens eine Zelle der Pflanze und
b) das Regenerieren der Pflanze.

14. Ein Verfahren zur Pathogeninduzierbarkeit- oder Elikitorinduzierbarkeit-vermittelten Expression eines endogenen Gens unter Kontrolle eines durch ein cis-regulatorisches Element modifizierten nativen Promotors umfassend das Einbringen eines cis-regulatorischen Elements mit dem Kernsequenzmotiv vaaagtm in den nativen Promotor des endogenen Gens.

15. Eine pflanzliche Zelle, welche ein endogenes Gen unter der Kontrolle eines chimären Promotors gemäß einem der Ansprüche 5 bis 7, oder unter der Kontrolle des nativen Promotors des endogenen Gens, der durch das Einbringen eines cis-regulatorische Elements mit dem Kernsequenzmotiv vaaagtm modifiziert ist, exprimiert.

16. Pflanze regeneriert aus der pflanzlichen Zelle gemäß Anspruch 15.

## Claims

1. Use of an isolated DNA sequence which comprises a nucleic acid molecule, the nucleotide sequence of which has the core sequence motif vaaagtm or a core sequence motif complementary thereto as a pathogen-inducible or elicitor-inducible-mediating cis-regulatory element in a chimeric promoter wherein 'v' stands for adenine (a), guanine (g) or cytosine (c), and 'm' stands for adenine (a) or cytosine (c).

2. Use according to claim 1, **characterized in that** the nucleotide sequence of the nucleic acid molecule represents the core sequence of the cis-regulatory element, wherein the core sequence is essential for the functionality of the cis-regulatory element.

3. Isolated cis-regulatory element which comprises a nucleic acid molecule, the nucleotide sequence of which has a family motif according to SEQ ID NO: 1, comprising the core sequence motif, or a family motif complementary thereto, wherein 'v' stands for adenine (a), guanine (g) or cytosine (c) and 'm' stands for adenine (a) or cytosine (c) and
wherein the nucleic acid molecule has
a) a nucleotide sequence according to SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 or SEQ ID NO: 15 or
b) a nucleotide sequence complementary to a nucleotide sequence from a).

4. Isolated cis-regulatory element according to claim 3, further **characterized in that** the cis-regulatory element has a length of less than 50 nucleotides.

5. Chimeric promoter which is suitable for effecting expression of an operatively linked nucleic acid molecule in a plant cell, induced by pathogen infection or a treatment with a pathogenic elicitor, and which comprises a minimal promoter and at least one cis-regulatory element, **characterized in that** the cis-regulatory element
a) comprises a nucleic acid molecule, the nucleotide sequence of which has the core sequence motif vaaagtm or a core sequence motif complementary thereto, or
b) is a cis-regulatory element according to claim 3 or 4.

6. Chimeric promoter according to claim 5, **characterized in that** the chimeric promoter comprises at least one multimer of cis-regulatory elements, wherein at least one of the cis-regulatory elements is a cis-regulatory element as defined in claim 5.

7. Chimeric promoter according to claim 6, **characterized in that** the multimer is a dimer or tetramer.

8. A recombinant gene which comprises the chimeric promoter of any one of claims 5 to 7.

9. A vector which comprises the chimeric promoter according to any one of claims 5 to 7 or the recombinant gene according to claim 8.

10. A prokaryotic or eukaryotic host cell which comprises the chimeric promoter according to any one of claims 5 to 7, the recombinant gene according to claim 8, or the vector according to claim 9.

11. A transgenic plant transformed with the chimeric promoter according to any one of claims 5 to 7, with the recombinant gene according to claim 8 or with the vector according to claim 9.

12. Seed, cell, tissue or part of the plant according to claim 11 containing the chimeric promoter according to one of claims 5 to 7, the recombinant gene according to claim 8 or the vector according to claim 9.

13. Method for producing a plant according to claim 11, comprising
a) introducing the chimeric promoter according to one of claims 5 to 7, the recombinant gene according to claim 8 or the vector according to claim 9 into at least one cell of the plant and
b) regenerating the plant.

14. Method for the pathogen-inducible or elicitor-inducible-mediated expression of an endogenous gene under the control of a native promoter modified by a cis-regulatory element comprising introducing a cis-regulatory element having the core sequence motif vaaagtm into the native promoter of the endogenous gene.

15. A plant cell which expresses an endogenous gene under the control of a chimeric promoter according to any one of claims 5 to 7, or under the control of the native promoter of the endogenous gene which is modified by introducing a cis-regulatory element having the core sequence motif vaaagtm.

16. Plant regenerated from the plant cell according to claim 15.

## Revendications

1. Utilisation d'une séquence ADN isolée, comprenant une molécule d'acide nucléique dont la séquence nucléotidique comporte le motif de séquence nucléaire vaaagtm ou un motif de séquence nucléaire complémentaire de celui-ci, en tant qu'élément régulateur de cis promoteur inductible par pathogène ou inductible éliciteur dans un promoteur chimère, dans lequel 'v' signifie adénine (a), guanine (g) ou cytosine (c) et 'm' signifie adénine (a) ou cytosine (c).

2. Utilisation selon la revendication 1 **caractérisée en ce que** la séquence nucléotidique de la molécule d'acide nucléique représente la séquence nucléaire de l'élément régulateur de cis, la séquence nucléaire étant essentielle pour la fonctionnalité de l'élément régulateur de cis.

3. Élément régulateur de cis isolé, lequel comprend une molécule d'acide nucléique, dont la séquence nucléotidique comporte un motif familier selon SEQ ID NO: 1, comprenant le motif de séquence nucléaire vaaagtm, ou un motif familier complémentaire de celui-ci, 'v' signifiant adénine (a), guanine (g) ou cytosine (c) et 'm' signifiant adénine (a) ou cytosine (c), la molécule d'acide nucléique
a) comportant une séquence nucléotidique selon SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 ou SEQ ID NO: 15 ou
b) une séquence nucléotidique complémentaire à une séquence nucléotidique selon a).

4. Élément régulateur de cis isolé selon la revendication 3, **caractérisé par** ailleurs en ce que l'élément régulateur de cis présente une longueur inférieure à 50 nucléotides.

5. Promoteur chimère, lequel est apte, induit par une infection pathogène ou un traitement avec un éliciteur pathogène à provoquer une expression d'une molécule d'acide nucléique liée de façon opérationnelle dans une cellule végétale et lequel comprend un promoteur minimal et au moins un élément régulateur de cis, **caractérisé en ce que** l'élément régulateur de cis
a) comprend une molécule d'acide nucléique dont la séquence nucléotidique comporte le motif de séquence nucléaire vaaagtm ou un motif de séquence nucléaire complémentaire de celui-ci ou
b) est un élément régulateur de cis selon la revendication 3 ou 4.

6. Promoteur chimère selon la revendication 5,**caractérisé en ce que** le promoteur chimère comprend au moins un multimère d'éléments régulateurs de cis, au moins l'un des éléments régulateurs de cis étant un élément régulateur de cis défini dans la revendication 5.

7. Promoteur chimère selon la revendication 6, **caractérisé en ce que** le multimère est un dimère ou un tétramère.

8. Un gène recombinant, lequel comprend le promoteur chimère selon l'une quelconque des revendications 5 à 7.

9. Un vecteur, lequel comprend le promoteur chimère selon l'une quelconque des revendications 5 à 7 ou un gène recombinant selon la revendication 8.

10. Une cellule hôte procaryote ou eucaryote, laquelle gène recombinant selon la revendication 8 ou le vecteur selon la revendication 9.

11. Une plante transgénique, transformée avec le promoteur chimère selon l'une quelconque des revendications 5 à 7, avec le gène recombinant selon la revendication 8 ou avec le vecteur selon la revendication 9.

12. Semence, cellule, tissu ou partie de la plante selon la revendication 11 contenant le promoteur chimère selon l'une quelconque des revendications 5 à 7, le gène recombinant selon la revendication 8 ou le vecteur selon la revendication 9.

13. Méthode de production d'une plante selon la revendication 11, comprenant
a) l'introduction du promoteur chimère selon l'une quelconque des revendications 5 à 7, du gène recombinant selon la revendication 8 ou du vecteur selon la revendication 9 dans au moins une cellule de la plante et
b) la régénération de la plante.

14. Un procédé pour l'expression inductible par pathogène ou inductible par éliciteur d'un gène endogène sous contrôle d'un promoteur natif modifié par un élément régulateur de cis, comprenant l'introduction d'un élément régulateur de cis avec le motif de séquence nucléaire vaaagtm dans le promoteur natif du gène endogène.

15. Une cellule végétale, laquelle exprime un gène endogène, sous le contrôle d'un promoteur chimère selon l'une quelconque des revendications 5 à 7, ou sous le contrôle du promoteur natif du gène endogène qui est modifié par l'introduction d'un élément régulateur de cis avec le motif de séquence nucléaire vaaagtm.

16. Plante, régénérée à partir de la cellule végétale selon la revendication 15.


### SEQUENCE LISTING

<110> KWS SAAT AG
<120> Neue aus Pflanzen stammende cis-regulatorische Elemente für die Entwicklung Pathogen-responsiver chimärer Promotoren
<130> KWS 0200 PCT
<150> DE 102011122267.0
   <151> 2011-12-23
<160> 44
<170> PatentIn version 3.5
<210> 1
   <211> 18
   <212> DNA
   <213> Artificial Sequence
<220>
   <223> Familienmotiv Gruppe 27
<220>
   <221> misc_feature
   <222> (1)..(2)
   <223> n is a, c, g, or t
<220>
   <221> misc_feature
   <222> (6)..(7)
   <223> n is a, c, g, or t
<220>
   <221> misc_feature
   <222> (9)..(14)
   <223> Kernsequenzmotiv
<220>
   <221> misc_feature
   <222> (15)..(15)
   <223> n is a, c, g, or t
<400> 1
   nnhkdnnvaa agtmndhy 18
<210> 2
   <211> 16
   <212> DNA
   <213> Artificial Sequence
<220>
   <223> Familienmotiv Gruppe 11
<220>
   <221> misc_feature
   <222> (2)..(2)
   <223> n is a, c, g, or t
<220>
   <221> misc_feature
   <222> (6)..(6)
   <223> n is a, c, g, or t
<220>
   <221> misc_feature
   <222> (7)..(12)
   <223> Kernsequenzmotiv
<220>
   <221> misc_feature
   <222> (15)..(15)
   <223> n is a, c, g, or t
<400> 2
   ynamcnaaac cawwny 16
<210> 3
   <211> 13
   <212> DNA
   <213> Artificial sequence
<220>
   <223> Familienmotiv Gruppe 12
<220>
   <221> misc_feature
   <222> (2)..(2)
   <223> n is a, c, g, or t
<220>
   <221> misc_feature
   <222> (5)..(10)
   <223> Kernsequenzmotiv
<400> 3
   wnrmscaaam smw 13
<210> 4
   <211> 13
   <212> DNA
   <213> Artificial sequence
<220>
   <223> Familienmotiv Gruppe 1
<220>
   <221> misc_feature
   <222> (1)..(2)
   <223> n is a, c, g, or t
<220>
   <221> misc_feature
   <222> (5)..(9)
   <223> Kernsequenzmotiv
<220>
   <221> misc_feature
   <222> (11)..(11)
   <223> n is a, c, g, or t
<400> 4
   nnmsacrcgy nwm 13
<210> 5
   <211> 14
   <212> DNA
   <213> Artificial Sequence
<220>
   <223> Familienmotiv Gruppe 21
<220>
   <221> misc_feature
   <222> (2)..(9)
   <223> Kernsequenzmotiv
<400> 5
   asktgkactw kgwm 14
<210> 6
   <211> 15
   <212> DNA
   <213> Artificial sequence
<220>
   <223> Familienmotiv Gruppe 5
<220>
   <221> misc_feature
   <222> (2)..(2)
   <223> n is a, c, g, or t
<220>
   <221> misc_feature
   <222> (6)..(12)
   <223> Kernsequenzmotiv
<220>
   <221> misc_feature
   <222> (15)..(15)
   <223> n is a, c, g, or t
<400> 6
   knwymmrtsa ckwmn 15
<210> 7
   <211> 25
   <212> DNA
   <213> Arabidopsis thaliana
<220>
   <221> misc_feature
   <222> (10)..(16)
   <223> Kernsequenz
<400> 7
   acaacagacg acttttcata attca 25
<210> 8
   <211> 25
   <212> DNA
   <213> Arabidopsis thaliana
<220>
   <221> misc_feature
   <222> (10)..(16)
   <223> Kernsequenz
<400> 8
   ctatatgaca aaagtcaaac ataaa 25
<210> 9
   <211> 27
   <212> DNA
   <213> Arabidopsis thaliana
<220>
   <221> misc_feature
   <222> (7)..(21)
   <223> Kernsequenz
<400> 9
   tgttcacttt gaaaagtatt ctttgag 27
<210> 10
   <211> 26
   <212> DNA
   <213> Arabidopsis thaliana
<220>
   <221> misc_feature
   <222> (7)..(20)
   <223> Kernsequenz
<400> 10
   cgatcagact tttctacgca agagaa 26
<210> 11
   <211> 35
   <212> DNA
   <213> Arabidopsis thaliana
<220>
   <221> misc_feature
   <222> (12)..(24)
   <223> Kernsequenz
<400> 11
   taatttctct tgcgtagaaa agtctgatcg ggaag 35
<210> 12
   <211> 35
   <212> DNA
   <213> Arabidopsis thaliana
<220>
   <221> misc_feature
   <222> (15)..(21)
   <223> Kernsequenz
<400> 12
   tcgttcttca gtcaaaaagt caaactatct ctctc 35
<210> 13
   <211> 30
   <212> DNA
   <213> Arabidopsis thaliana
<220>
   <221> misc_feature
   <222> (12)..(18)
   <223> Kernsequenz
<400> 13
   gagcgtgaat tgactttgac caaaaccaaa 30
<210> 14
   <211> 38
   <212> DNA
   <213> Arabidopsis thaliana
<220>
   <221> misc_feature
   <222> (14)..(20)
   <223> Kernsequenz
<400> 14
   ggtcagcatg ttggactttc caaattcatt gaccaaag 38
<210> 15
   <211> 39
   <212> DNA
   <213> Arabidopsis thaliana
<220>
   <221> misc_feature
   <222> (20)..(29)
   <223> Kernsequenz
<400> 15
   aaaataaaca gctacttgac gaaaagtcaa accaaattc 39
<210> 16
   <211> 36
   <212> DNA
   <213> Arabidopsis thaliana
<220>
   <221> misc_feature
   <222> (14)..(23)
   <223> Kernsequenz
<400> 16
   gttttgactt ttgacctaaa ccatttccat gtagaa 36
<210> 17
   <211> 36
   <212> DNA
   <213> Arabidopsis thaliana
<220>
   <221> misc_feature
   <222> (13)..(24)
   <223> Kernsequenz
<400> 17
   ccgtcttagt ttaccgaaac caaagtggct ttttct 36
<210> 18
   <211> 35
   <212> DNA
   <213> Arabidopsis thaliana
<220>
   <221> misc_feature
   <222> (11)..(25)
   <223> Kernsequenz
<400> 18
   cgtaataatg gtttggtttg gtttgatcaa gtctt 35
<210> 19
   <211> 25
   <212> DNA
   <213> Arabidopsis thaliana
<220>
   <221> misc_feature
   <222> (8)..(18)
   <223> Kernsequenz
<400> 19
   gacttttgac ctaaaccatt tccat 25
<210> 20
   <211> 26
   <212> DNA
   <213> Arabidopsis thaliana
<220>
   <221> misc_feature
   <222> (9)..(17)
   <223> Kernsequenz
<400> 20
   caacacaaaa cgcaaacgca gacctc 26
<210> 21
   <211> 35
   <212> DNA
   <213> Arabidopsis thaliana
<220>
   <221> misc_feature
   <222> (14)..(22)
   <223> Kernsequenz
<400> 21
   aattgacaaa agacacgcaa acgattccaa cgacc 35
<210> 22
   <211> 35
   <212> DNA
   <213> Arabidopsis thaliana
<220>
   <221> misc_feature
   <222> (14)..(22)
   <223> Kernsequenz
<400> 22
   aaataattat ttatggtttg gtcatttggt caaat 35
<210> 23
   <211> 35
   <212> DNA
   <213> Arabidopsis thaliana
<220>
   <221> misc_feature
   <222> (15)..(21)
   <223> Kernsequenz
<400> 23
   agtcaaaacg tagaccaaaa caaaaacatg taact 35
<210> 24
   <211> 38
   <212> DNA
   <213> Arabidopsis thaliana
<220>
   <221> misc_feature
   <222> (12)..(19)
   <223> Kernsequenz
<400> 24
   tgcacacaca cacacgtgta ctaggtcaaa ccaaacgt 38
<210> 25
   <211> 41
   <212> DNA
   <213> Arabidopsis thaliana
<220>
   <221> misc_feature
   <222> (20)..(25)
   <223> Kernsequenz
<400> 25
   caaaaagtca acacatacga cgcgtttcca ttgactaaat a 41
<210> 26
   <211> 25
   <212> DNA
   <213> Arabidopsis thaliana
<220>
   <221> misc_feature
   <222> (9)..(17)
   <223> Kernsequenz
<400> 26
   tctcatctct cgacacgcaa cttcc 25
<210> 27
   <211> 25
   <212> DNA
   <213> Arabidopsis thaliana
<220>
   <221> misc_feature
   <222> (7)..(19)
   <223> Kernsequenz
<400> 27
   cacacacgtg tactaggtca aacca 25
<210> 28
   <211> 35
   <212> DNA
   <213> Arabidopsis thaliana
<220>
   <221> misc_feature
   <222> (14)..(22)
   <223> Kernsequenz
<400> 28
   aggacttttc accagttgga ctttgaagcc accaa 35
<210> 29
   <211> 34
   <212> DNA
   <213> Arabidopsis thaliana
<220>
   <221> misc_feature
   <222> (11)..(19)
   <223> Kernsequenz
<400> 29
   aagtctaaat ctttgacccc aaaaaagaga gcaa 34
<210> 30
   <211> 35
   <212> DNA
   <213> Arabidopsis thaliana
<220>
   <221> misc_feature
   <222> (15)..(21)
   <223> Kernsequenz
<400> 30
   tgttgagtcg tttacgtcac gtcgagaatt ttctc 35
<210> 31
   <211> 35
   <212> DNA
   <213> Arabidopsis thaliana
<220>
   <221> misc_feature
   <222> (15)..(21)
   <223> Kernsequenz
<400> 31
   tgtcattatt aatacgtgac gaaactgtag ctctg 35
<210> 32
   <211> 36
   <212> DNA
   <213> Arabidopsis thaliana
<220>
   <221> misc_feature
   <222> (11)..(25)
   <223> Kernsequenz
<400> 32
   ttacgtgtca agaagtgatt ggagaggaca ctctac 36
<210> 33
   <211> 35
   <212> DNA
   <213> Arabidopsis thaliana
<220>
   <221> misc_feature
   <222> (15)..(21)
   <223> Kernsequenz
<400> 33
   ccatacaata taaaccacca aaccataacc acaaa 35
<210> 34
   <211> 26
   <212> DNA
   <213> Arabidopsis thaliana
<220>
   <221> misc_feature
   <222> (9)..(18)
   <223> Kernsequenz
<400> 34
   caatctactc gtctcttctc ttacat 26
<210> 35
   <211> 10
   <212> DNA
   <213> Arabidopsis thaliana
<400> 35
   tcgtctcttc 10
<210> 36
   <211> 19
   <212> DNA
   <213> Artificial Sequence
<220>
   <223> PCR Primer
<400> 36
   gtggagaggc tattcggta 19
<210> 37
   <211> 20
   <212> DNA
   <213> Artificial Sequence
<220>
   <223> PCR Primer
<400> 37
   ccaccatgat attcggcaag 20
<210> 38
   <211> 182
   <212> DNA
   <213> Oryza sativa
<400> 38
   tcctctctac tcctcagcat ctatataagg gccttgcagg cagtggctct cacaccaacg 60
   aaacaaggag agactcagag aggaggcgtg ttggttagct cattggcagc agcacacaca 120
   aaccacatct cctatatata gctcattttt agcttttgga attgagagag gttttgagag 180
   aa 182
<210> 39
   <211> 149
   <212> DNA
   <213> Triticum aestivum
<400> 39
   atctgcaggc gctgcctatt taatcccttc ccctccctcc attcccctcc aagaagagcc 60
   acagcttcat ctgcagctac agctcctctt cgtcttcgac acacaagtat tttttcagga 120
   caaagatcaa tccagataca catacacct 149
<210> 40
   <211> 1133
   <212> DNA
   <213> zea mays
<220>
   <221> Intron
   <222> (129)..(1133)
<400> 40
   cttcctcgcc cgccgtaata aatagacacc ccctccacac cctctttccc caacctcgtg 60
   ttgttcggag cgcacacaca cacaaccaga tctcccccaa atccacccgt cggcacctcc 120
   gcttcaaggt acgccgctcg tcctcccccc ccctctctac cttctctaga tcggcgttcc 180
   ggtccatggt tagggcccgg tagttctact tctgttcatg tttgtgttag atccgtgttt 240
   gtgttagatc cgtgctgcta gcgttcgtac acggatgcga cctgtacgtc agacacgttc 300
   tgattgctaa cttgccagtg tttctctttg gggaatcctg ggatggctct agccgttccg 360
   cagacgggat cgatttcatg attttttttg tttcgttgca tagggtttgg tttgcccttt 420
   tcctttattt caatatatgc cgtgcacttg tttgtcgggt catcttttca tgcttttttt 480
   tgtcttggtt gtgatgatgt ggtctggttg ggcggtcgtt ctagatcgga gtagaattct 540
   gtttcaaact acctggtgga tttattaatt ttggatctgt atgtgtgtgc catacatatt 600
   catagttacg aattgaagat gatggatgga aatatcgatc taggataggt atacatgttg 660
   atgcgggttt tactgatgca tatacagaga tgcttttgtt cgcttggttg tgatgatgtg 720
   gtgtggttgg gcggtcgttc attcgttcta gatcggagta gaatactgtt tcaaactacc 780
   tggtgtattt attaattttg gaactgtatg tgtgtgtcat acatcttcat agttacgagt 840
   ttaagatgga tggaaatatc gatctaggat aggtatacat gttgatgtgg gttttactga 900
   tgcatataca tgatggcata tgcagcatct attcatatgc tctaaccttg agtacctatc 960
   tattataata aacaagtatg ttttataatt attttgatct tgatatactt ggatgatggc 1020
   atatgcagca gctatatgtg gattttttta gccctgcctt catacgctat ttatttgctt 1080
   ggtactgttt cttttgtcga tgctcaccct gttgtttggt gttacttctg cag 1133
<210> 41
   <211> 18
   <212> DNA
   <213> Artificial sequence
<220>
   <223> Familienmotiv Gruppe 21n
<220>
   <221> misc_feature
   <222> (2)..(2)
   <223> n is a, c, g, or t
<220>
   <221> misc_feature
   <222> (4)..(6)
   <223> n is a, c, g, or t
<220>
   <221> misc_feature
   <222> (7)..(12)
   <223> Kernsequenzmotiv
<220>
   <221> misc_feature
   <222> (13)..(15)
   <223> n is a, c, g, or t
<220>
   <221> misc_feature
   <222> (17)..(17)
   <223> n is a, c, g, or t
<400> 41
   snsnnnwwkg wcnnnsnm 18
<210> 42
   <211> 29
   <212> DNA
   <213> Arabidopsis thaliana
<220>
   <221> misc_feature
   <222> (5)..(25)
   <223> Kernsequenzmotiv
<400> 42
   ctcaaaggcc agaattgacg cagccgttt 29
<210> 43
   <211> 28
   <212> DNA
   <213> Arabidopsis thaliana
<220>
   <221> misc_feature
   <222> (7)..(22)
   <223> Kernsequenzmotiv
<400> 43
   ccttggccca gtccttggtc gtcgtatc 28
<210> 44
   <211> 11
   <212> DNA
   <213> Arabidopsis thaliana
<220>
   <221> misc_feature
   <222> (5)..(11)
   <223> Kernsequenzmotiv
<400> 44
   gttggacttt c 11